# EUROPEAN PATENT SPECIFICATION

(11) **EP 3 836 149 B1**
(45) Date of publication and mention of the grant of the patent: **14.10.2026**
(21) Application number: 20197880.6
(22) Date of filing: 06.11.2012
(51) Int. Cl.: G16B 20/10, G16B 20/20, G16B 20/30, G16B 50/00, G16B 50/10, G16B 20/00, G16B 30/00

(54) **METHODS AND SYSTEMS FOR IDENTIFICATION OF CAUSAL GENOMIC VARIANTS**
VERFAHREN UND SYSTEME ZUR IDENTIFIZIERUNG KAUSALER GENOMVARIANTEN
PROCÉDÉS ET SYSTÈMES POUR L'IDENTIFICATION DE VARIANTS GÉNOMIQUES CAUSALS

(30) Priority: 07.11.2011 US 201161556599 P; 07.11.2011 US 201161556758 P
(43) Date of publication of application: 16.06.2021
(62) Document number(s) of the earlier application(s) in accordance with Art. 76 EPC: 12847098.6 / 2 776 962
(73) Proprietor: QIAGEN Redwood City, Inc., Redwood City, California 94063 (US)
(72) Inventor: BASSETT, Douglas E. JR., Kirkland, WA 98034 (US); RICHARDS, Daniel R., Palo Alto, CA 94301 (US)
(74) Representative: Vossius & Partner Patentanwälte Rechtsanwälte mbB

(56) References cited:
- US-A1- 2003 190 649

## Description

### CROSS-REFERENCE TO RELATED APPLICATIONS

This application claims the benefit of and priority to U.S. Provisional Patent Application No. 61/556,599 filed November 7, 2011, entitled "Method and Systems for Identification of Causal Genomic Variants;" and U.S. Provisional Patent Application No. 61/556,758 filed November 7, 2011, entitled "Method and Systems for Identification of Causal Genomic Variants."

### BACKGROUND OF THE INVENTION

Full genome sequencing can provide information regarding about six billion base pairs in the human genome, yet the analysis of this massive amount of information has proven challenging. For example, between genomes there is a large amount variation, but only some of the variants actually affect phenotype. Of the variants that affect phenotype, only a subset these are relevant to a particular phenotype, for example a disease. At present, a clinician or researcher who obtains full genome sequence information from a subject faces the challenge of sifting through the huge amount variant information to try and identify the subset of variants which may matter for a particular phenotype. Herein described are systems and methods to focus the attention of the researcher or clinician on potentially relevant genomic variants. US 2003/190649 A1 relates to a data mining of SNP databases for the selection of intragenic SNPs.

### SUMMARY OF THE INVENTION

Methods and systems for filtering variants in data sets comprising genomic information are provided herein. The invention is specified by the independent claims. Preferred embodiments are defined in the dependent claims.

In some embodiments a biological context filter wherein the biological context filter: is configured to receive a data set comprising variants, is in communication with a database of biological information, and is capable of transforming the data set by filtering the data set by variants associated with biological information, wherein the filtering comprises establishing associations between the data set and some or all of the biological information. In some embodiments the biological the database of biological information is a knowledge base of curated biomedical content, wherein the knowledge base is structured with an ontology. In some embodiments the associations between the variants and the biological information comprises a relationship defined by one or more hops. In some embodiments a user selects the biological information for filtering. In some embodiments the filtering unmasks variants associated with the biological information. In some embodiments the filtering masks variants not associated with the biological information. In some embodiments the filtering masks variants associated with biological information. In some embodiments the filtering unmasks variants not associated with the biological information. In some embodiments biological information for filtering is inferred from the data set. In some embodiments biological information for filtering is inferred from study design information previously inputted by a user.

In some embodiments a biological context filter: is configured to receive a data set comprising variants wherein the data set comprises variant data from one or more samples from one or more individuals, is in communication with a database of biological information, and is capable of transforming the data set by filtering the data set by variants associated with biological information, wherein the filtering comprises establishing associations between the data set and some or all of the biological information.

In some embodiments biological context filter is combined with other filters in a filter cascade to generate a final variant list. In some embodiments the biological context filter is combined with one or more of the following filters in a filter cascade to reach a final variant list of less than 200 variants: common variant filter, predicted deleterious filter, cancer driver variants filter, physical location filter, genetic analysis filter, expression filter, user-defined variants filter, pharmacogenetics filter, or custom annotation filter. In some embodiments the biological context filter is combined with one or more of the following filters in a filter cascade to reach a final variant list of less than 50 variants: common variant filter, predicted deleterious filter, cancer driver variants filter, physical location filter, genetic analysis filter, expression filter, user-defined variants filter, pharmacogenetics filter, or custom annotation filter.

In some embodiments stringency of the biological context filter can be adjusted by a user, and wherein the stringency adjustment from the user alters one or more of the following: the number of hops in an association used for filtering, the strength of hops in an association used for filtering, the net effect of the hops in an association used for filtering, and/or the upstream or downstream nature of hops in an association used for filtering. In some embodiments the stringency of the biological filter is adjusted automatically based upon the desired number of variants in the final filtered data set, wherein the stringency adjustment alters one or more of the following: the number of hops in an association used for filtering, the strength of hops in an association used for filtering, the net effect of the hops in an association used for filtering, and/or the upstream or downstream nature of hops in an association used for filtering.

In some embodiments only upstream hops are used. In some embodiments only downstream hops are used. In some embodiments the net effects of hops are used. In some embodiments the biological information for filtering is biological function.

In some embodiments the biological function is a gene, a transcript, a protein, a molecular complex, a molecular family or enzymatic activity, a therapeutic or therapeutic molecular target, a pathway, a process, a phenotype, a disease, a functional domain, a behavior, an anatomical characteristic, a physiological trait or state, a biomarker or a combination thereof. In some embodiments the stringency of the biological context filter is adjusted by selection of the biological information for filtering. In some embodiments the biological context filter is configured to accept a mask from another filter previously performed on the same data set.

In some embodiments the biological context filter is in communication with hardware for outputting the filtered data set to a user. In some embodiments a computer program product bearing machine readable instructions enacts the biological context filter.

In some embodiments a cancer driver variants filter is provided wherein the cancer driver variants filter: is configured to receive a first data set comprising variants, and is capable of transforming the first data set by filtering the first data set by variants associated with one or more proliferative disorders. In some embodiments the cancer driver variants filter is in communication with hardware for outputting the filtered data set to a user. In some embodiments the first data set is suspected to contain variants associated with one or more proliferative disorders. In some embodiments the first data set was derived from a patient with a proliferative disorder. In some embodiments the proliferative disorder is cancer. In some embodiments a user specifies one or more proliferative disorders of interest for filtering. In some embodiments the filtering unmasks variants associated with the one or more proliferative disorders. In some embodiments filtering masks variants not associated with the one or more proliferative disorders. In some embodiments the filtering masks variants associated with the one or more proliferative disorders. In some embodiments the filtering unmasks variants not associated with the one or more proliferative disorders.

In some embodiments a cancer driver variants filter: is configured to receive a data set comprising variants wherein said data set comprises variant data from one or more samples from one or more individuals, and is capable of transforming the data set by filtering the data set by variants associated with one or more proliferative disorders.

In some embodiments a cancer driver variants filter: is configured to receive a data set comprising variants wherein said data set comprises variant data from one or more samples from one or more individuals, and is capable of transforming the data set by filtering the data set by variants associated with one or more proliferative disorders.

In some embodiments the one or more proliferative disorders for filtering is inferred from the data set. In some embodiments the one or more proliferative disorders for filtering is inferred from study design information previously inputted by a user.

In some embodiments the cancer driver variants filter is combined with other filters in a filter cascade to generate a final variant list. In some embodiments the cancer driver variants filter is combined with one or more of the following filters in a filter cascade to reach a final variant list of less than 200 variants: common variant filter, predicted deleterious filter, biological context filter, physical location filter, genetic analysis filter, expression filter, user-defined variants filter, pharmacogenetics filter, or custom annotation filter. In some embodiments the cancer driver variants filter is combined with one or more of the following filters in a filter cascade to reach a final variant list of less than 50 variants: common variant filter, predicted deleterious filter, biological context filter, physical location filter, genetic analysis filter, expression filter, user-defined variants filter, pharmacogenetics filter, or custom annotation filter.

In some embodiments the filtered variants are variants observed or predicted to meet one or more of the following criteria: are located in human genes having animal model orthologs with cancer-associated gene disruption phenotypes, impact known or predicted cancer subnetwork regulatory sites, impact cancer-associated cellular processes with or without enforcement of appropriate directionality, are associated with published cancer literature findings in a knowledge base at the variant- and/or gene-level, impact cancer-associated pathways with or without enforcement of appropriate directionality, and/or are associated with cancer therapeutic targets and/or upstream/causal subnetworks. In some embodiments the criteria are restricted to one or more specific cancer disease models.

In some embodiments the cancer driver variants filter is in communication with a database of biological information, wherein the database of biological information is a knowledge base of curated biomedical content, wherein the knowledge base is structured with an ontology.

In some embodiments the stringency of the cancer driver variants filter is user adjustable, wherein the stringency adjustment from the user alters the number of hops and/or the strength of hops in a relationship and/or whether or not the variants are observed or predicted to have one or more of the following characteristics: are located in human genes having animal model orthologs with cancer-associated gene disruption phenotypes, impact known or predicted cancer subnetwork regulatory sites, impact cancer-associated cellular processes with or without enforcement of appropriate directionality, are associated with published cancer literature findings in a knowledge base at the variant- and/or gene-level, impact cancer-associated pathways with or without enforcement of appropriate directionality, and/or are associated with cancer therapeutic targets and/or upstream/causal subnetworks.

In some embodiments the stringency of the cancer driver variants filter is adjusted automatically based upon the desired number of variants in the final filtered data set, wherein the stringency adjustment alters the number of hops and/or the strength of hops in a relationship and/or whether or not the variants are observed or predicted to have one or more of the following characteristics: are located in human genes having animal model orthologs with cancer-associated gene disruption phenotypes, impact known or predicted cancer subnetwork regulatory sites, impact cancer-associated cellular processes with or without enforcement of appropriate directionality, are associated with published cancer literature findings in a knowledge base at the variant- and/or gene-level, impact cancer-associated pathways with or without enforcement of appropriate directionality, and/or are associated with cancer therapeutic targets and/or upstream/causal subnetworks.

In some embodiments the variants associated with one or more proliferative disorders are variants which are one or more hops from variants that are predicted or observed to have one or more of the following characteristics: are located in human genes having animal model orthologs with cancer-associated gene disruption phenotypes, impact known or predicted cancer subnetwork regulatory sites, impact cancer-associated cellular processes with or without enforcement of appropriate directionality, are associated with published cancer literature findings in a knowledge base at the variant- and/or gene-level, impact cancer-associated pathways with or without enforcement of appropriate directionality, and/or are associated with cancer therapeutic targets and/or upstream/causal subnetworks.

In some embodiments the stringency of the cancer driver variants filter is adjusted by weighting the strength of the hops. In some embodiments the stringency of the cancer driver variants filter is adjusted by altering the number of hops. In some embodiments the hops are upstream hops or the hops are downstream hops. In some embodiments the net effects of the hops are determined and only variants associated with cancer driving net effects are filtered. In some embodiments the cancer driver variants filter is configured to accept a mask from another filter previously performed on the same data set.

In some embodiments a computer program product bearing machine readable instructions to enact the cancer driver variants filter.

In some embodiments a genetic analysis filter is provided wherein the genetic analysis filter is configured to receive a first data set comprising variants, is capable of transforming the first data set by filtering the first data set according to genetic logic. In some embodiments the genetic analysis filter is in communication with hardware for outputting the filtered data set to a user. In some embodiments the genetic analysis filter is further configured to receive one or more additional data sets obtained from samples genetically related to a source of the first data set.

In some embodiments the genetics analysis filter is configured to receive information optionally identifying samples from the same individual or hereditary relationships among individuals with samples in the data set.

In some embodiments at least one sample in the data set is a disease case sample and another sample in the data set is a normal control sample from the same individual, wherein the filtering comprises filtering variants either observed in both the disease and normal samples or observed uniquely in either the disease sample or the normal sample.

In some embodiments the one or more samples in the data set are genetic parents of another sample in the data set.In some embodiments the filtering comprises filtering variants from the data set that are incompatible with Mendelian genetics. In some embodiments the filtering comprises filtering variants that are heterozygous in parents and homozygous in samples from their progeny. In some embodiments the filtering comprises filtering variants absent in at least one of the parents of a homozygous child. In some embodiments the filtering comprises filtering variants absent in both of the parents of a child with the variant.

In some embodiments the data set has been previously filtered and wherein a subset of the data points in the data set have been masked by the previous filter.

In some embodiments the filtering comprises filtering variants that are present at a given zygosity in greater than or equal to a specified fraction of case samples but less than or equal to a specified fraction of control samples, and/or filtering variants that are present at a given zygosity in less than or equal to a specified fraction of case samples but greater than or equal to a specified fraction of control samples.

In some embodiments the filtering comprises filtering variants that are present at a given quality level in greater than or equal to a specified fraction of case samples but less than or equal to a specified fraction of control samples, and/or filtering variants that are present at a given quality level in less than or equal to a specified fraction of case samples but greater than or equal to a specified fraction of control samples.

In some embodiments the first data set is from a tumor sample and a second data set is from a normal sample from the same individual, wherein the filtering comprises filtering variants either observed in both the first and second data sets or observed uniquely in either the tumor sample or the normal sample.

In some embodiments the genetic logic is configured based on presets from a user for recessive hereditary disease, dominant hereditary disease, de novo mutation, or cancer somatic variants.

In some embodiments variants are filtered that are inferred to contribute to a gain or loss of function of a gene in either (a) greater than or equal to a specified fraction of case samples but less than or equal to a specified fraction of control samples, or (b) less than or equal to a specified fraction of case samples but greater than or equal to a specified fraction of control samples.

In some embodiments the one or more additional data sets comprises data sets from either or both of the genetic parents of the source of the first data set. In some embodiments the filtering comprises filtering variants from the first data set that are incompatible with Mendelian genetics. In some embodiments the filtering comprises filtering variants that are homozygous in both parents of the source of the first data set but heterozygous in the first data set. In some embodiments the filtering comprises filtering variants absent in at least one of the parents of the source of the first data set but homozygous in the first data set. In some embodiments the filtering comprises filtering variants absent in both of the parents of the source of the first data set but present in the first data set. In some embodiments filtered variants are single copy variants located in a hemizygous region of the genome.

In some embodiments the filtering comprises filtering variants that are a) absent in the child when at least one parent is homozygous, and/or (b) heterozygous in the child if both parents are homozygous.

In some embodiments the genetic analysis filter is further in communication with a database of biological information, wherein the database of biological information is a knowledge base of curated biomedical content, wherein the knowledge base is structured with an ontology, and wherein the variants from the first data set can be associated with the biological information by hops.

In some embodiments the biological information comprises information regarding haploinsufficiency of genes. In some embodiments heterozygous variants associated with haploinsuffucient genes are filtered.

In some embodiments variants are filtered that occur with zygosity and/or quality settings specified by the user in either (a) at least a specified number or minimal fraction of case samples and at most a specified number or maximum fraction of control samples, or (b) at most a specified number or maximum fraction of case samples and at least a specified number or minimum fraction of control samples. In some embodiments variants are filtered that affect the same gene in either (a) at least a specified number or minimal fraction of case samples and at most a specified number or maximum fraction of control samples, or (b) at most a specified number or maximum fraction of case samples and at least a specified number or minimum fraction of control samples.

In some embodiments variants are filtered that affect the same network within 1 or more hops in either: (a) at least a specified number or minimal fraction of case samples and at least a specified number or maximum fraction of control samples, or (b) at most a specified number or maximum fraction of case samples and at least a specified number or minimum fraction of control samples. In some embodiments the stringency of the genetic analysis filter is adjusted by weighting the strength of the hops.

In some embodiments the stringency of the genetic analysis filter is adjusted altering the number of hops. In some embodiments the hops are upstream hops. In some embodiments the hops are downstream hops.

In some embodiments the genetic first data set has been previously filtered and wherein a subset of the data points in the first data set have been masked by the previous filter. In some embodiments the stringency is adjusted by a user. In some embodiments the filter stringency is adjusted automatically based on the desired number of variants in the final filtered data set.

In some embodiments the genetic analysis filter is combined with other filters in a filter cascade to yield a final filtered data set of interest to a user. In some embodiments the genetic analysis filter combined with one or more of the following filters in a filter cascade to reach a final variant list of less than 50 variants: common variant filter, predicted deleterious filter, biological context filter, physical location filter, cancer driver variants filter, expression filter, user-defined variants filter, pharmacogenetics filter, or custom annotation filter. In some embodiments genetic analysis filter is with one or more of the following filters in a filter cascade to reach a final variant list of less than 200 variants: common variant filter, predicted deleterious filter, biological context filter, physical location filter, cancer driver variants filter, expression filter, user-defined variants filter, pharmacogenetics filter, or custom annotation filter.

In some embodiments the stringency adjustment alters a zygosity requirement of the filter. In some embodiments the stringency adjustment alters a variant quality requirement of the filter. In some embodiments the stringency adjustment alters the required number or fraction of case samples for filtering.

In some embodiments the stringency adjustment alters whether the genetic analysis filter is filtering variants based on whether they (a) occur with zygosity and/or quality settings specified by the user, or (b) affect the same gene, or (c) affect the same network within 1 or more hops. In some embodiments the stringency of the genetic analysis filter is adjusted by weighting the strength of the hops. In some embodiments the stringency of the genetic analysis filter is adjusted by altering the number of hops. In some embodiments the net effects of the hops are determined and only variants associated with user selected net effects are filtered. In some embodiments the cancer driver variants filter is configured to accept a mask from another filter previously performed on the same data set.

In some embodiments a genetic analysis filter: is configured to receive a data set comprising variants wherein said data set comprises variant data from one or more samples from one or more individuals, and is capable of transforming the data set by filtering the data set according to genetic logic.

In some embodiments a computer program product bearing machine readable instructions enacts the genetic analysis filter.

In some embodiments a pharmacogenetics filter is provided wherein the pharmacogenetics filter is configured to receive a data set comprising variants, is in communication with a database of biological information, wherein the database of biological information is a knowledge base of curated biomedical content, wherein the knowledge base is structured with an ontology, wherein the biological information is information related to one or more drugs, and is capable of transforming the data set by filtering the data set by variants associated with biological information, wherein the filtering comprises establishing associations between the data set and some or all of the biological information. In some embodiments the pharmacogenetics filter is in communication with hardware for outputting the filtered data set to a user. In some embodiments information related to one or more drugs comprises drug targets, drug responses, drug metabolism, or drug toxicity. In some embodiments the associations between the variants and the biological information comprises a relationship defined by one or more hops. In some embodiments a user selects the biological information for filtering.

In some embodiments a pharmacogenetics filter: is configured to receive a data set comprising variants, wherein the data set comprises variant data from one or more samples from one or more individuals; is in communication with a database of biological information, wherein the database of biological information is a knowledge base of curated biomedical content, wherein the knowledge base is structured with an ontology, wherein the biological information is information related to one or more drugs; and is capable of transforming the data set by filtering the data set by variants associated with biological information, wherein the filtering comprises establishing associations between the data set and some or all of the biological information.

In some embodiments the filtering unmasks variants associated with the biological information. In some embodiments the filtering masks variants not associated with the biological information. In some embodiments the filtering masks variants associated with biological information. In some embodiments the filtering unmasks variants not associated with the biological information.

In some embodiments biological information for filtering is inferred from the data set. In some embodiments biological information for filtering is inferred from study design information previously inputted by a user. In some embodiments the biological context filter is combined with other filters in a filter cascade to generate a final variant list.

In some embodiments the pharmacogenetics filter is combined with one or more of the following filters in a filter cascade to reach a final variant list of less than 200 variants: common variant filter, predicted deleterious filter, cancer driver variants filter, physical location filter, genetic analysis filter, expression filter, user-defined variants filter, biological context filter, or custom annotation filter. In some embodiments the pharmacogenetics filter is combined with one or more of the following filters in a filter cascade to reach a final variant list of less than 50 variants: common variant filter, predicted deleterious filter, cancer driver variants filter, physical location filter, genetic analysis filter, expression filter, user-defined variants filter, biological context filter, or custom annotation filter.

In some embodiments the stringency of the pharmacogenetics filter can be adjusted by a user, and wherein the stringency adjustment from the user alters one or more of the following: the number of hops in an association used for filtering, the strength of hops in an association used for filtering, whether or not predicted drug response biological information is used for filtering, whether or not predicted drug metabolism or toxicity information is used for filtering, whether or not established drug target(s) are used for filtering, the net effect of the hops in an association used for filtering and/or, the upstream or downstream nature of hops in an association used for filtering.

In some embodiments the stringency of the pharmacogenetics filter is adjusted automatically based upon the desired number of variants in the final filtered data set, wherein the stringency adjustment alters one or more of the following: the number of hops in an association used for filtering the strength of hops in an association used for filtering, whether or not predicted drug response biological information is used for filtering, whether or not predicted drug metabolism or toxicity information is used for filtering, whether or not established drug target(s) are used for filtering, the net effect of the hops in an association used for filtering and/or, the upstream or downstream nature of hops in an association used for filtering.

In some embodiments in the pharmacogenetics filter only upstream hops are used, only downstream hops are used., and/or the net effects of hops are used.

In some embodiments stringency of the pharmacogenetic filter is adjustable by the user. In some embodiments the pharmacogenetics filter is configured to accept a mask from another filter previously performed on the same data set.

In some embodiments a computer program product bearing machine readable instructions enacts the pharmacogenetic filter variants filter.

In some embodiments a predicted deleterious filter is provided wherein the predicted deleterious filter: is configured to receive a data set comprising variants, and is capable of transforming the data set by filtering the data by variants predicted to be deleterious or non-deleterious. In some embodiments the predicted deleterious filter is in communication with hardware for outputting the filtered data set to a user.

In some embodiments the filtering comprises utilizing at least one algorithm for predicting deleterious or non-deleterious variants in the data set and then filtering the predicted deleterious or non-deleterious variants. In some embodiments the at least one algorithm is SIFT, BSIFT, PolyPhen, PolyPhen2, PANTHER, SNPs3D, FastSNP, SNAP, LS-SNP, PMUT, PupaSuite, SNPeffect, SNPeffectV2.0, F-SNP, MAPP, PhD-SNP, MutDB, SNP Function Portal, PolyDoms, SNP@Promoter, Auto-Mute, MutPred, SNP@Ethnos, nsSNPanalyzer, SNP@Domain, StSNP, MtSNPscore, or Genome Variation Server.

In some embodiments conserved variants are filtered. In some embodiments the predicted deleterious variants are filtered based on a gene fusion prediction algorithm. In some embodiments the predicted deleterious variants are filtered based on variants creating or disrupting a predicted or experimentally validated miRNA binding site. In some embodiments the predicted deleterious variants are filtered based on a predicted copy number gain algorithm. In some embodiments the predicted deleterious variants are filtered based on a predicted copy number loss algorithm. Ios the predicted deleterious variants are filtered based on a predicted splice site loss or splice site gain. In some embodiments the predicted deleterious variants are filtered based on disruption of a known or predicted miRNA or ncRNA. In some embodiments the predicted deleterious variants are filtered based on disruption of or creation of a known or predicted transcription factor binding site. In some embodiments the predicted deleterious variants are filtered based on disruption of or creation of a known or predicted enhancer site. In some embodiments the predicted deleterious variants are filtered based on disruption of an untranslated region (UTR).

In some embodiments the predicted deleterious filter is further in communication with a database of biological information, wherein the database of biological information is a knowledge base of curated biomedical content, wherein the knowledge base is structured with an ontology, and wherein the variants from the first data set can be associated with the biological information either (a) directly based on one or more mutation findings in the knowledge base, (b) by a combination of gene findings and a functional prediction algorithm. In some embodiments the biological information comprises a deleterious phenotype, wherein the variants associated with the deleterious phenotypes are filtered. In some embodiments the deleterious phenotype is a disease.

In some embodiments predicted deleterious variants comprise variants which are directly associated with a mutation finding in the knowledge base, predicted deleterious (or non-innocuous) single nucleotide variants; predicted or known splice sites, predicted to create or disrupt a transcription factor binding site, predicted or known non-coding RNAs, predicted or known miRNA targets, or predicted or known enhancers.

In some embodiments the predicted deleterious variants comprise variants which are directly associated with a variant finding in the knowledge base, predicted deleterious (or non-innocuous) single nucleotide variants; predicted to create or disrupt a RNA splice site, predicted to create or disrupt a transcription factor binding site, predicted to disrupt non-coding RNAs, predicted to create or disrupt a microRNA target, or predicted to disrupt known enhancers.

In some embodiments the predicted deleterious filter is combined with other filters in a filter cascade to yield a final filtered data set of interest to the user. In some embodiments the predicted deleterious filter is combined with one or more of the following filters in a filter cascade to reach a final variant list of less than 50 variants: common variant filter, biological context filter, physical location filter, genetic analysis filter, cancer driver variants filter, expression filter, user-defined variants filter, pharmacogenetics filter, or custom annotation filter. In some embodiments the predicted deleterious filter is combined with one or more of the following filters in a filter cascade to reach a final variant list of less than 200 variants: common variant filter, biological context filter, physical location filter, genetic analysis filter, cancer driver variants filter, expression filter, user-defined variants filter, pharmacogenetics filter, or custom annotation filter.

In some embodiments a stringency of the predicted deleterious filter is adjustable by the user. In some embodiments the stringency is adjusted automatically based on the desired number of variants in the final filtered data set. In some embodiments the predicted deleterious variants are filtered based on a pathogenicity annotator.

In some embodiments the predicted deleterious filter is configured to accept a mask from another filter previously performed on the same data set.

In some embodiments a predicted deleterious filter: is configured to receive a data set comprising variants, wherein the data set comprises variant data from one or more samples from one or more individuals; and is capable of transforming the data set by filtering the data by variants predicted to be deleterious or non-deleterious.

In some embodiments a computer program product bearing machine readable instructions enacts a predicted deleterious filter.

In some embodiments the pathogenicity annotator categorizes variants using a predicted deleterious filter and a database of biological information, wherein the database of biological information is a knowledge base of curated biomedical content, and wherein the knowledge base is structured with an ontology.

In some embodiments the pathogenicity annotator is in communication with hardware for outputting the categorization to a user. In some embodiments the variants outputted into the following categories: Pathogenic, Likely Pathogenic, Uncertain, Likely Benign, Benign based upon a combination of the results of the predicted deleterious filter and the weight of evidence in the knowledge base supporting or refuting each variant's association with a deleterious phenotype. In some embodiments the terminology is varied or there are more or less categories, for instance the variants can be outputted into the following categories: Pathogenic, Presumed Pathogenic, Unknown, Presumed Benign, Benign based upon a combination of the results of the predicted deleterious filter and the weight of evidence in the knowledge base supporting or refuting each variant's association with a deleterious phenotype. In some embodiments the categorization includes one or more of the following catagoties: unknown, untested, non-pathogenic, probable-non-pathogenic, probable-pathogenic, pathogenic, drug-response, histocompatibility, or other. In some embodiments a) "pathogenic" means <0.07% frequency of the variant in a database of genomes of individuals free from known genetic disease, and 2 or more findings drawing a causal or associative link between the variant and a deleterious phenotype from multiple different articles in the biomedical literature; "Presumed Pathogenic" "Probable Patogenic" or Likely Pathogenic" means <0.07% frequency of the variant in a database of genomes of individuals free from known genetic disease, and 1 finding drawing a causal or associative link between the variant and a deleterious phenotype; "Unknown" or "Uncertaion" means between 0.07% and 0.1% frequency of the variant in a database of genomes of individuals free from known genetic disease; "Presumed Benign" or "Likely Benign" or "Probable non-pathogenic" means between 0.1% and 1% frequency of the variant in a database of genomes of individuals free from known genetic disease; and
"benign" means >=1% frequency of the variant in a database of genomes of individuals free from known genetic disease.

In some embodiments, the pathogenicity annotator is in communication with a knowledge base of disease models that define variants, genes, and pathways that are associated with that disease, wherein pathogenicity annotator utilizes the disease models to provide a pathogenicity assessment for a particular combination of a specific variant and a specific disease.

In some embodiments a preconfigurator is the preconfigurator is: configured to receive information provided by a user related to a data set comprising variants, in communication with one or more filters, in communication with the data set comprising variants, and capable of controlling the filters at least in part according to the information provided by the user wherein the preconfigurator selects filters and filter stringency related to the information provided by the user to yield a final filtered data set.

In some embodiments the preconfigurator controls the addition, removal, and stringency settings of one or more of the following filters: common variants filter, predicted deleterious filter, genetic analysis filter, biological context filter, pharmacogenetics filter, physical location filter, or cancer driver variants filter.

In some embodiments the preconfigurator optimizes the addition or removal of filters and filter stringency settings to achieve a final filtered data set of no more than 200 variants

In some embodiments the preconfigurator optimizes the addition or removal of filters and filter stringency settings to achieve a final filtered data set of no more than 50 variants.

In some embodiments the information provided by the user includes the mode of inheritance of a disease of interest. In some embodiments the information provided by the user includes a user input which can be recognized by the preconfigurator as an instruction for selecting filtering which: identifies causal disease variants, identifies cancer driver variants, identifies variants that stratify or differentiate one population from another, or analyzes a genome to identify variants of interest for health management, treatment, personalized medicine and/or individualized medicine.

In some embodiments the preconfigurator is in communication with a knowledge base of curated biomedical content, wherein the knowledge base is structured with an ontology.

In some embodiments the information from a user includes biological information including one or more genes, transcripts, proteins, drugs, pathways, processes, phenotypes, diseases, functional domains, behaviors, anatomical characteristics, physiological traits or states, biomarkers or a combination thereof.

In some embodiments a computer program product bearing machine readable instructions enacts the preconfigurator.

In some embodiments provided herein are methods for identifying prospective causal variants comprising: receiving a list of variants, filtering the list of variants with one or more common variants filters, filtering the list of variants with one or more predicted deleterious filters, filtering the list of variants with one or more genetic analysis filters, filtering the list of variants with one or more biological context filters, outputting the filtered list of variants as a list of prospective causal variants.

In some embodiments the causal outputting step occurs less than 1 day following the receiving step.

In some embodiments the causal outputting step occurs less that 1 week following the receiving step.

In some embodiments the list of variants comprises more than 1 million variants and the outputted filtered list of variants comprises less than 50 variants.

In some embodiments a graphical user interface is used for displaying the output of a filter cascade, wherein the filter cascade comprises one or more of the following: a common variants filter, a predicted deleterious filter, a genetic analysis filter, or a biological context filter.

In some embodiments provided herein are methods for the delivery of an interactive report method comprising the steps of: receiving a request for a quotation, wherein the quotation request comprises a disclosure of a number by a customer, wherein the number is the number of samples the costumer would like a price quotation on for genomic analysis services; transmitting a price quotation based at least in part upon the number of samples, wherein the price quotation comprises the cost of an interactive report for the biological interpretation of variants in the samples using a database of biological information, wherein the database of biological information is a knowledge base of curated biomedical content, and wherein the knowledge base is structured with an ontology; receiving an order from a customer, wherein the order comprises ordering the interactive report for the biological interpretation of variants using a database of biological information; and providing a hyperlink to the customer, wherein the hyperlink directs the customer the interactive report for the biological interpretation of variants using a database of biological information.

In some embodiments provided herein are methods for the delivery of an interactive report method comprising the steps of: receiving a request for a quotation, wherein the quotation request comprises a disclosure of a number by a customer, wherein the number is the number of samples the costumer would like a price quotation on for genomic analysis services; transmitting a price quotation at least in part based upon the number of samples, wherein the price quotation comprises the cost of an interactive report for the biological interpretation of variants using a database of biological information; receiving an order from a customer, wherein the order does not include ordering the interactive report for the biological interpretation of variants using a database of biological information; and providing a hyperlink to the customer, wherein the hyperlink directs the customer to a second price quotation for the interactive report for the biological interpretation of variants using a database of biological information. In some embodiments the interactive report for the biological interpretation of variants using a database of biological information has been generated prior to providing the second price quotation. In some embodiments the second price quotation comprises a preview of the analysis. In some embodiments the preview of the analysis is variants predicted to be of interest to the customer.

In some embodiments provided herein are methods for providing an interactive report to a customer for the biological interpretation of variants using a database of biological information comprising: receiving a data set comprising genomic information from a partner company, wherein the partner company received the sample from a customer and generated the data set from the sample, and loading the data set into a software system for biological interpretation of variants for future access by the user. In some embodiments the software system comprises one or more of the filters described herein. In some embodiments the methods further comprise: receiving a confirmation of an order from the customer after the generation of the interactive report; and providing the interactive report to the customer. In some embodiments the database of biological information is a knowledge base of curated biomedical content, and wherein the knowledge base is structured with an ontology.

In some embodiments the customer is a healthcare provider. In some embodiments the customer is an individual. In some embodiments the customer is a healthcare consumer. In some embodiments the customer is an organization.

In some embodiments the data set delivered by the provider of genomic analysis services and the interactive report for said data set are delivered to the customer on the same day. In some embodiments the data set delivered by the provider of genomic analysis services and the interactive report for said data set are delivered to the customer in the same week. The delivery can, in some embodiments, occur nearly simultaneous to payment by the customer.

In some embodiments the genomic analysis services and the interactive report for the data set to be produced by said genomic analysis services are quoted to the customer on the same day. In some instances the quote is within an hour, minutes, or is simultaneous.

In some embodiments the genomic analysis services and the interactive report for the data set to be produced by said genomic analysis services are quoted to the customer on the same day.

In some embodiments the interactive report is generated using a filter cascade, wherein the filter cascade comprises one or more of: a pharmacogenetics, a common variant filter, a predicted deleterious filter, a cancer driver variants filter, a physical location filter, a genetic analysis filter, a expression filter, a user-defined variants filter, a biological context filter, or a custom annotation filter.

In some embodiments a method for displaying genetic information to a user comprises: displaying to a user a two dimensional grid with samples on one axis and variants occurring in one or more samples on the other axis, wherein each cell of the grid represents a distinct instance of a variant (or lack thereof) in each sample, displaying, in each cell one or more colored icons, wherein the color of the one or more icons in each cell of the grid varies depending upon whether the variant represented by that cell is predicted to cause a gain-of-function, loss-of- function, or result in normal function of a gene or gene network in the sample represented by that cell.

In some embodiments a number of visually distinct shapes within a cell representing a particular variant and a particular sample correlates linearly with zygosity and/or copy number at the position of said particular variant in said particular sample.

In some embodiments the icon in a cell is distinct in shape and/or color if the sample represented by that cell has a genotype that is identical to the reference genome.

In some embodiments the color intensity is varied according to genotype quality, wherein higher color intensity indicates a higher quality measurement

In some embodiments one or more of the icons in a cell change shape and/or color if the variant represented by that cell is predicted to create a gene fusion in the sample represented by that cell.

In some embodiments the icon in a cell is distinct in shape and/or color if the location of the variant represented by that cell has no data or there is an inability to make an accurate genotype call at the position of that variant in the sample represented by that cell.

In some embodiments a computer program product bearing machine readable instructions to enacts a method for displaying genetic information to a user.

In some embodiments a computer-implemented pedigree builder wherein is configured to utilize input from the user to identify the sample most likely derived from the mother of the individual from which a given sample was derived. On other embodiments, the pedigree builder is configured to utilize input from the user to identify the sample most likely derived from the father of the individual from which a given sample was derived. In other embodiments, the pedigree builder is configured to construct pedigree information and make available to a genetic analysis filter of item 62 for further filtering of variants. In some embodiments, the pedigree builder may also infers all trios and family relationships within a given study, or identify potential pedigree inconsistencies such as that between relationships derived from user input, derived from computational analysis or where inconsistencies may comprise non-paternity, sample mislabeling or sample mix-up errors.

In some embodiments, the pedigree builder may assign the same individual identifier to multiple samples derived from the same individual, such that the program is able to infer a patient's normal genome and the matched tumor genome(s) from the same patient.

In some embodiments, a computer-implemented statistical association filter wherein the statistical association filter is configured to utlilize inputs of a previous filter in a filter cascade as input; filter variants using a basic allelic, dominant, or recessive model that are statistically significantly different between two or more sample groups; filter variants that perturb a gene differently between two or more sample groups with statistical significance using a burden test; and filter variants that perturb a pathway/gene set differently between two or more sample groups using a pathway or gene set burden test.

In some embodiments, the statistical filter is able to distinguish between disease affected and unaffected states using a burden test selected from the following: a case-burden, control-burden, and 2-sided burden test. In other embodiments, the statistical association filter is able to distinguish between disease affected and unaffected states using a burden test that utilizes only variants that pass the previous filter in the filter cascade inputed into the program in computing statistically significant variants.

In some embodiments, the statistical association filter is able to identify variants that are deleterious and contribute to inferred gene-level loss of function or inferred gene-level gain-of- function by utilizing the predicted deleterious filter and the genetic analysis.

In some embodiments, the statistical association filter, is able to distinguish between disease affected and unaffected states by utilizing a knowledge base of findings from the literature and to identify genes that together form a collective interrelated set based upon one or more shared elements selected from one or more of the following: pathway biology, domain, expression, biological process, disease relevance, group and complex annotation.

In some embodiments, the statistical association filter is able to distinguishe between disease affected and unaffected states by identifying variants that perturb said pathway or gene set significantly more or significantly less between two or more sample groups.

In some embodiments, the statistical association filter of item 187 wherein the pathway or gene set burden test can be performed across a library of pathways/genesets or a user-specified subset thereof.

In some embodiments, a computer-implemented Publish Feature wherein the Publish Feature is configured to enable the use to specify an analysis of interest; enable the user to enter a brief name and/or description of said analysis; provide the user with a URL internet link that can be embedded by the user in a publication; provide the user with the ability to release the published analysis for broad access; and upon said release by the user, provide access to the user's published analysis to other users who access the URL of step (c) or who browse a list of available published analyses.

In some embodiments, a computer -implemented Druggable Pathway Feature wherein, given one or more variants that are causal or driver variants for disease in one or more patient samples, the Druggable Pathway Feature is configured to: identify drugs that are known to target, activate and/or repress a gene, gene product, or gene set that co-occurs in the same pathway or genetic network as said one or more variants; identify the predicted net effect of said one or more variants in the patient sample on the pathway or genetic network above through causal network analysis; and further identify drugs identified in step (a) that have a net effect on the pathway or genetic network that is directly opposite of the predicted impact of the variant on the said pathway or genetic network.

In some embodiments, the Druggable Pathway Feature is used to to identify patient samples representing patients likely to respond to one or more specific drugs of interest based on their sequence variant profiles.

In some embodiments, a Frequent Hitters Filter is configured to: access a knowledge base of hypervariable genes and genomic regions that are mutated among a collection of samples derived from individuals unaffected by the disease or phenotype of interest; filter variants that occur within hypervariable genes and/or genomic regions; and enumerate trinucleotide repeats through a trinucleotide repeat annotator.

In some embodiments, the trinucleotide annotator of the Frequency Hitter Filter is configured to: interact with a knowledge base of known trinucleotide repeat regions that contains information on the number of repeats that are benign and the number of repeats that are associated with one or more human phenotypes or severities thereof; assess the number of trinucleotide repeats at one or more genomic regions defined in the knowledge base in one or more patient whole genome or exome sequencing samples; assess whether the trinucleotide repeat length calculated in (b) is sufficient to cause a phenotype based on the knowledge base, for each trinucleotide repeat; and communicate with a predicted deleterious filter to enable filtering of variants t cause a phenotype based on the results of the trinucleotide repeat annotator.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

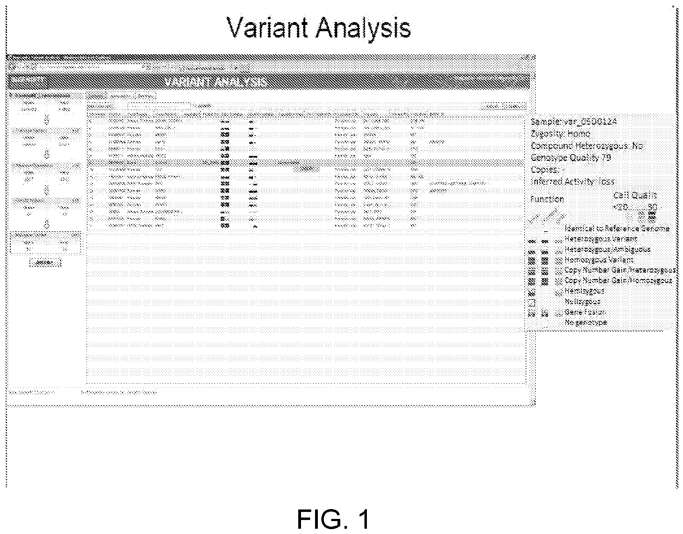

**Figure** 1 depicts one embodiment of a user interface representing a filter cascade vertically along the left hand side comprising one or more filters, in this case consisting of a Common Variants filter, a Predicted Deleterious filter, a Genetic Analysis filter, and a Biological Context filter. Each filter can "keep", "exclude" or "add back" variants from the variant data set. Each filter may also optionally take one or more masks from previous filters as input, which stipulates which variants have been retained and which variants have been masked out in previous filter steps in the filter cascade. In this non-limiting example, the final filtered variant data set is presented to the user, and the number of variants and associated genes represented in the final filtered variant data set is presented to the user at the bottom of the filter cascade in the leftmost vertical bar. Details on the variants that have not been masked out are shown in the table view on the right for the variants retained at the selected step of the filter cascade on the left.
The color-coded "Case Samples" and "Control Samples" columns combine a spectrum of useful information for the analysis of genetic information into a single multi-color graphical display, with legend for said display shown at the right. Blue color indicates loss of function at the gene level, orange color indicates gain-of- function, and black indicates probable normal function of the gene. Graphical icons allow rapid visual detection by the user of multiple key elements of genetic information for each case sample and each control sample including: (a) copy number gain, (b) copy number loss, (c) zygosity of the variant, (d) identity to the reference genome, (e) variant or genotype quality, (f) gene fusion status, (g) uncertainty or lack of ability to make a genotype call in a given sample at that position, and/or (g) loss of function including by such causes as a homozygous variant, a heterozygous variant in a hemizygous region, a heterozygous variant in a gene in which compound heterozygosity or haploinsufficiency occurs.

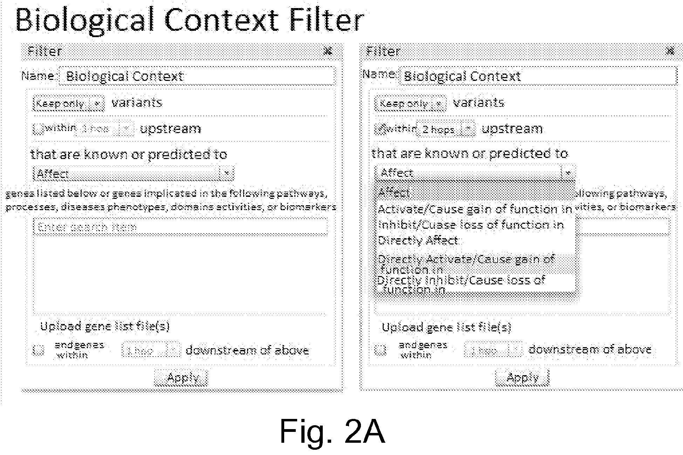

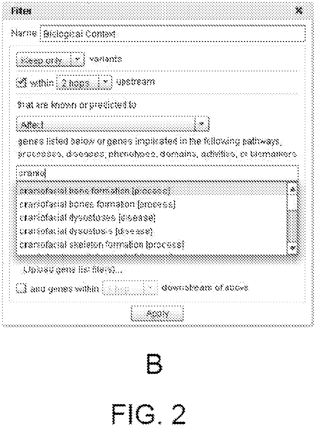

**Figure** 2 depicts (A) views of one embodiment of a Biological Context Filter user interface. Note that the Biological Context filter user interface on the right shows an example of user adjustment of stringency of the filter, wherein in this particular example the user has selected 2 hops and is about to specify variants that "Directly Activate/Cause gain of function in" a biological process of interest. The filter user interface also allows the user to specify downstream hops and one or more biological concepts of interest with autocompletion, leveraging a knowledge base organized using an ontology. (B) Filters linked to a knowledge base structured using an ontology can benefit from autocompletion, wherein the user types all or a portion of the name of a biological concept and matches to the characters entered, including synonyms from the ontology, are presented to the user that are dynamically updated with each user keystroke. This allows for convenient selection of biological information and biological concepts by the user, and for biological information implicated in concepts subsumed in the ontology by each biological concept of interest to be automatically included. This non-limiting example shows the application of autocompletion based on a knowledge base structured using an ontology within the user interface for a Biological Context filter.

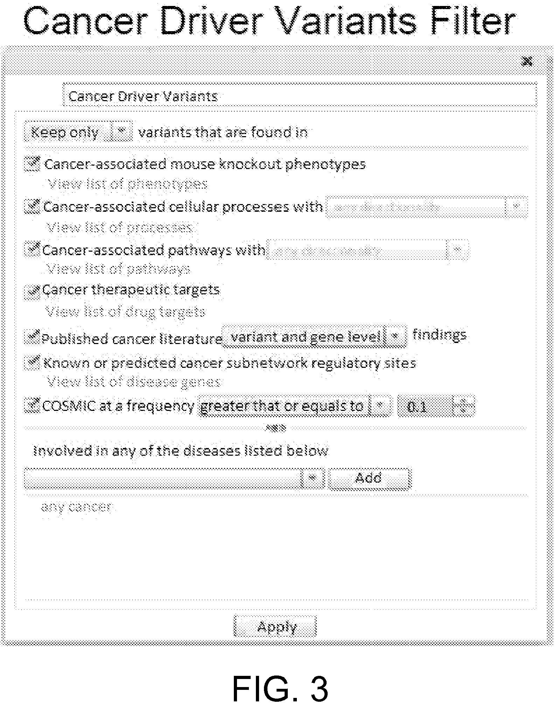

**Figure 3** depicts one embodiment of a user interface for a Cancer Driver Variants filter, wherein the filtered variants are observed or predicted to meet one or more of the following criteria:
   1. in human genes having mouse orthologs with cancer-associated gene disruption phenotypes,
   2. impact cancer-associated cellular processes with or without enforcement of appropriate directionality,
   3. impact cancer-associated pathways with or without enforcement of appropriate directionality,
   4. associated with cancer therapeutic targets and/or upstream/causal subnetworks,.
   5. associated with published cancer literature findings in a knowledge base at the variant- and/or gene-level,
   6. in the COSMIC database of somatic variants at a given frequency, and or
   7. impact known or predicted cancer pathways subnetwork regulatory sites.
This filter also benefits from selection of a disease model (e.g. "breast cancer") which focuses all of the other filter elements on the biological information relevant to the specific form of cancer described by the disease model.

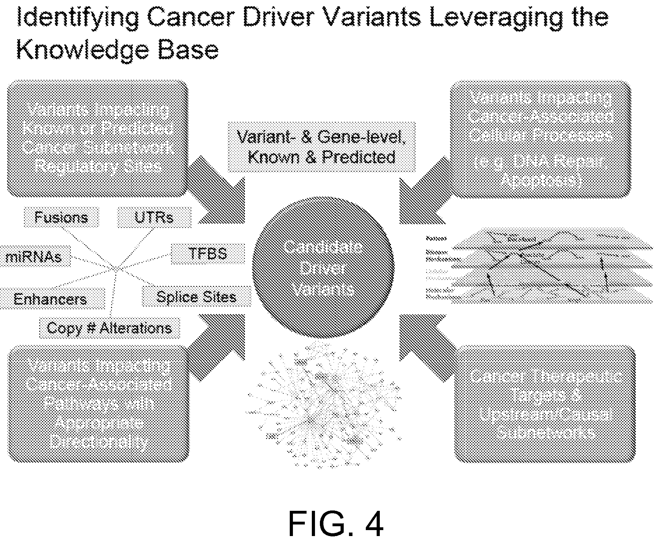

**Figure 4** depicts a knowledge base being utilized to identify cancer driver variants.

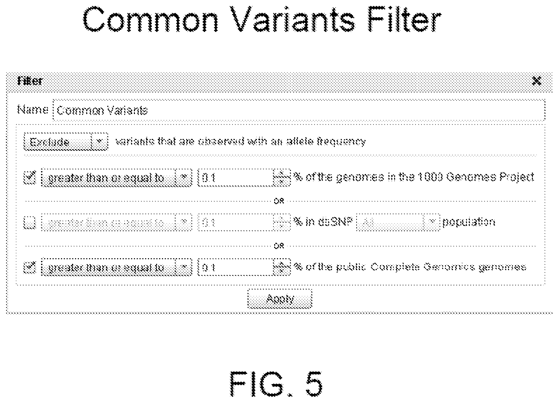

**Figure 5** depicts the common variants filter in one embodiment. In this embodiment, the common variants filter is able to filter variants based on their frequency(ies) in one or more databases of variants. This allows a fast and convenient mechanism for users to filter (i.e. mask or unmask) variants within a variant data set that have been observed to occur at, above, or below a given frequency in a given population.

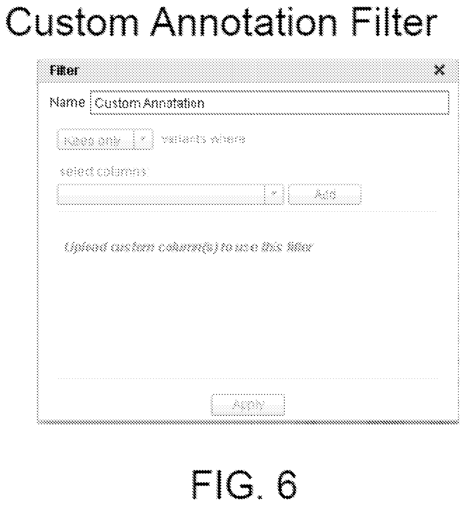

**Figure 6** depicts one embodiment of a Custom Annotation variants filter user interface. In some embodiments of the invention, users can create custom filters based on alphanumeric annotations of the variants in the variant dataset, finding variants where the "Chromosome" annotation column equals "X", for example, would be equivalent to a physical location filter used to identify variants on the X chromosome. Also in some embodiments, users can import custom columns into the variant data set and can apply the custom annotation filter to filter on the annotations present in these custom columns. This filter can be used with columns of imported expression data from RNA-Seq, proteomics, or microarray studies, for example, to identify variants that are present on exons expressed at greater than or equal to a given level, or to filter for variants that occur in regions identified in chromatin immunoprecipitation or methylation studies.

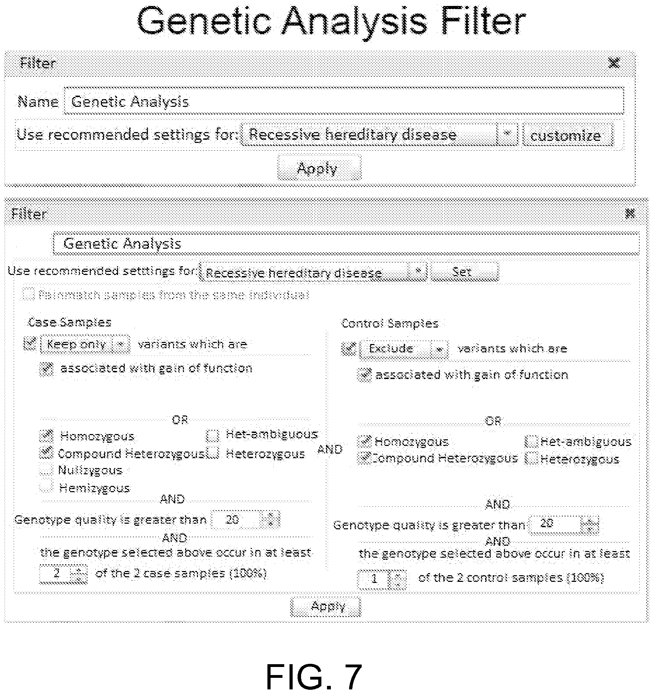

**Figure 7** depicts one embodiment of a Genetic Analysis filter user interface, allowing for adjustment of stringency by altering (a) the case and/or control zygosity and/or (b) the case and/or control variant quality or genotype quality, and/or (c) the number or fraction of case samples in which the variant (i) occurs with said case zygosity and case quality and/or (ii) affects the same gene, and/or (iii) affects the same network within 1 or more hops, and/or (d) the number of control samples in which the variant (i) occurs with said control zygosity and control quality and/or (ii) affects the same gene, and/or (iii) affects the same network within 1 or more hops. The interface to accomplish (ii) and (iii) are not shown here, but are readily accomplished in the current invention by modifying, for example, the text at the bottom to "the genotypes selected above [occur|affect the same gene|affect the same network (1 hop)] in at least [112] of the 2 case samples (100%)". The top box shows an example of a simplified Genetic Analysis filter user interface, which could be expanded to the more complex and richly featured Genetic Analysis filter displayed at the bottom by clicking the Customize button.

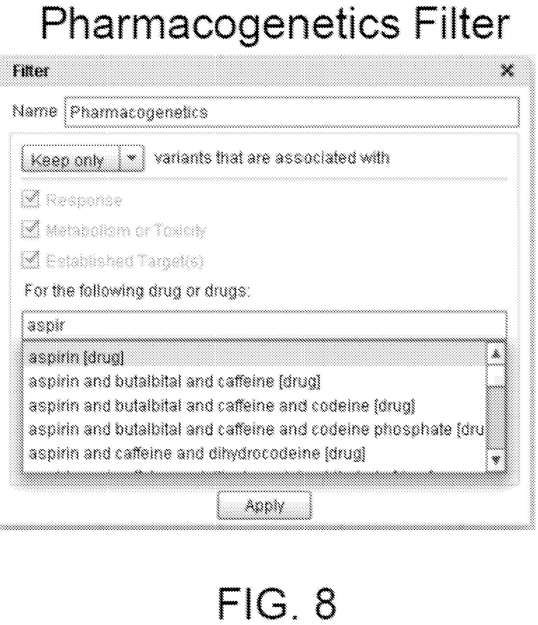

**Figure 8** depicts an embodiment of a Pharmacogenetics filter user interface. This filter, in communication with a knowledge base of curated biomedical content structured with an ontology, can apply structured biomedical information related to one or more drugs or drug targets to rapidly identify variants that are observed or predicted to impact drug response, drug metabolism, drug toxicity, or impact the targets of one or more drugs. In a preferred embodiment, the default behavior of the filter is to identify variants meeting one or more of these criteria in relation to any drug, with an optional ability to filter to particular drugs or drug targets of interest using an autocompletion widget which shows the user with each keystroke matches within the ontology to biological information of interest, in this case drugs, drug targets, and their established synonyms, where applicable. Like other filters, the pharmacogenetics filter can be configured to exclude (i.e. mask or remove the variants that meet the filter criteria), keep only (i.e., mask or remove all variants that do not meet the filter criteria), or add (i.e. unmask or add back all variants that meet the filter criteria) as part of the filter's operation.

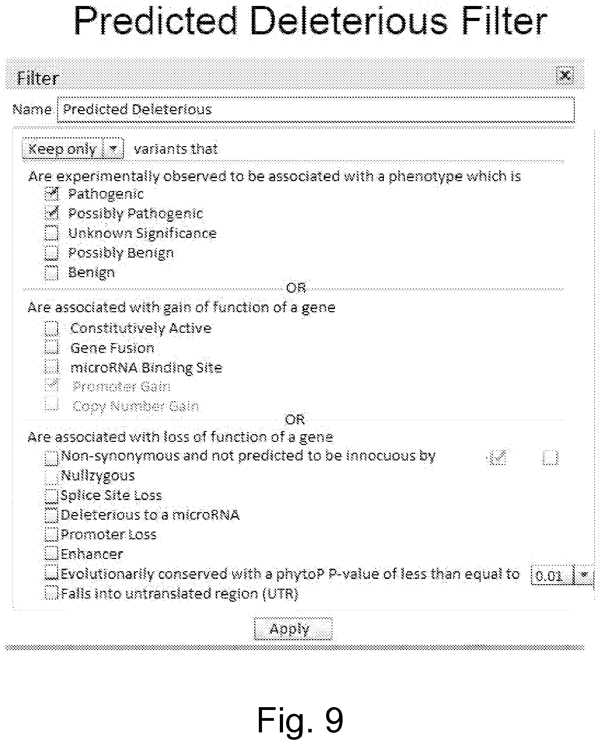

**Figure 9** depicts one embodiment of a Predicted Deleterious filter user interface, allowing the user to conveniently configure the stringency of the filter which will mask or unmask variants in the data set based on whether they are in selected pathogenicity categories of interest based on a pathogenicity annotator, whether they are predicted or observed to be associated with a gain of function of a gene, or whether they are predicted or observed to be associated with the loss of function of a gene. Like the other filters, the Predicted Deleterious filter can interact with other upstream and downstream filters, receiving a variant data set and optionally one or more masks from previous filters and masking or unmasking variants within the dataset based on the filter settings.

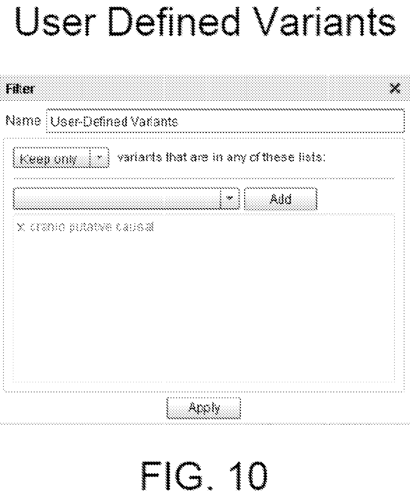

**Figure 10** depicts one embodiment of a User-Defined Variants filter user interface. In some embodiments of the invention, users can save user-defined lists of genes and/or variants, and recall those lists from a computer system for use in an instance of the user defined variants filter. In this non-limiting example, the user has recalled a set of putative causal variants from a study, and is applying the user defined variants filter to "keep only" variants that are in this list. This has the effect of masking or removing all other variants that are not present on the "cranio putative causal" variant list.

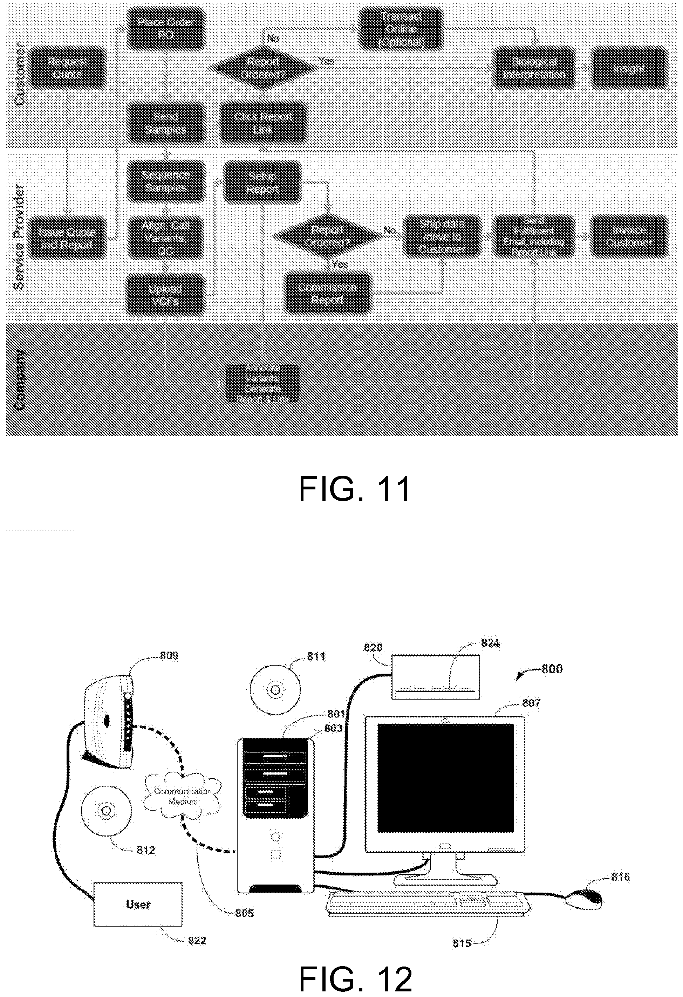

**Figure 11** depicts an example flow chart for providing interactive reports for biological interpretation of variants to a customer. This process involves a customer, a genomic service provider who generates variant data sets, and a provider of interactive reports for biological interpretation of variants. The quotation for the interactive report for biological interpretation of variants is provided along with the service provider's quotations for genomic services and is priced on a per-sample basis. Furthermore, the genomic service provider uploads the data set generated from a customer's samples directly to the interactive report system when the data set becomes available, streamlining the customer experience and allowing the customer nearimmediate access to the interactive report for their variant data set once it has been generated by the genomic services provider. Note that this data upload step is performed regardless of whether the customer ordered the report when she ordered her genomic services. This provides a second opportunity to transact the interactive report with the customer after the customer receives notification from the genome service provider that their data set is ready. When the genomic services have been completed and the customer's data set is ready, the genomic service provider sends the customer a link that directs the customer to the interactive report. The customer receives this link at about the same time as they receive communication from the service provider that their sequencing results are available.
**Figure 12** is a block diagram showing a representative example logic device through which reviewing or analyzing data relating to the present invention can be achieved.

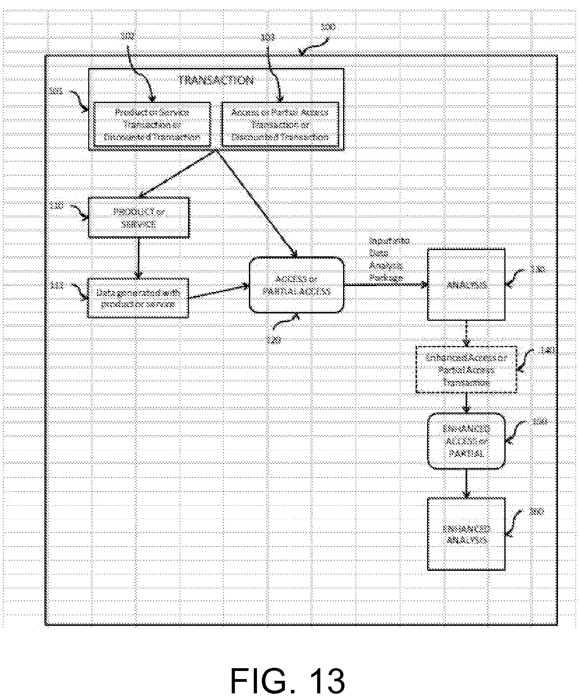

**Figure 13** depicts a flow diagram of an embodiment of a system constructed in accordance with the present invention is illustrated. The system provides a method for bundling the transaction for gaining access to a data analysis package with a transaction for a product or service that is used to generate a data set to be entered into the data analysis package for analysis.

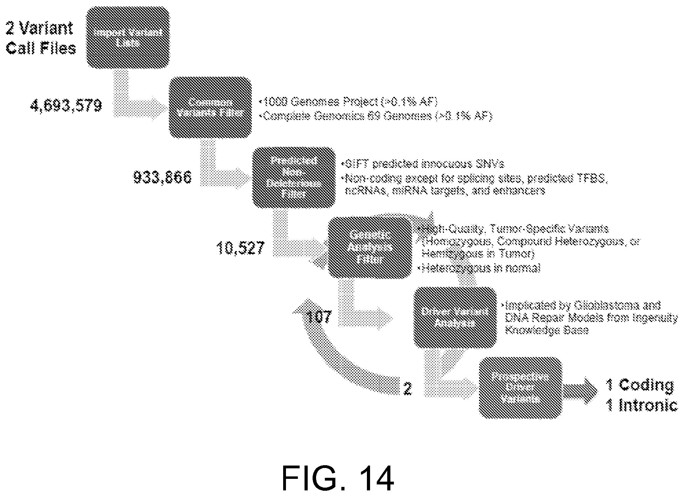

**Figure 14** depicts identification of a prospective causal variant for familial glioblastoma.

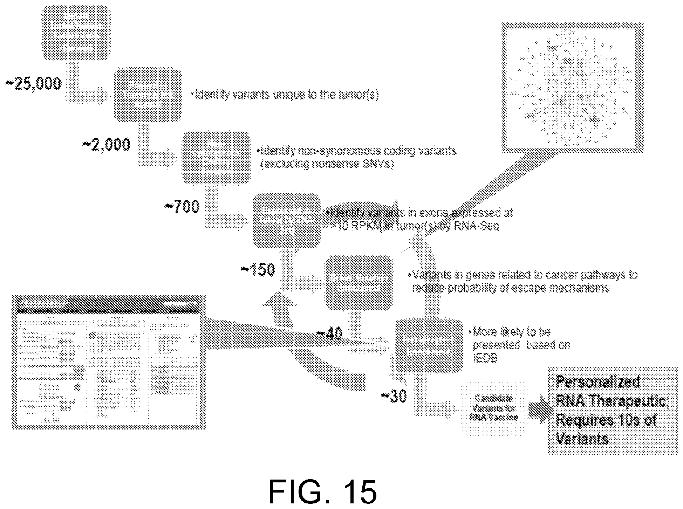

**Figure 15** depicts identification of individualized cancer RNA variants.

### DETAILED DESCRIPTION OF THE INVENTION

### Definitions

As used in the description that follows:
"Disease" means any phenotype or phenotypic trait of concern, including by way of example a disease or disease state, a predisposition or susceptibility to a disease, or an abnormal drug response. Illustrative and non-limiting examples of disease states include cancer, high cholesterol levels, congestive heart failure, hypertension, diabetes, glucose intolerance, depression, anxiety, infectious disease, toxic states, drug therapy side effects, inefficacy of drug therapy, alcoholism, addiction, trauma, etc.

A "disease-related pathway" is a series of biochemical reactions in the body that result in disease, i.e., it is a series, linear or branched, of biological interactions in the body that collectively have an effect on a disease state, e.g., initiation, progression, remission, or exacerbation. Such biological interactions, i.e., biological effects or functional relationships, are the biological processes that occur within the body, e.g., binding, agonizing, antagonizing, inhibiting, activating, modulating, modifying, etc.

"Therapy" and "therapeutic" include prophylaxis and prophylactic and encompass prevention as well as amelioration of symptoms associated with a disease state, inhibition or delay of progression of a disease state and treatment of a disease state.

"Protein" or "gene product" means a peptide, oligopeptide, polypeptide or protein, as translated or as may be modified subsequent to translation. A gene product can also be an RNA molecule.

"Findings" are the data that is used to build an information database. This data may come from public sources, such as databases and scientific publications, but it may also include proprietary data or a mix of proprietary and public data. In various embodiments, findings are derived from natural language (e.g., English language) formalized textual content according to methods outlined in greater detail below.

"Biological effect" includes the molecular effects of a given biological concept as well as the effects of such concept at the level of a cell, tissue or organism.

"Variant" means any particular change in a nucleotide or nucleotide sequence relative to an established reference nucleotide or nucleotide sequence, such reference including without limitation the public reference human genome sequences referred to as NCBI36/hg18 and GRCh37/hg19. This also includes without limitation nucleic acid modifications such as methylation, as well as abnormal numbers of copies of the nucleotide or nucleotide sequence in the genome

"Whole Genome" means the sequence comprising the substantial majority of an subject's sequenceable genome, including exons, introns, and intergenic regions.

"Whole Genome Analysis" means the interpretation of data arising from the sequencing of one or more whole genomes.

"Subject" generally means a biological organism with associated and sequence information, and optionally phenotypic information, available for analysis.

"User" means a person who is using one or more methods described herein to analyze or interpret nucleotide sequence information.

A "disease model" is a representation in an ontology of scientifically-established phenomenon implicated in progression of the disease. These phenomena include: symptoms characteristic of the disease that afflicted individuals typically present with; cellular processes, or signaling or metabolic pathways that are typically dysregulated in the disease state; variants, genes, or molecular complexes known to impact disease progression or that are targets of drugs for the disease. Phenomena in the disease model can be translated into genes from independent biomedical findings reporting a relationship between those genes and the phenomenon. Phenomena in the disease model may have an associated directionality in the disease state (either over-active or under/in-active) and how each gene from the biomedical findings has been established to impact the phenomenon (increasing/activating or decreasing/inhibiting) can be used to determine if the net effect variants in a dataset have on the gene (gain or loss of function) is consistent with promoting disease progression.

"Filtering" means annotating or altering one or more data sets. Filtering can mean keeping, adding, subtracting, or adding back data points from a data set. Filtering can mean masking one or more data points within the data set. Filtering can mean unmasking data points in a data set. In some embodiments filtering is an iterative process. In some embodiments filtering is performed with one or more filters. In some embodiments data points removed or masked by one filter are added back or unmasked by a second filter. In some embodiments filtering is performed on a list of variants. A filtered dataset can be smaller or larger than the original dataset. In some embodiments the filtered dataset comprises data points not removed from the original data set. In some embodiments a filtered dataset comprises more information than the original dataset. For example, a filtered dataset can comprise one or more of the following: the original data set, information regarding whether each data point is currently masked, information regarding whether each data point was previously masked, and information regarding previous filtering. The information regarding previous filters can be the kind of filter that was applied, any variables selected for the application of that filter, any assumptions made by the filter and or any information relied upon by the filter (e.g. information from a database).

"Physical location filter:"A physical location filter is a filter which takes a variant data set as input, wherein the variant data set comprises variant data from one or more samples from one or more individuals, that filters variants based upon the chromosome on which each variant occurs and, optionally, the physical location of each variant on said chromosome. This can be a very useful component of a filter cascade as it allows the user to identify variants that are at a location consistent with an inherited disease of interest. In one simple and non-limiting example, a physical location filter could be used to identify those variants that are located on the X chromosome for use in identifying a causal variant for an X chromosome-linked disorder. The physical location filter could accept one or more physical locations of interest from a user and identify variants that are within or overlapping with any or all of those physical locations. A logical "and" or logical "or" relationship could exist between the physical locations specified for filtering. In another embodiment, the physical locations could be selected automatically based on study design parameters specified by the user and/or inferred from the user's data set and study design. The one or more physical locations could each include a chromosome and an optional numeric coordinate range comprising a start and optional stop coordinate of interest on said chromosome. The physical location could also be specified as one or more cytological bands or band ranges (e.g. "13q14.3-q21.1"). The physical location could also be specified as a coordinate range bounded by two genetic markers, wherein said genetic markers may include one or more of the following: RFLP (or Restriction Fragment Length Polymorphism), SSLP (or Simple Sequence Length Polymorphism), AFLP (or Amplified Fragment Length Polymorphism), RAPD (or Random Amplification of Polymorphic DNA), VNTR (or Variable Number Tandem Repeat), microsatellite polymorphism, SSR (or Simple Sequence Repeat), SNP (or Single Nucleotide Polymorphism), STR (or Short Tandem Repeat), SFP (or Single Feature Polymorphism), DArT (or Diversity Arrays Technology), RAD markers (or Restriction site Associated DNA markers).

The physical location filter can mask or unmask variants from the data set based on whether the variants are within (or, optionally overlapping with) the coordinate range specified by the user and located on the specified chromosome or chromosomes. In some embodiments, the stringency of the physical location filter could be adjusted by the user, for example, selecting one or more chromosomes and coordinate ranges. In some embodiments, the stringency of the physical location filter could be automatically configured based on a desired target number of variants in the final filtered data set, and/or based on aspects of the data set and/or aspects of the study design. The physical location filter may be combined with other filters into a filter cascade to transform a variant data set into a final dataset with, for example less than 200 or less than 50 variants. In some embodiments, the function of the physical location filter can be accomplished by a Custom Annotation filter.

"Custom Annotation filter": In various embodiments of the invention, the Custom Annotation filter users can create custom filters based on alphanumeric annotations of the variants in the variant dataset, finding variants where, for example, a "Chromosome" annotation column equaling "X", would be equivalent to a physical location filter used to identify variants on the X chromosome. Also, in some embodiments, users are able to import custom columns into the variant data set and are able to apply the custom annotation filter to filter on the annotations present in these custom columns or any other columns in the data set. In some embodiments, the user interface for the Custom Annotation filter provides options to the user for filtering, which are optimized based upon the contents of a given column of interest in the data set for which a filter is being created. For example, the Custom Annotation filter could provide "greater than", "greater than or equal to", "equal to", "less than", "between", or "less than or equal to" as convenient filtering options for a numeric column. In some embodiments, the filter provides a pick list to the user for selecting among filtering options for a column with low cardinality contents. In some embodiments, the Custom Annotation filter provides filtering options such as "contains", "begins with", "ends with" and "is" for filtering on a column containing textual information. This filter can be used with columns of imported expression data from RNA-Seq, proteomics, or microarray studies, for example, to filter variants that are present on exons expressed at greater than or equal to a given level, or, for another example, to filter variants that occur in regions identified in a chromatin immunoprecipitation study, or, for yet another example, to filter variants that affect or that are within genes that are expressed at a given level in either absolute or relative terms. The Custom Annotation filter, like other filters, may mask or unmask, remove or add back variants that meet the filter criteria specified. In one embodiment, the custom annotation filter allows users to "keep only", "exclude", or "add" variants that meet the specified filter criteria. The Custom Annotation filter, like all other filters described herein, may be combined with one or more other filters into a filter cascade to transform a variant data set into a final dataset. In some embodiments, filters may be automatically or manually configured in combination to yield a final data set with, for example less than 200 or less than 50 variants for communication to the user.

"Expression filter": An expression filter is a filter which takes a variant data set as input, wherein the variant data set comprises variant data from one or more samples from one or more individuals, that filters variants to "keep", "exclude" or "add" variants based upon the degree to which the exon, transcript, gene, protein, peptide, miRNA, non-coding RNA or other biological entity is expressed in a given sample. In some embodiments, the expression filter operates on a differential expression data set that contains relative expression values from two or more samples. In some embodiments, expression values for various samples are able to be pre-loaded into a database for use by the expression filter. In some embodiments, said database is a knowledge base structured according to an ontology. In some embodiments, the expression filter enables a user to import one or more expression data sets, for example from microarray, RNA-Seq or proteomic studies. In some embodiments, the data sets imported by the user correspond directly to the invididuals and samples represented in the variant data set. In some embodiments, expression filter is accomplished by a custom annotation filter. The Expression filter, like all other filters described herein, may be combined with one or more other filters into a filter cascade to transform a variant data set into a final dataset. In some embodiments, filters are automatically or manually configured in combination to yield a final data set with, for example less than 200 or less than 50 variants for communication to the user.

Unless otherwise specified, "include" and "includes" mean including but not limited to and "a" means one or more.

### Obtaining Genomic Information

Researchers and clinicians are able to obtain large amounts of genomics information from subjects. Generally a subject can be any biological organism with a genome. The subjects can be humans, e.g. a subject person who pays to have her genome sequenced. The subjects can be patients, e.g. patients with a suspected genetic disease. The subjects can also be research subjects, e.g. apparently normal individuals or individuals with a phenotype or disease of interest. The subjects can also be animals, e.g. research animals or domesticated animals. The subject can also be a bacteria or a plant. In some cases the subject is an artificially produced series of nucleotides. In some cases genomics information is obtained from multiple subjects. In some cases genomics information is obtained from related subjects.

In various embodiments the present invention allows for the analysis and interpretation of genomics data. To use the system a user can obtain a genomic data set or multiple data sets. The data can be purchased or given to the user, but typically the user will be a researcher or clinician who performing a biological experiment or diagnosis. The data can be data which is extracted or outputted from software. For example, the data can be a data file that is generated from a sequencing experiment. The system can, in some embodiments, accept data from multiple sources, for example from multiple users or across multiple experiments. In various embodiments, the content of the data set comprises data related to gene expression, genotyping, sequencing, single nucleotide polymorphism, copy number variation, haplotyping, genomic structure, or genomic variation. The data sets can be related to diagnostics or clinical data or the data sets can be generated for basic scientific research.

Generally genomics information is obtained through the analysis of a sample from a subject. The sample can be any material that contains some or all of the genome of the subject. For example, a blood sample, hair sample, or cheek smear can be obtained from a patient in order to analyze the genome. Multiple samples can be obtained from the same subject. In some instances a sample is obtained from cancerous tissue in a subject. In some instances a sample is obtained from the immune system of a subject. In some instances samples are obtained from the same subject at different points in time; sometimes the timing of the samples is regular (e.g. once per day or once per week), and sometimes the timing of the samples is directed by the state of a disease (e.g. a genomic sample can be taken upon the increase in symptoms of a disease or when a patient responds favorably to a drug treatment).

Several methods exist to generate genomics information by analyzing the genome. Sequencing can be accomplished through classic Sanger sequencing methods which are well known in the art. Sequencing can also be accomplished using high-throughput systems some of which allow detection of a sequenced nucleotide immediately after or upon its incorporation into a growing strand, i.e., detection of sequence in real time or substantially real time. In some cases, high throughput sequencing generates at least 1,000, at least 5,000, at least 10,000, at least 20,000, at least 30,000, at least 40,000, at least 50,000, at least 100,000 or at least 500,000 sequence reads per hour; with each read being at least 50, at least 60, at least 70, at least 80, at least 90, at least 100, at least 120 or at least 150 bases per read.

In some embodiments, high-throughput sequencing involves reversible terminator-based sequencing by synthesis chemistry. For example the Illumina's HiSeq 2000 machine can produce 200 billion DNA reads in eight days.

In some embodiments, high-throughput sequencing is based on sequential ligation with dye-labeled oligonucleotides. For example by use of technology available by ABI Solid System. This genetic analysis platform that enables massively parallel sequencing of clonally-amplified DNA fragments linked to beads.

In some embodiments, high-throughput sequencing involves the use of technology available by Ion Torrent Personal Genome Machine (PMG). The PGM can do 10 million reads in two hours.

In some embodiments, high-throughput sequencing involves the use of technology available by Helicos BioSciences Corporation (Cambridge, Massachusetts) such as the Single Molecule Sequencing by Synthesis (SMSS) method. SMSS allows for sequencing the entire human genome in up to 24 hours. This fast sequencing method also allows for detection of a SNP nucleotide in a sequence in substantially real time or real time. SMSS is powerful because, like the MIP technology, it does not require a pre-amplification step prior to hybridization. SMSS does not require any amplification. SMSS is described in part in US Publication Application Nos. 2006002471 I; 20060024678; 20060012793; 20060012784; and 20050100932.

In some embodiments, high-throughput sequencing involves the use of technology available by 454 Lifesciences, Inc. (Branford, Connecticut) such as the Pico Titer Plate device which includes a fiber optic plate that transmits chemiluninescent signal generated by the sequencing reaction to be recorded by a CCD camera in the instrument. This use of fiber optics allows for the detection of a minimum of 20 million base pairs in 4.5 hours.

Methods for using bead amplification followed by fiber optics detection are described in Marguiles, M., et al. "Genome sequencing in microfabricated high-density pricolitre reactors", Nature, doi: 10.1038/nature03959; and well as in US Publication Application Nos. 20020012930; 20030058629; 20030100102; 20030148344; 20040248161 ; 20050079510, 20050124022; and 20060078909.

In some embodiments, high-throughput sequencing is performed using Clonal Single Molecule Array (Solexa, Inc.) or sequencing-by-synthesis (SBS) utilizing reversible terminator chemistry. These technologies are described in part in US Patent Nos. 6,969,488; 6,897,023; 6,833,246; 6,787,308; and US Publication Application Nos. 20040106130; 20030064398; 20030022207; and Constans, A., The Scientist 2003, 17(13):36.

In some embodiments, high-throughput sequencing of RNA or DNA can take place using AnyDot.chjps (Genovoxx, Germany). In particular, the AnyDot-chips allow for 10x - 50x enhancement of nucleotide fluorescence signal detection. AnyDot.chips and methods for using them are described in part in International Publication Application Nos. WO02/088382, WO03/020968, WO03/031947, WO2005/044836, PCT/EP05/105657, PCT/EP05/105655; and German Patent Application Nos. DE 101 49 786, DE 102 14 395, DE 103 56 837, DE 10 2004 009 704, DE 10 2004 025 696, DE 10 2004 025 746, DE 10 2004 025 694, DE 10 2004 025 695, DE 10 2004 025 744, DE 10 2004 025 745, and DE 10 2005 012 301.

Other high-throughput sequencing systems include those disclosed in Venter, J., et al. Science 16 February 2001; Adams, M. et al, Science 24 March 2000; and M. J, Levene, et al. Science 299:682-686, January 2003; as well as US Publication Application No. 20030044781 and 2006/0078937. Overall such systems involve sequencing a target nucleic acid molecule having a plurality of bases by the temporal addition of bases via a polymerization reaction that is measured on a molecule of nucleic acid, i e., the activity of a nucleic acid polymerizing enzyme on the template nucleic acid molecule to be sequenced is followed in real time. Sequence can then be deduced by identifying which base is being incorporated into the growing complementary strand of the target nucleic acid by the catalytic activity of the nucleic acid polymerizing enzyme at each step in the sequence of base additions. A polymerase on the target nucleic acid molecule complex is provided in a position suitable to move along the target nucleic acid molecule and extend the oligonucleotide primer at an active site. A plurality of labeled types of nucleotide analogs are provided proximate to the active site, with each distinguishably type of nucleotide analog being complementary to a different nucleotide in the target nucleic acid sequence. The growing nucleic acid strand is extended by using the polymerase to add a nucleotide analog to the nucleic acid strand at the active site, where the nucleotide analog being added is complementary to the nucleotide of the target nucleic acid at the active site. The nucleotide analog added to the oligonucleotide primer as a result of the polymerizing step is identified. The steps of providing labeled nucleotide analogs, polymerizing the growing nucleic acid strand, and identifying the added nucleotide analog are repeated so that the nucleic acid strand is further extended and the sequence of the target nucleic acid is determined.

In one embodiment, sequence analysis of the rare cell's genetic material may include a four-color sequencing by ligation scheme (degenerate ligation) (e.g., SOLiD sequencing), which involves hybridizing an anchor primer to one of four positions. Then an enzymatic ligation reaction of the anchor primer to a population of degenerate nonamers that are labeled with fluorescent dyes is performed. At any given cycle, the population of nonamers that is used is structure such that the identity of one of its positions is correlated with the identity of the fluorophore attached to that nonamer. To the extent that the ligase discriminates for complementarity at that queried position, the fluorescent signal allows the inference of the identity of the base. After performing the ligation and four-color imaging, the anchor primer:nonamer complexes are stripped and a new cycle begins. Methods to image sequence information after performing ligation are known in the art.

In some embodiments of the invention the genomics information is obtained by a user or customer. The genomics information can be transmitted via a network to an entity which receives the genomics information, analyzes the information, and transmits analysis results back to the user or network. In some embodiments only a subset of the genomics information is transmitted for analysis. Once the genomics information is obtained or transmitted over a network it can be stored electronically.

### 3. Identification of Genomic Variation

The variation in the genomic information is useful to identify because it may be indicative of the cause of phenotypic variation among subjects - one theory being that invariant regions of the genomes of normal subjects are likely important for coding essential components necessary for the development and survival of those subjects. The variants may account for the normal phenotypic differences between people and or the variantsmay account for disease related variations.

Once genomic information is obtained from a subject that genomic information can be investigated to determine where the subject's genome is different than a standard or control genome or genomes. In some instances, the genomic information comprises a genome or partial genome. These areas of differences are referred to as "variants." The variants can be single nucleotide differences or can be longer stretches of the genome, for instance more than 10, 100, or 1000 base pairs or longer. A variant can also comprise a deletion on one or more chromosomes. A variant can also comprise an insertion on one or more chromosomes. A variant can comprise an inversion or translocation. In some instances a variant comprises a region of homozygosity. In some instances a variant comprises a repeated sequence in the genome, for example one or more trinucleotide repeats (e.g., one or more CAG repeats or one or more CGG repeats). In some instances, variance comprise a difference in the number of repeat sequences. In some instances a variant is a SNP or a SNV. In some instances a variant exists on mitochondrial genetic material, plasmid genetic material, or a chloroplast genetic material. In some instances, a variant is in a specific chromosome, such as a se chromosome. In some instances, a variant is in a specific location within a chromosome.

In some instances, system and methods described herein are applied to find and investigate variants in a transcriptome or partial transcriptome.So, in some instances, a variant is in a mature mRNA, rRNA, tRNA, or non-coding RNA.

In some instances a variant exists on an artificially produced nucleotide sequence. Accordingly, in some embodiments the methods and systems disclosed herein can be used to analyze samples containing an artificially produced nucleotide sequence.

The variants can be identified by comparing the genomic information to a database of previously collected genomic information. Alternatively or in combination, the genomic information can be compared to samples collected coincident with a test sample to identify variants. Alternatively or in combination, multiple samples can be collected from a single subject. For example, genomic samples from a family could be collected. How these samples differ from a database of a large number of previously collected samples can inform the researcher of the variation from the larger population. The genomic samples from the family can also be compared to one another to determine the variation between the samples. For another example, a genomic sample of cancerous cells and a genomic sample of non-cancerous cells can be collected from a single subject. The variants between the multiple genomic samples from a single subject can be determined, and optionally compared to previously collected genomic information or to family members. The genomic comparisons can be performed statistically to determine the variants in a genomic sample.

### 4. Analysis of Variants

From a given sample or sample it is likely that many variants will be found, but only some of the variants will be relevant to the user (e.g. variants related to a disease). Accordingly, there exists a need for analyzing the importance of variants.

As described by the systems and methods herein variants can be analyzed. The methods and systems for the analysis of variants can be used to sort or filter variants to focus a users attention on the potentially relevant variants. Automated methods and systems for insuring that a user is presented with a tractable amount of data are presented.

### A) Algorithmic Analysis of Variant Properties

Variants identified in genomic information can be investigated using algorithms, for example to predict how the variant may function, how a variant may exert a biological outcome, or to determine whether a particular variant is associated with a particular phenotype. Various algorithmic methods can be used to analyze variants. For example, the following can be used alone or in combination to analyze variants: SIFT, PolyPhen, PolyPhen2, PANTHER, SNPs3D, FastSNP, SNAP, LS-SNP, PMUT, PupaSuite, SNPeffect, SNPeffectV2.0, F-SNP, MAPP, PhD-SNP, MutDB, SNP Function Portal, PolyDoms, SNP@Promoter, Auto-Mute, MutPred, SNP@Ethnos, nsSNPanalyzer, SNP@Domain, StSNP, MtSNPscore, or Genome Variation Server. These algorithms all attempt to predict the effect a mutation has on protein function/activity. The predictions of these algorithms can be outputted to a user. Alternatively the predictions of the algorithms can serve as part of a system for sorting or filtering variants. In some instances, a variant causes a sequence change in a gene product, such as an RNA or a protein. In some instances, a variant causes differences in the transcriptional or translational regulation of a gene product. In some instances, a variant is located in a promoter, enhancer, silencer or another regulatory sequence regulating one or more genes of interest. In some instances, a variant causes a change in the splicing of a gene product. In some instances, a variant causes a change in the post-translational modification or localization of a protein, e.g. a change in phorphorylation, intercellular transport or secretion. In some instances, a variant causes a difference in the immunogenicity of a gene product.

### B) Common Variants

By comparing multiple genomic samples it is possible to determine how common individual variants are across those samples. A number or score can be assigned to a variant that represents, for example, the distribution of that variant in a given population. For example, the 1000 genome project has collected whole genomes for over 1000 human subjects. These genomes have been compared to quantify human genetic variation. Comparison to current research in the US National Library of Medicine or human reference genome revision 18 (hg18) can also be performed. Accordingly, the system of the present invention can determine how common (or the value of a commonality score) for individual variants in a sample.

Without being bound by theory, the identification of common variant may be useful in the identification of disease causing variants. For example, if an subject with a disease has a large number of variants an investigator can determine which of those variants are common in a population which does not have the disease. These common variants can be removed from consideration as disease causing variants. Alternatively, these common variants can be ranked lower in the likelihood of being a disease causing variants.

Associations between common and uncommon variants can also be determined. For example the likelihood of two or more variants appearing in a given subject in a given population can be calculated. A researcher can use the system of the present invention to determine whether, for example, a subject with a disease has an unlikely combination of variants. In some instances, haplotype information is utilized in the analysis in order to, for example, determine the likelihood of carrying two variants simultaneously.

### C) Associating Variants with Information

Variants identified from a subject, and regions of the genome around or associated with the variants, may have already been studied to some degree. A researcher or clinician will be motivated to collect and analyze the previously known information related to variants identified in a sample, for example information in the scientific literature. Collecting this information can be time consuming for all of the identified variants. The collection may also be difficult because the literature may be inconsistent in using terms to describe a property which may be associated with a variant. The researcher or clinician may be left with an intractable amount of information to sift though in a reasonable timeframe. Accordingly, described herein are methods and systems for identifying information from the scientific literature associated with genomic variants. For example, once a variant is located in the genome and associated with a particular gene an investigator will wish to learn as much as possible about that gene, the protein it may encode, the pathways that the protein is involved in, and any diseases known to be affected by that pathway. This knowledge can help the researcher or clinician determine whether the variant is likely to be related to a disease or phenotype of interest. So for each variant a researcher or clinician could use the vast expanse of published scientific literature to attempt to determine whether a variant is likely to be associated with a disease of interest, and in some embodiments the present invention has methods and systems for expediting this process. In other embodiments the methods and systems herein are useful for narrowing down which variants a clinician or researcher should pay attention to by sorting or filtering the phenotypes according to which are most likely to be of interest to the researcher or clinician.

Variants can be investigated by comparing the variant to information known about the variant's particular region on the genome. For example, if it is known that a variant exists in a genomic region known to encode a particular protein or regulate the expression of a particular protein, then that variant can be linked to that protein, any disease associated with that protein, any pathways that protein may function in, any drugs known to target the protein, and so on. Because the variants can be located across the genome the amount of information that might be associated with the variant is very large. In order to make the comparison of a large number of variants to the huge amount of biological data available various computerized systems and databases can be used.

The number of variants in a given sample may be very large, e.g. more than 1,000, 5,000, 10,000, 25,000, 50,000, 100,000, 500,000, 1,000,000 or more. A researcher or clinician may wish to narrow down or prioritize the number of variants to learn about. Filters can be used to sort the variants.. In some instances, the application of one or more filters identifies less than 500, 200, 100, 50, 30, 10, 5 or fewer variants for further inquiry and output the one or more identified variants to a user. For example, a researcher can obtain a sample from a patient with a disease. The researcher can then obtain the whole genome sequence. The researcher can then identify the variants in the whole genome sequence. The researcher can then use the systems and methods described herein to identify the scientific literature associated with the variants. The researcher can then sort or filter the variants by properties known to be associated with the variants. So, for example, the researcher could provide an instruction to a computer to identify variants which have a known relationship with a known property, for example a particular disease, protein, gene, pathway, or patient population. Accordingly methods and systems for sorting or ranking variants using known information, for example information in the scientific literature, are described herein.

The sequence surrounding variants can also be compared to previously collected data to predict the function of the genomic region surrounding the variant. In various embodiments genes or genomic regions close to, but not overlapping, with a variant are compared to known information. The distance between the variant and a gene or genomic region compared to known information can be a measure of how likely a variant is to effect or be related to the gene or genomic region. For example a researcher may choose to instruct a computer to select all variants in a sample located within a particular distance from a gene of interest. If too many results are retuned the researcher may decrease the distance in order to lower the number of identified variants. In some cases a computer will automatically adjust the distance between the variants and the genes of interest in order to output a predetermined number of variants.

### D) Databases Useful for Variant Analysis

Comparing the vast amount of known information to lists of variants is accomplished using the specialized databases and computer systems described herein. Accordingly, various embodiments of the invention provide systems and methods to map and/or compare user provided genomic datasets with the contents of an ontology or knowledge base. In some embodiments, a mapping and/or comparison is performed between the contents of the user provided dataset and the biological entities represented in the ontology or the knowledge base. In some embodiments, a subset of the biological entities are selected for comparison and/or mapping. A comparison may comprise an analysis of the difference between the value of a property of a biological entity in the knowledge base or ontology. A mapping may comprise the identification or matching of one or more biological entities in a user provided dataset with one or more biological entities stored in the knowledge base or ontology. A mapping may also comprise identification of a shared behavior, e.g. increase, of a property of one or more biological entities in a user provided data set and of one or more biological entities in a knowledge base or ontology. The user provided data set may comprise a variety of suitable data types known in the art, for example, gene expression, genotyping, sequencing, single nucleotide polymorphism, variants, copy number variation, haplotyping, or genomic structure. The data sets can be related to diagnostics or clinical data or the data sets can be generated for basic scientific research.

In various embodiments, information, for example scientific findings, is stored in, and accessed using one or more databases which can interact. For example, a first database can be a knowledge base ("KB") of scientific findings structured according to predetermined, causal relationships that generally take the form of effector gene (and/or product) -> object gene (and/or product) type relationships (hereinafter the "Findings KB"). In some cases, the database structure for this Findings KB is a frame-based knowledge representation data model, although other database structures may alternatively be used for structuring the scientific findings. A second database can be an ontology. An ontology is a multiple-hierarchical representation of the taxonomy and formal concepts and relationships relevant to the domain of interest, preferably organized in a frame-based format. The Findings KB and ontology are herein collectively referred to as a knowledge representation system ("KRS"). Other database structures, comprising one or more knowledge bases comprising a KRS, may be employed for representing a body of knowledge when practicing the invention. However, when an ontology is used together with other KBs to form a KRS, or solely as a KRS, the methods of the invention can utilize the taxonomy and formal concepts and relationships defined in an ontology for purposes of inferring conclusions about scientific findings which may not otherwise be readily apparent, especially where findings form part of a complex, or multi-directional series of causal events. Accordingly, provided below is a further description of an exemplary ontology that may be used to practice the invention.

The system described herein can use a structured database to organize data. In some embodiments the system comprises an ontological database. In some embodiments, an ontological database in the data analysis package comprises organized information related to the biological content of the data set. Methods and systems related to ontological databases are described in US 2011-0191286 A1, US 2008-0033819 A1, US 7,650,339, US 2004-0236740 A1, US 7,577,683, US 2007-0178473 A1, and US 2006-0036368 A1.

In various embodiments, the systems and methods described herein relate to the organization and analysis of genomic information, which can comprise information relating to genes, their DNA sequences, mRNA, the proteins that result when the genes are expressed, and one or more biological effects of the expressed proteins but which can include other, related information. It will be clear to the reader that the genomics information can also be information relating to other genomics, proteinomics, metabolic and behavioral information, as well to other biological processes and to biological components other than proteins and genes, such as cells, including, e.g., the biological effects of cells. An example of an ontology structure stores its contents in a frame-based format, which allows searching of the ontology to find relationships between or to make inferences about items stored in the ontology. In this illustrative ontology, the primary organizational grouping is called a class. A class represents a group of things that share similar properties. For example, in the ontology described herein, one class is human cells, which class includes lung cells, skin cells, brains cell and so on. Each of the members of a class is an "instance" of that class, which instances represent single items or elements belonging within the specified class. Thus, an subject blood cell is an instance of the class of human cells.

The relationships between different instances in the ontology are defined by "slots." Slots can be thought of as the verbs that relate two classes. For example, pancreatic Beta cells have a slot, "produce," linking them to insulin. A "facet" represents more detailed information about a "slot" and can in some cases restrict the values that a slot can have when related to specific instances of a class. The slots and facets define and structure the taxonomic relationships and partonomic relationships between classes.

When scientific findings are entered into the ontology, each finding is separated into its discrete components, or "concepts." So, for example, in the finding: "Human Bax protein accelerated the death by apoptosis of rat dorsal root ganglion ("DRG") neurons after infection with Sindbis Virus," each of the following bracketed phrases is a concept: [Human Bax protein] [accelerated] the [death] by [apoptosis] of [rat] [DRG neurons] after [infection] with [Sindbis Virus]. The actor concepts are the physical biological components of the pathway that cause or lead to another reaction in the pathway. In the instant example, the actor concepts are Human Bax protein and Sindbis Virus. Actor concepts are likely to be genes or gene products (including, e.g., receptors and enzymes) but can also be, e.g., other DNA sequences (including, e.g., DNA that is not transcribed or that is not transcribed and translated,) RNA (including, e.g., mRNA transcripts,) cells, and bacteria, viruses or other pathogens.

To increase ontology effectiveness, it is useful to develop a common set of terms for like things. It is a well-recognized problem in fast moving scientific fields, like genomics, for different terms to be applied by different laboratories to the same genes, proteins or other biological materials, and for terminologies to change over time as conventions develop. Thus, the storing and accessing of genomics information will preferably be organized to ensure semantic consistency. For example, data entry could be limited to a pre-set, or glossary of terms, inclusion of a scientific thesaurus that automatically converts inputted terms into accepted terms, and human review to update the thesaurus or glossary.

Regardless of the subject matter captured and described by the ontology, whether genomics or toxicology, it is necessary to examine closely the body of knowledge that comprises the subject matter so that the knowledge can be organized into the proper classes and linked by the appropriate slots and facets and finally stored in a form that will allow the contents and the relationships contained in the ontology to be properly represented, searched, accessed and maintained.

The selection of sources for the information or "facts" that will be included in the ontology and the methods used to digest those sources so that the facts can be supplied to the ontology in proper form are described inthe commonly assigned US patents : (1) 6,772,160; (2) 6,741,986; and (3) 7,577,683.

Scientists who read the articles that comprise a data source for the ontology may abstract the facts contained in those articles by filling in fact templates. An abstracted fact refers to a fact retrieved from an information source that is rewritten (e.g., by using a template) in the computational information language of the ontology. A completed fact template is called an instantiated template. The contents of the instantiated templates are placed in the ontology. The type and format of these fact templates are dictated by the content and structure of the ontology. The information contained in these facts are also stored in the Findings KB, which, as mentioned above, is used to store scientific findings. Although all information in the Findings KB is also contained in the ontology, it may be advantageous to use the Findings KB when specific findings are later retrieved as this can facilitate computational efficiency for searches of multiple findings where information about the classification of, e.g., the effector and/or object in the finding within the ontology is not needed.

Each type of permitted fact of the ontology can also be associated with a fact template that is created to facilitate the proper entry of the information or data comprising that particular type of fact into the ontology. These fact templates are presented to scientists as they abstract information from the sources. Systems described herein for the generation of an ontology and/or knowledge base may provide computer interfaces for data entry. For example, pull-down menus within a template may present an operator of the system with the appropriate classes, slots and facets for the particular fact type.

The process of abstracting information is called structuring knowledge, as it places knowledge into the structure and architecture of the ontology. The method for structuring the knowledge is based on formalized models of experimental design and biological concepts. These models provide the framework for capturing a considerable portion of the loosely articulated findings typically found in academic literature. The specific level of experimental results that is of greatest value to a user of the systems described herein, for example an industrial and academic scientist, can be particularly targeted for capture. For example, in the field of genomics, knowledge that focuses on the effects that both perturbation to genes, gene products (RNA and proteins) and small molecules and various physical stimuli have upon biological systems can be singled out. These perturbations and stimuli form the backbone of an exemplary ontology and provide the necessary framework for developing a more sophisticated representation of complex biological information.

Examples of the types of facts and biological relationships that can be translated into the ontology are: a) an increase in the amount of Fadd protein increases apoptosis; b) a decrease in Raf levels increases activation of Rip2; and c) the allele delta32 of CCR5, compared to the wild-type allele, decreases HIV transmission. In some embodiments, biological systems are defined in terms of processes and objects. Discrete objects are physical things such as specific genes, proteins, cells and organisms. Processes are actions that act on those objects. Examples of processes include phosphorylation, which acts on discrete objects such as proteins, and apoptosis, which acts on cells. Perturbation of an object can have an effect on a process or on an object. Using these concepts of objects and processes, the information in the ontology may be represented by a variety of fact types.

As mentioned above, templates are associated with each fact type. In some embodiments, there are five template types used for fact entry into the ontology. The corresponding fact types may be described as observational facts, comparison facts, case control facts, case control modifier facts, or case-control comparison facts. Of course, the structure and variety of fact types depend on the field of knowledge of the ontology, all of which will be known to those skilled in the art.

Examples of each of the aforementioned fact types of some embodiments follow. Observational facts (OFs) are observations about something. An example of an OF is "Tyrosine phosporylation of INRS-1 was observed." Comparison facts (CFs) compare a property of one thing to a property of another thing. An example of a CF is "The size of a lymphocyte in one organism is greater than the size of a lymphocyte in another organism." Case control facts (CCFs) describe an alteration in something which causes changes to a property aspect of something. An example of a CCF is "Mouse-derived Brca-1 increased the rate of apoptosis of 293 cells." Case control comparison facts (CCCFs) compare the effect that something has in a first fact to the effect that something has in a second fact. An example of a CCCF is "Fas increases total apoptosis of 293 cells with Brd4 (introduced by vector transformation) more than it increases total apoptosis of 293 cells without Brd4. "Case control modifier facts (CCPMFs) express an alteration in something that causes changes to a property of a modifier of a process. An example of a CCPMF is "Mouse-derived BRCA-1 increased the rate of the induction of 293 cell apoptosis."

In some embodiments, a fact verification scheme includes a natural language display of the fact derived from the template so that a scientist can verify, by reviewing the natural language representation of the structured fact entered into the template, whether the fact entered into the template was the fact as intended.

Alternatively, or additionally, information is extracted automatically by use of a computer to "read" and analyze papers and to extract data therefrom for inclusion in the ontology. In these embodiments, a natural language (e.g., English) source text is first interpreted using computational linguistics to determine, to the extent possible, the precise meaning of the "fact" contained in the natural language source. After this "fact" has been determined, it may be reviewed and then abstracted according to an automated procedure, manual procedure (i.e., human involvement) or a combination of both. In some embodiments, a combination manual and automated procedure is used to verify that the fact extracted from the source text is both a fact of interest, that it accurately reflects the intended meaning of the source text, and that it is appropriately structured for storage in the ontology. The data sources are not restricted to journal articles. Other data sources include, e.g., public databases, private databases, and proprietary data such as confidential data developed within and confined to a particular laboratory.

Findings information may come from informal sources, as well as the more formalized documents and publication sources discussed above. For example, findings may be extracted using a network search tool that searches a network and then attempts to extract information contained in pages that seem to be about a biological concept of interest (e.g., a web-crawler that searches over the internet). Alternately, or additionally, a search engine may be used to scan corporate email, discussion groups, PowerPoint presentations, etc., to try to identify and then extract information relating to biological functions. Of course, one should expect a lower quality of results from these sources, both because the data parsing would be automatic, there would likely be higher error rates than manually entered content, and the content sources will more likely be informal or invalidated discussions, rather than peer-reviewed journals and the like.

Findings need not be limited to literature-based private or public information. For example, findings could include findings derived from, e.g., a company's microarray chip experiments. In this case, the array data could be reviewed to try to identify which genes are being co-expressed and/or co-regulated, from which a "A<-->B" relationship could be deduced. These findings could then go into the KB directly or into a graph structure directly. The data may also include findings that scientists enter directly, or could be data straight from experiments (i.e. w/out interpretation by scientists). The findings acquisition process discussed above may also be useful as a tool for publication, in addition to a data extraction or entry process. Much in the way that authors need to include abstracts and indexing keywords when proposing a publication for submission, they might also be required to write down their key conclusions in "findings format". In this contemplated use, the author or a 3rd party may perform the findings extraction (e.g., as in the way the National Library of Medicine is currently responsible for approving, if not creating, the keywords associated with paper abstracts). KRS technology is not required for creating a structured database. While KRS technology may be advantageous in some cases as it can simplify certain tasks in the data acquisition and data structuring process, it is also possible to create a KB using existing relational, object or XML database technology.

With data from multiple sources acquired and stored in the database, such as is described above, it is possible to determine relationships among variants, genes and gene products that previously would have been exceedingly difficult or even impossible to identify because, e.g., of the number of sources from which data are required and the use of inconsistent language (e.g., different names for the same protein are used simultaneously or over time.) So, while it may be possible for one or a small number of subjects to stay abreast of all or most publications relating to a very narrowly defined field, it is impractical to think of scouring public data sources to identify disease pathways that are related to a large number of variants without the aid of a structured database, such as is described above. Even with respect to particular variant, diseases, genes or gene products, this task can be enormously difficult and time-consuming without the aid of a structured database.

Various embodiments of the invention relate to methods and systems that group biological entities in a knowledge base or ontology. In some cases, the groupings are constructed using the methodologies to create profiles described above. The profiles can be generated using process or pathway association of the biological entities. In some cases, a biological association shared by a statistically significant set of genes in a profile or grouping will be annotated to the profile or grouping. In some cases, profiles or groupings sharing similar biological associations, such as a biological process, pathway, or tissue specific expression, will be compiled into collections of profiles and groupings. However, the underlying reason to generate a collection of profiles or groupings is not limited to biological association. Collections of profiles and groupings can be formed using other shared characteristics formulated by the knowledge base or the ontology. In some cases, the shared characteristics can be formulated by other sources than the knowledge base or the ontology, such as the administrator of the system or a user. Alternatively, the collections can be generated without any apparent reason or at the will of a user (e.g. user's favorite profiles and groupings).

Various embodiments of the invention provide methods and system to filter biological entities in an ontology or knowledge base to a subset of entities. In some cases, preformed groups or profiles or collections thereof are used to filter the biological entities to a subset. In some cases, the system allows for a user to generate a filter or a set of filters through a user interface. Alternatively, the system may provide preconfigured filters or sets of filters. In some cases, the system uses input provided by a user to generate, choose and/or modify preconfigured filters. In various embodiments, sequence variants in a user provided data are filtered through criteria described herein, providing a manageable set of variants to a user. In many cases, filters are applied in context of the purpose of the study where the data sets have originated.

A "profile" may include information about, and be defined according to concepts such as a particular combination of genes or gene products that appear to act in a biologically coordinated manner, e.g., form all or part of a disease related pathway, cells and/or cellular components, anatomical parts, molecular, cellular or disease processes, and the relationships between them. A "profile" as used in this discussion refers to a subset of the data contained in the database that is defined according to criterion(s) suited to the researcher's goals. As such, criteria (or a criterion) means any attribute of a profile that is determined, at least in part, by the researcher's needs. This may include criterion defined in terms of one or more biological concepts, the size of the profile (e.g., graph size), or the findings connectivity in the profile. It should therefore be remembered that the examples of profile criteria enumerated below are intended only as exemplary embodiments of profile defining criteria. In general, it is understood and indeed expected that profile defining criteria will vary from one application of the invention to another since a profile structure according to the invention is driven by research goals.

Thus, the effectiveness of one or more profiles in communicating information depends upon the criterion (or criteria) used to define the profile(s), which naturally depends upon the particular scientific goal for which information is being sought. For example, if it is believed that information relating to a particular cellular process would be highly informative of a targeted pathway, then findings relating to this cellular process would be a factor to consider when selecting a profile criterion. In another situation, the source of the findings (e.g., tissue type) or the size of the profile (e.g., the size of a graph structure illustrating the profile) may be effective profile selection criterion.

Various aspects of the analysis of the present invention generate computational models for biological pathways. These models, referred to as "profiles", become tools for interrogating and interpreting genomic data sets, e.g. variants. They are constructed from findings in the KB, and consist of sets of gene (product) abstractions, together with their known macromolecular interactions, and various biological processes the KB asserts the genes to play roles in.

In an exemplary embodiment, gene abstractions comprise official LocusLink gene symbols to which are mapped known instances of gene and gene products in the KB, potentially from both human and nonhuman species. The intermolecular interactions consist of specific instances of effector gene (product) → object gene (product) relations; the mapping of gene (product) instances to the more abstract gene symbols thus allows inferred generalized effector gene symbol → object gene symbol relationships (as discussed earlier). Borrowing concepts from graph theory, the available genes and gene interactions can be represented computationally as collections of "nodes" (for genes) connected by directed "edges" (for interactions), with various properties being associated with each node (e.g. gene properties), and various properties associated with each edge (e.g. molecular process types, direction of process changes, number of findings/publications asserting the interaction, etc). In addition, various properties can be associated with the entire profile, including for example, biological processes, the number of genes in the profile, the method of construction, etc.

The ability to associate a rich set of node, edge, and graph properties with profiles provides opportunities to apply a variety of selection criteria on the profiles: Criteria applied during selection of nodes and/or edges can provide diversity in the composition and structure of the profiles produced. Criteria applied after profile construction but prior to scoring against user provided data can reduce unproductive false 'hits' or provide a more focused analysis. Criteria applied after profile construction and after scoring against user provided data can provide additional ranking of profiles (by criteria other than scoring) for review by researchers. In various embodiments, the methods and systems described herein use filters to apply criteria on profiles, groupings or collections thereof, to rank, emphasize, deemphasize or eliminate said profiles, groupings or collections thereof.

Profile generation can begin with a dynamic pre-calculation of a master graph (or network) that fits a certain set of criteria. The criteria may be pre-set by the system or defined by the user and may pertain to any category in the database, e.g., genes or gene products, chemicals, protein complexes, protein families, processes, sources of findings, experimental techniques, organism context, or other criteria, e.g., genes that are absent according to the user's data. Then profiles are created from this graph based on further criteria pre-set by the system or defined by the user, e.g. genes of particular interest to the user, maximum number of nodes per profile, etc.

Conceptually, each profile is a response to a query against the KB to find networks of findings that meet the criteria. These profiles may be pre-built off of a copy of the KB to optimize performance (producing a library of pre-built profiles), or the profiles may be built directly against the KRS, so as to allow profiles to incorporate recently discovered findings as they are stored in the KB. Profiles could also be built using something of a "bootstrap approach": an initial set of profiles could be built, then tested for sensitivity in changes in further supplied data, such as expression changes, and the best profiles could be enlarged (by adding more gene members, by merging profiles, or by otherwise changing the criteria that define the profile model), and the sensitivity test repeated.

In an exemplary embodiment, the profiles are generated by first extracting a subset of the KB findings and then converting findings into a large graph data structure. This is essentially a simplified version of the KB that is amenable to high-performance graph data structure operations. Part of this simplification may include converting findings from a literature-based representation, where each finding represents a result from a performed experiment, to a biology-based representation, where each finding represents a conclusion about biology. The profile generation algorithm can then process this graph to produce a collection of subnetworks (profiles) that may be analysis-specific, e.g., user-provided biological data, such as sequencing, variants, or array expression data, input as parameters to a profile generation algorithm, and that match input criteria. Examples of input criteria are the size of the profile (number of nodes in each profile), whether they show differential results in the user's data sets or otherwise flagged as of interest to the user, the processes involved (e.g., "activation + cleavage" or "phosphorylation"), and/or the source of a finding (e.g., only observed in human liver cells). Many such collections can be pre-generated given a profile generation algorithm and a set of parameters. If the profile collections are built upon a copy of the KB, they may be re-built when the KB changes (e.g. when new findings are added) to keep the profiles up-to-date. The collections may also be dynamically built, i.e., as the KB changes or as new user-provided biological data becomes available. Either configuration is contemplated and considered within the scope of invention.

Various profile generation algorithms can be used to generate the profiles described herein, such as a gene-centric algorithm. In some embodiments, the algorithm creates one profile for each gene in the KB. Each gene's profile consists of the gene that "anchors" the profile and a set of "nearby" genes that match a certain criteria. A "nearby" gene or gene product may refer to those genes or gene products that are most directly related to the anchor (or "seed") through some association defined by findings linking the gene to the anchor gene and or the number of such findings. This approach is termed "model-driven" because the profiles are based on a predefined algorithmic model. Alternatively, a "data-driven" model may be used, where the profile is not pre-generated but instead is assumed to be the dataset of interest to a user (e.g. variants) together with their known interactions as revealed by the KB. Essentially all the user genes can be connected in this manner using findings from the KB.

In some embodiments, a "nearby" biological entity, most commonly a gene or gene product, most directly related to a second biological entity, is termed to be one "hop" away from the second biological entity. In some embodiments, biological entities that are one hop away from each other are nodes connected by an edge in a knowledge base structured by an ontology. "Hops" as used herein may comprise a relationship between biological entities (including but not limited to genes/gene products) in a knowledge base structured according to an ontology. Such relationships may include, but are not limited to: "binds", "activates", or "represses".

The strength or quality of hops may be defined in a non-limiting example by degree of literature support from the knowledge base and/or prioritizing direct interactions over indirect interactions. For example, a hop is stronger if there are many representations of a particular fact in the knowledge base, and a hop is weaker if there are contradictory representations of a particular fact in the knowledge base. In another example, a hop can be stronger if a causative relationship is the source of the hop and weaker if an association is the source of the hop. In some embodiments, the number of hops can be used at least in part to determine the strength of a hop. For example, a first hop can be given more weight than a second hop, and the second hop can be given more weight than a third hop.

In the previously discussed embodiment, a hybrid model and a data driven approach are used which determines the nature of the constructed profiles based, at least in part, on a user-prescribed set of data, e.g., variants. "Gene" is used herein to describe a gene or gene product interchangeably, as it refers to biological entities represented in a knowledge base structured by an ontology or in an ontology. Profiles may alternatively be constructed using a purely model-driven approach. This approach may be regarded as "gene-centric" in nature: A pathway profile is constructed around each of the gene symbols in the KB, using each as a "seed" gene, and including other genes with which the seed is known to interact in the KB. In this way, the profiles come to represent the "interaction neighborhood" or "sphere of influence" of the seed gene. Profiles may alternatively be constructed using non-gene concepts as the "seeds". For example, a cellular process like apoptosis, can be used to select a number of genes to act as a seeds, in this case, all or some subset of the genes the KB that are implicated in apoptosis. The seed forming genes can be added to the profile, together with their known inter-molecular interactions (as edges). The profile can be expanded further by adding a desired number of "nearby" genes, once, twice or more times, adding more genes that may not be directly connected with the original seed genes. Regardless of the nature of the "seed" in the profile, profiles can be used to give further meaning to a data set, if they can be correlated with a user provided data set, such as genomic data set (e.g. variants), then the "seed" becomes the focus of interpretation.

Beyond the "seed" node and edges connecting the seed to other nodes, profiles may be constructed in a myriad of ways. Many of these approaches are driven to handle the following concerns: The complete set of macromolecular interactions represented by a KRS will usually be too large and too diverse to be compared in its entirety with a user provided data set, often with a genomic content. Hence, an algorithm is needed to "carve up" this large "macromolecular interaction space" into numerous practical-sized interaction neighborhoods to support a finer-grained probing of genomic data sets. This carving up can be done with considerable gene overlap among the different profiles to minimize the chance that a rare combination of genes might be missed. On the one hand, profiles that are modest in size can be designed so that the set of biological functions that might be ascribed to the profile are not too diverse or heterogeneous. Smaller size profiles also aid significantly in human review and interpretation. On the other hand, profiles should be sufficiently large (i.e., they should include, e.g., a sufficient number of genes) so that there will be enough statistical power when computing correlations with genomic data sets and/or with biological associations, such as molecular, cellular, organismal, and/or disease processes defined in the KB. Another consideration is the relative symmetry of a profile in the collection of genes connected to the central "seed" gene. In other words, a highly interconnected "1st tier" gene (i.e., a gene connected directly to the seed) should not swamp the profile with 2nd-tier genes (i.e., genes one step removed from the seed) because this can change the seedgene-centricity of the profile. For studies focusing on genes that are one or more hops away from a gene of interest, the profiles can be designed to allow for a desired amount of hops from a desired gene. For example, profiles can be generated including genes that are 1, 2, 3, 4, 5, 6, 7, 8, 9 or 10 "hops" away from a target gene.

An example of an alternative algorithm developed to address the above goals is referred to as a "spiral" algorithm. In this algorithm, profiles are generated from a fully-extended master graph of all known interactions. The graph is constructed from a complete set of the pair-wise macromolecular interactions held in the KB, and will naturally differ in density (i.e., connectedness among nodes) in different parts of it. For each gene or gene product concept represented by a node in the master graph: 1) Designate the gene (e.g., a random gene or a gene comprising a variant, or a gene selected by another criterion, for example one of the genes associated with a particular biological pathway) or its product as the "seed" node. 2) Add all immediate neighbor nodes (genes known to participate in interactions with the seed gene) as long as the number of findings supporting the claim that the seed and the neighbor interact is greater than 1, or stop adding if the maximum number of nodes has been reached. The elimination of interactions based on only a single finding is thought to weed out unconfirmed or weakly-substantiated findings. These are the 1st tier nodes and the connections from the seed to the nodes are 1st tier edges. 3) For each 1st tier node, compile a list of nodes and edges (besides the seed) that are neighbors of the 1st tier node, as long as the number of findings supporting the interactions is 4 or more. This increases the stringency for scientific confidence in the interactions, which as explained above is consistent with assumptions about a decrease in the degree of influence of one gene over another when there are intervening genes between them. These additional nodes and edges are considered "2nd tier" candidates. 4) Sort the 2nd tier candidate edges by decreasing findings counts. 5) After all 2nd tier edge candidates have been enumerated and sorted by the findings count, begin adding 2nd tier candidates to the profile in a round-robin fashion, picking one 2nd tier edge candidate for each of the 1st tier nodes by selecting the 2nd tier edge with the highest number of findings. 6) Repeat the round-robin edge addition in step 5) until either the number of 2nd tier edge candidates is exhausted, or the maximum number of nodes for the profile has been reached. This results in a profile based on edges with the largest number of scientific findings substantiating the interactions.

The above "spiral" approach (essentially a breadth-first search of available nodes) aims to enlarge the profile in a symmetrical fashion. Second tier edges are added from 1st tier nodes with equal opportunity (but preferentially those with more findings counts), reducing the chance that a highly-connected 1st tier node (with lots of 2nd tier edges) will swamp the profile with its connections. Thus, the sphere of influence surrounding the seed gene is optimally represented. Additional profile assembly algorithms may also be used.

The above algorithm, when applied to each gene or product in the KB, results in a profile library where a model of each gene's sphere of influence is collected. Profile libraries may be constructed which use specific edge types / molecular process criterions, cellular process types, disease states, etc (e.g. binding only, functional interactions only, or all types) when selecting from available edges. Edge directionality can be a criterion, as well, designating an upstream or downstream role to the nodes in many cases. When analyzing a genomic data set (e.g. sequence variant data set), each subject model in the profile library (or libraries) can be available to be used to interrogate the data set. In some cases, a corresponding fit between the model and the data set is computed. In some cases, the interactions defined in various model profiles can guide the data analysis. For example, "nearby" genes within a model profile that are a desired number of "hops" away from one or more "seed" genes can be considered in an analysis. These "nearby" genes can be selected to relate to the "seed" genes with a selected directionality. The net effect (the concordance of either an activating/increasing or inhibiting/decreasing effect of one gene known to be either active or inactive on the activity of other genes) of a change in the "nearby" genes on the "seed" genes can be a criterion. The net effect of a "seed" gene on the "nearby" genes can also be a criterion when analyzing the user provided data.

This approach is referred to as "model-driven". As mentioned above, a fundamentally different, "data-driven" approach to profile construction may also be performed.

Uses of the assembled profiles have focused on interrogating and interpreting large scale genomic data sets where the profiles are treated as static models. Additional uses of the profiles are also possible. For example, the pathway profiles could be fed to simulation software that could allow the dynamic behavior of the interacting genes to be explored. The process nature and directionalities (increases/decreases) of the intermolecular interactions can be used to track "what if" scenarios regarding the changes (abundance) in one or more genes in the profile and the consequences of that change on the other members of the profile. Boolean networks and Petri nets offer some technologies that might be used in such simulations. Another example of how the pathways could be used is in the generation of testable hypotheses. Computational systems could be devised to generate experimentally verifiable predictions about the molecular interactions, and perhaps even report on reagents available (e.g. mouse knockouts in some of the profile's genes) and additional information for performing the experiments. There could also be computational support for the revision/fine-tuning of the profile models to reflect new knowledge obtained from those experimental verifications.

In various embodiments, profiles are selected and ranked based on their relationship to the user supplied biological data sets, e.g. variants. For example, sequence variant data from a number of subjects sharing a disease can be analyzed. Profiles containing a large number of variants commonly shared by the subjects can be ranked higher. Ranking can be adjusted further, if the shared variants are not commonly found in normal subjects. Rankings can be adjusted further considering the statistical significance of finding the said set of variants in a given profile. Rankings can also be adjusted based on a matching between the profile and the disease based on a biological concept. Profiles can be scored by computing a P-value that ranks a profile against the user-supplied data, e.g., sequence variant data or gene expression data. In a particular application, there may be many profile libraries generated, each of which contains profiles matching the user or system specified criteria.

In some embodiments, one may develop an aggregate scoring metric that includes graph-theoretic metrics, either as a compound score or a coarser ranking for profiles that match based on the existing score. For example, for N profiles that score equally well using a first metric, rank them further based on, e.g., graph connectivity metrics under the assumption that the more connected the genes, more likely they are working together.

In another embodiment, the system could allow user annotation to indicate (hypothesized) dependencies within the expression dataset. Specifically, if users have a priori knowledge about dependencies between the genes (e.g genes comprising a variant or variants) in their experiment, the users can be allowed to include the a priori knowledge (e.g. as edge annotations, additions of new edges, or removal of edges whose evidence is hypothesized to be weak) in the set of genes to be analyzed. This feature, may require that the analysis gene sets have edge drawings (if it is desirable to display this information in graph form) which use the same semantics of directness as those underlying the profile edges, i.e., a data-driven profile can be constructed from user-supplied information. Alternatively, forms may be provided to input edges and tables provided for visual output for the edges. Thus, in addition to findings from the literature, users can add their own findings, or modify existing ones by, e.g., specifying a confidence measure. These user findings could be modifications to the KB itself or to the graph itself. Updates to the KB may use templates to enter these new findings. If these findings are added to the graph, then templates customized for graph edits may be used. This resulting data or model driven profile (or profiles, if there is more than one hypothesized dependency for a gene set) may then be used to further rank existing profiles by, e.g., doing an isomorphism comparison with model-based profiles. Thus, in some embodiments, data- or model-driven profiles are ranked against both the prior knowledge asserted in the KRS and the user's personal knowledge assumptions about the data.

The results output may be delivered to the user online as part of an integrated site that makes available all related KB applications. This can be advantageous because a number of pieces of information generated in all of the outputs is based on concepts and findings stored in the KB, which can also be made available to clients located on a network (e.g., the internet) for purposes of interrogating the KB for more detailed information related to the results. Thus, embodiments of the invention can be tightly integrated with supporting content, for example by allowing "click-thru" and "drill-down" functionality to take users from the high-level results to the detailed supporting evidence.

Biological phenomena from the KB that is associated with the collection of genes in profiles in a statistically significant fashion can be revealed. Although the 20 or 40 genes in a profile are each likely to be associated with many biological processes, the ones of most interest are those that are shared by many of the genes in the profile. To be statistically significant, the shared biological associations should occur at a frequency that is higher than that expected by chance alone. Further, a measure of statistical significance can be calculated for these associations, for example using p-values.

As an example, let's assume that Profile X has 20 genes, and of those 20 genes 12 are known (from the KB) to be associated with the cellular process "migration". The question to be answered is: could the 12 out of 20 genes linked to "migration" be explained as simply reflecting the frequency of "migration" cellular processes among the set of genes in the entire KB, or is this concentration of "migration" genes unusual. To answer this question, one would need to know the probability (p) that any randomly-selected gene in the KB will be associated with "migration". This probability can be determined by computing the distribution of KB genes across the various cell processes represented in the KB. This distribution may then be made available for quick access by the analysis software by storing the information in a database. In one illustrative example, 386 genes are linked to the cellular process of "migration" out of a total of 10,500 genes in this KB. This means the probability that any randomly selected gene will be a "migration" gene is 386 ÷ 10,500 or 0.0368. The probability of 12 out of 20 randomly selected genes being linked to "migration" may be computed using the Binomial Distribution: $P \left(k\right) = \left(\begin{matrix}n \\ k\end{matrix}\right) {p}^{k} {\left(1-p\right)}^{\left(n-k\right)}$ (1), where n is the number of randomly-selected items, k is the number of observed events of one kind, and p is the probability (frequency) of a single item being of the particular event. The $\left(\begin{matrix}n \\ k\end{matrix}\right)$ term is "n Choose k" which is equivalent to: $\left(\begin{matrix}n \\ k\end{matrix}\right) ≡ \frac{n !}{k ! \left(n-k\right) !} = \frac{1}{k !} \frac{n !}{\left(n-k\right) !}$ (2)

From the example above, p would be 0.0368. From (1), and p = 0.0368, we can calculate the probability that 12 out of a random selection of 20 genes would be linked to "migration" as: $P \left(12\right) = \left(\begin{matrix}20 \\ 12\end{matrix}\right) {0.0368}^{12} {\left(1-0.0368\right)}^{\left(20-12\right)} = 5.7567 e - 13$ (3)

It is important to note that this computes the probability of exactly 12 genes out of 20 being linked to "migration". In judging the significance of this, we are interested in the cumulative probability of 12 "or more" genes out of 20. This is computed from (1) by summing the binomial probabilities: $Significance = {\sum }_{k = k 1}^{n} \left(\begin{matrix}n \\ k\end{matrix}\right) {p}^{k} {\left(l-p\right)}^{\left(n-k\right)}$ (4), wherek1 = 12,n = 20, *p* = 0.0368.

For the "migration" cellular process, this gives the cumulative probability that any observation of 12 or more genes out of a profile of 20 occuring by chance of 1.9e-12. This is the P-value, and in this case gives 1 in 1.0e12 chance that the results are due to chance.

This test is commonly referred to as the "Fischer Sign Test", and in the some embodiments is automatically performed on a profile for any of the cellular, organismal, and disease associations linked to the genes in the KB.

Other types of results may be provided to the user, e.g. profiles annotated with drug target information by visually highlighting those genes (or variants associated with those genes) that are known drug targets (i.e. for which a targeting molecule has been found or created) or for which there is evidence that suggests that they may be good drug targets based on e.g. gene family membership. Drug target information may be integrated into the results by simply highlighting the genes on a profile diagram, or drug target information could be taken into account when scoring the profiles. The biological entities that triggered the identification of a profile can also be highlighted. Profiles can be further displayed with annotations related to unwanted side effects for a drug. Biological contexts, such as tissue specificity related to the focus of a study, can be considered in the scoring of a profile. Scoring of profiles can further be at least partially based on the number of patented biological entities in the profile.

With an ontology such as described above, it is practical to query the knowledge representation system for actor concepts, e.g., variants, genes, and gene products, related to a disease and thereby to construct a disease-related pathway that extends back several steps, and that branches out to identify overlapping disease-related pathways, as described above. Each gene or gene product in the pathway can be associated with one or more variants, and the variants from a given sample which are related to the disease related pathway can be identified.

It will be clear to persons of skill in the art that further validation may be appropriate. Such further validation, if any, can be done in an number of ways including by correlating the variants with other relevant data, such as differential gene expression data as described herein, or by use of animal models.

In general, the database is queried to identify pathways to a phenotypic trait, e.g., a disease state or a predisposition to a disease state or other phenotypic trait of interest, by constructing a query designed to produce a response, following computational analysis of the database (or ontology), that reveals all concepts that are biologically related to the phenotypic trait state or to a biological component of the body that is already known to be biologically related to the phenotypic trait. The query can also fix the number of steps removed from the phenotypic trait or other biological component.

The means for storing and accessing, genomics information and the means for computational analysis of complex relationships among the stored concepts will typically comprise a computer system, i.e., any type of system that comprises stored, e.g., digitized, data and a means to query the stored data. Such computer system can be a stand alone computer, a multicomponent computer, e.g., one in which the stored data are physically remote from the user interface, networked computers, etc. Any known means for querying the database will also be useful, e.g., software and hardware for electronically searching fields, categories or whole databases.

Thus, in one aspect, the systems and the methods described herein are used for identifying a disease associated variant by (a) providing a means for storing and accessing genomics information wherein said means permits computational analysis of complex relationships among the stored concepts; (b) querying the database to identify a disease-related pathway; and (c) identifying the biochemical reactions in the disease-related pathway whereby one or more of the actor concepts involved the biochemical reactions comprise a variant associated with the disease. The disease associated variants can further be used for diagnostic purposes. For example, a subject can be screened for the presence or other related biological properties, such as expression profiles, associated with a sequence variant found in a disease associated target.

In some embodiments, a model of transcript (e.g. gene) activity is inferred for each physical sample in the data set. A physical sample refers to the variants found in one individual's genome taken from a particular location (e.g. a tissue or a tumor) at a particular point in time (e.g. before or after therapy). Based on default (or customized) predicted deleterious filter settings, biological knowledge of gene function and structure from the database of biological information, and genetic principles, each gene in a physical sample is inferred to either have the ability to function normally, or be overactive (gain of function), or be inactive (loss of function). This permits identification of genes (and corresponding deleterious variants) with abnormal function in one physical sample that are not present in another sample from the same individual (e.g. tumor and normal tissues). This also enables causal analytics to compute the "net effect" variants within a disrupted gene have on genes (e.g. disease-implicated) that are one or more regulatory hops downstream. Further, this enables causal inferences to be made in a a whole-genome scale, given the inferred ability of each gene in the physical sample and how each gene is known to exert activating/inhibiting effects on phenomena from biomedical findings, to determine how multiple deleterious variants in multiple genes within a physical sample are inferred to impact any or every phenomena represented in the data base.

In some embodiments computer systems or logic devices are used to implement the systems and methods provided herein. Figure 12 is a block diagram showing a representative example logic device through which reviewing or analyzing data relating to the present invention can be achieved. Such data can be in relation to a disease, disorder or condition in an individual. Figure 12 shows a computer system (or digital device) 800 connected to an apparatus 820 for use with the scanning sensing system 824 to, for example, produce a result. The computer system 800 may be understood as a logical apparatus that can read instructions from media 811 and/or network port 805, which can optionally be connected to server 809 having fixed media 812. The system shown in Figure 12 includes CPU 801, disk drives 803, optional input devices such as keyboard 815 and/or mouse 816 and optional monitor 807. Data communication can be achieved through the indicated communication medium to a server 809 at a local or a remote location. The communication medium can include any means of transmitting and/or receiving data. For example, the communication medium can be a network connection, a wireless connection or an internet connection. Such a connection can provide for communication over the World Wide Web. It is envisioned that data relating to the present invention can be transmitted over such networks or connections for reception and/or review by a party 822. The receiving party 822 can be but is not limited to a user, a scientist, a clinician, patient, a health care provider or a health care manager. In one embodiment, a computer-readable medium includes a medium suitable for transmission of a result of an analysis of a biological sample. The medium can include a result regarding a disease condition or state of a subject, wherein such a result is derived using the methods described herein.

### 4. Prioritizing and Filtering Variants

For a variety of reasons user may desire to prioritize or filter a number of variants identified in a genomic sample. For example, genomics information from a patient can be obtained and a large number of variants can be identified. The researcher or clinician can sort or filter the variants according to properties associated with those variants. These properties can be, for example, related to a disease of the patient. In the end the clinician will thereby identify variants with association to the patient's disease. The clinician can then assess whether the variant is causative or whether a certain treatment regime is preferred. The systems and methods described herein identify the associations and perform the prioritization and/or filtering of the variants.

A computer can be configured to aid in the prioritization or filtering of the variants. In some cases a number of variants can be rank ordered by a computer according to properties selected by a user. For example, a user may input a genomic data set, identify the variants within that data set, select properties of interest, command a computer to identify which variants are associated with the properties of interest, and receive information in the form of a ranking of how strongly associated each variant is with the selected properties. In some embodiments a computer is configured to receive one or more genomic data sets, identify the variants within that data set, receive selections of properties of interest, and calculate an association between the property or properties of interest and each variant. The computer can be further configured to output the information in the form of a ranking or filtering based upon how strongly associated each variant is with the selected properties. Alternatively, a list of variants that are associated with the selected properties above a threshold level can be provided by the system. In some cases, a measure of association can also be provided for each variant.

In some embodiments, variants are prioritized based upon the kind of relationship the variant has with biological facts. Some relationships with facts may indicate that a variant is likely to be causative of or correlated with a disease or phenotype while other relationships might mean that a variant is less likely to be involved with a disease or phonotype. For example, variants associated with gene products that phosphorylate or activate a second gene product may be of special interest because the phosphorylation relationship is likely to be biologically relevant. Similarly, variants associated with gene products that are involved in particular pathways, processes, disease phenotypes, or biomarkers may be of particular interest. These variants could be prioritized highly. On the other hand, variants that are commonly observed in the population, are poorly evolutionarily conserved, are not expected to perturb a biological process, or whose associated gene product(s) are not associated with relevant pathways, processes, disease phenotypes, or biomarkers may have a lower likelihood of representing causal or driver variants for a phenotype of interest. Similarly, genes that have highly redundant links, i.e., are involved in multiple other pathways, may be deprioritized because as targets their disruption may be expected to disrupt a number of pathways, which may be expected to not cause a particular disease. Similarly, associations that are established by methods or experiments with high false positive rates may be deprioritized.

In addition to or in combination with prioritizing, filtering can be used to identify variants of interest. Filters can enable a user to start with a large number of variants and eliminate variants that do not satisfy a filter. Accordingly, various filters are described herein. Filters can be used alone or in combination. The filters can be activated in a variety of ways. At a most basic level a user could filter the results manually. For example, a clinician can obtain a list of variants from a sample and then look at each variant one by one and exclude variants based upon a property of interest. For example, the researcher could exclude variants that are not located near a gene of interest. Such a manual approach is cumbersome and time consuming. In preferred embodiments the filters are activated on a computer. The filters can be enacted by a user on a computer selecting from a variety of predetermined filters. The number of variants that survive the filter can be displayed coincident with the selection of the filter to provide a user near instantaneous feedback regarding the degree to which a set of variants is reduced by the application of a filter. In other embodiments the filters are enacted automatically according to a predetermined or predicted need of the user.

### A) Common Variant Filter

As described herein the likelihood of a given variant can be calculated in a given population. The given population can, for example, be a population that is not known to be affected by a particular disease or phenotype. A computer can be configured to filter a set of variants by removing, keeping only, or adding back the common variants. Such a filter is referred to herein as a common variant filter. Without being bound by theory the common variant filter may be useful because if a variant is common in a normal population it may be less likely to be causative of a disease. Alternatively, keeping common variants could be useful to a researcher interested in commonly observed alleles impacting a given pathway. The stringency of a common variant filter can be adjusted by filtering for more or less common variants. So, for example, in some embodiments, a computer is configured to receive a set of variants. The computer then queries a database of common variants and removes the common variants list of variants to be outputted to a user. In some embodiments the computer removes variants or deprioritizes that appear one or more time in a sample of about 1000 subjects known not to have a disorder of interest. In some embodiments variants that appear in 2 or fewer of more than 1000, more than 2000, more than 5000, more than 20,000, or more than 50,000 randomly obtained genomes. In some embodiments the threshold for the common variant filter is approximately the known or predicted distribution of a phenotype or disease in a population. For example, if a disorder is known to occur in 1 in 100,000 subjects in a population then common variant filter can be set to remove or deprioritize variants that occur in, for example, in 5 or more in 100,000 subjects in that population. In some embodiments the computer is configured to compare an inputted list of variants to a statistical map of the genome, wherein the statistical map of the genome reflects a calculated level of statistical variability for genomic regions.

### B) Cancer Driver Variant Filter

Various filters can be applied to focus the attention of a user on variants that are more likely to be involved in cancer or other proliferative disorders. Such filters are herein referred to collectively as the cancer driver variant filter.

Genomic samples obtained from normal cells and from test cells (e.g., cancerous cells or suspected cancerous cells) in a subject can be obtained, the variants can be determined, and the variants in the samples can be analyzed. In some embodiments, a computer is configured to perform the analysis and comparison. For example, variants that are homozygous in the normal cells can be filtered from a list of variants obtained from the test sample. One rationale for this filter is that a cancerous sample has likely acquired a mutation that should not be found in the normal sample. Therefore, a variant that is in the cancerous cells and homozygous in the normal cell, is likely not to be the acquired mutation driving the cancer.

In some embodiments the cancer driver variant filter uses information stored in a database, for example a knowledge base of biomedical content curated using a knowledge base structured with an ontology, to predict and enrich for variants most likely to drive cancer phenotypes by identifying: a) variants impacting known or predicted cancer subnetwork regulatory sites, b) variants impacting cancer-associated cellular processes (e.g. DNA Repair, Apoptosis), c) variants impacting cancer-associated pathways with appropriate directionality, and/or d) cancer therapeutic targets & upstream/causal subnetworks.

In some embodiments the cancer driver filter is configured to use a combination of the above strategies. In some embodiments the combination is selected based upon a hypothesis generated by a user. In various embodiments, cancer driver variant filter uses information from multiple layers of information associated with the study. In some cases, one or more of patient level information (e.g. drug response), disease mechanism level information (e.g. information related to the course of prostate cancer), cellular mechanism level information (e.g. information related to apoptosis or angiogenesis), and molecular mechanism level information (e.g. information related to Fas pathway) can be incorporated into the analysis. In some embodiments the combination is selected automatically by a system to output a tractable number of variants for follow-up study by the user, when used alone or in combination with other filters to form a filter cascade.

The cancer driver variant filter can have its stringency adjusted to filter more or fewer variants. Various ways of adjusting the stringency of the cancer driver variant filter are discussed herein, for example adjusting the stringency by altering the number of hops between a variant and a biological function associated to cancer. To adjust the stringency, it may also be desirable to enable or disable whether the filter looks for variants that meet one or more of the following criteria: (a) affect human genes having animal model orthologs with cancer-associated gene disruption phenotypes, (b) impact known or predicted cancer subnetwork regulatory sites, (c) impact cancer-associated cellular processes with or without enforcement of appropriate directionality, (d) associated with published cancer literature findings in a knowledge base at the variant- and/or gene-level, or (e) impact cancer-associated pathways with or without enforcement of appropriate directionality, and/or (f) associated with cancer therapeutic targets and/or upstream/causal subnetworks.

### C) Predicted Deleterious Filter

A user may wish to keep, remove from, or add back to a list of variants those variants which either are or are not predicted to be deleterious. For example, a clinician investigating the genome of a patient with a suspected genetic disorder might wish to only examine variants predicted to have a negative effect on the biology of the patient. Accordingly, one aspect of the present invention is a predicted deleterious filter. In some embodiments the predicted deleterious filter comprises algorithms based on a sequence or sequences associated with the variants to be filtered. These algorithms can, for example, predict whether a single nucleotide variant (SNV) is predicted to be innocuous (e.g., using a functional prediction algorithm such as SIFT or Polyphen). The following algorithms can be used alone or in combination as a part of the predicted deleterious filter: SIFT, PolyPhen, PolyPhen2, PANTHER, SNPs3D, FastSNP, SNAP, LS-SNP, PMUT, PupaSuite, SNPeffect, SNPeffectV2.0, F-SNP, MAPP, PhD-SNP, MutDB, SNP Function Portal, PolyDoms, SNP@Promoter, Auto-Mute, MutPred, SNP@Ethnos, nsSNPanalyzer, SNP@Domain, StSNP, MtSNPscore, or Genome Variation Server. These algorithms and other suitable algorithms known in the art thatattempt to predict the effect a mutation has on protein function, activity, or regulation may be utilized. For example, predicted transcription factor binding sites, ncRNAs, miRNA targets, enhancers and UTRs can be incorporated into filters to carry out the data analysis. Variants associated with coding vs. non-coding regions can be treated differently. Similarly, variants associated with exons vs. introns can be treated differently. Further, synonymous vs. non-synonymous variants in a coding region can be treated differently. In some cases, the translational machinery of the subject can be considered when analyzing codon changes.

In some embodiments the predicted deleterious filter determines whether a sequence associated with a variant is evolutionarily conserved. Variants occurring in those sequences which have been highly conserved evolutionarily may be expected to be more deleterious, and accordingly in some embodiments the predicted deleterious filter can keep (or remove) these, depending on the application. One measure that can be used to quantify the degree of nucleotide-level evolutionary conservation is Genomic Evolutionary Rate Profiling (GERP).

In some embodiments the predicted deleterious filter assesses the nature of the amino-acid replacement associated with a variant. For example a Grantham matrix score can be calculated. In some instances variants associated with a high or low score are filtered. Similarly, in some embodiments variants are filtered according to Polymorphism Phenotyping or Sorting Intolerant from Tolerant algorithms.

In some embodiments the predicted deleterious filter uses information stored in a database, for example a knowledge base of biomedical content curated using a knowledge base structured with an ontology, to predict and enrich for variants most likely to be pathogenic. Conversely, the predicted deleterious filter can filter variants not likely to be pathogenic. The likelihood of pathogenicity can be established, for example, by identifying a connection between a variant and a known disease causing element.

The predicted deleterious filter can, in some embodiments, give more weight to information regarding whether a variant is likely to be pathogenic based upon the context of the information. For example, a single case or single observation that links a variant to a pathogenic phenotype can be weighed less than when there are multiple unrelated cases or controlled studies reported in the literature and stored in the knowledge base. Similarly data that is generated from a single family can be given less weight than data from multiple families. The weight of the evidence can contribute to whether a filter is applied. The stringency of the filter can be adjusted by including or excluding the weighted evidence. Another variable which in some instances can be used to give more weight to information regarding whether a variant is likely to be pathogenic for the predicted deleterious filter is the extent to which a particular fact has been validated. For example, information regarding a predicted loss of function mutation will be weighted more heavily if there is a reported experiment that demonstrates a change in phenotype or gene product function associated with the mutation. If the same mutation is re-created in an animal model to demonstrate causality even more weight may be given to the fact.

Other factors which can be used to weigh the context of the information related to a variant include but are not limited to the penetrance of a mutation associated with the variant, the statistical power of the studies underlying the information, the number and type of controls involved the studies underlying the information, whether therapeutics are known to act predictably based upon the information, whether multiple mutations in a pathway are known to cause predictable phenotypes, whether there is contradictory evidence in the knowledge base and the volume/credibility of such evidence, whether the variant or variants disrupting the same gene/pathway are frequently observed in healthy individuals, whether or not the position or region in which the variant occurs is highly evolutionarily conserved, and/or whether phenocopies exist and act predictably which are related to the variant.

In some embodiments information related to a predicted deleterious filter can be used to categorize variants according to whether the variants are likely to be pathogenic. This categorization can be performed by a pathogenicity annotator. In some embodiments the strength of data predicting the pathogenicity or nonpathogenicity of a property associated with a biological entity, expressed as likelihood based on entries in the ontology and/or knowledge base. Therefore in some embodiments the pathogenicity annotator expresses a numerical likelihood as a categorization protocol.

In another embodiment the pathogenicity annotator puts variants into categories that resonate with clinical & human genetics researchers that provide a convenient mechanism to get at those variants that have most compelling causal links to disease. This can be accomplished, for example, by leveraging a knowledge base of curated findings from the literature, structured using an ontology, and combining independent lines of literature evidence with analysis of evolutionary conservation and observed allele frequencies in "normal" human populations. In some embodiments the pathogenicity annotator annotates variants that have multiple independent lines of literature evidence supporting a causal association with a deleterious phenotype as a "pathogenic" variant. On the other hand, a variant that is cited by a single article as causal for a rare disease, but found to be present in a high percentage of a population that lacks the rare disease phenotype is more likely to be benign.

For example, variants can be categorized an annotated with the pathogenicity annotator as "Pathogenic," "likely Pathogenic," "uncertain," "Likely Benign," or "Benign," wherein "pathogenic" means <0.07% frequency of the variant in a database of genomes of individuals free from known genetic disease, and 2 or more findings drawing a causal or associative link between the variant (and/or optionally the gene or pathway disrupted by the variant) and a deleterious phenotype from multiple different articles in the biomedical literature; "Presumed Pathogenic" means <0.07% frequency of the variant in a database of genomes of individuals free from known genetic disease, and 1 finding drawing a causal or associative link between the variant (and/or optionally the gene or pathway disrupted by the variant) and a deleterious phenotype; "Unknown" means between 0.07% and 0.1% frequency of the variant in a database of genomes of individuals free from known genetic disease; "Presumed Benign" means between 0.1% and 1% frequency of the variant in a database of genomes of individuals free from known genetic disease; and "benign" : means >=1% frequency of the variant in a database of genomes of individuals free from known genetic disease.

In some embodiments, the pathogenicity annotator is in communication with a knowledge base of disease models that define variants, genes, and pathways that are associated with that disease. The Pathogenicity Annotator utilizes the disease models to provide a pathogenicity assessment for a particular combination of a specific variant and a specific disease.

In some embodiments evolutionary conservation is also used in this prediction. In some embodiments a predicted filter will infer pathogenic status for any variant that does not have variant-level literature finding(s) in the knowledge base to compute clinical significance. In such cases if the variant is in and/or predicted to be deleterious to (one of a few thousand) genes known from the knowledge base to be implicated in a disease, and if the variant is not synonymous and not predicted to be innocuous by a functional prediction algorithm (e.g. frameshift without SIFT prediction or nonsynonymous with no or damaging/activating SIFT prediction), then based on the 1000 Genomes frequencies used for variant-level findings it will be inferred to be pathogenic, presumed pathogenic, uncertain, likely benign, or benign as outlined above. The public SIFT analytic evaluates coding changes observed relative to degree of evolutionary divergence of protein and severity of biochemical change (e.g. hydrophilic to hydrophobic amino acid change) predicted to be caused by a given variant.

### D) Biological Context Filter

As described in the cancer driver variant filter and the predicted deleterious filter, biological context can serve as a variable to screen variants. The biological context filter can use information stored in a database, for example a knowledge base of biomedical content curated using a knowledge base structured with an ontology, to predict and enrich for variants most likely to be related to a biological function. The biological function can be for example a phenotype, a disease, a functional domain, a cellular process, a metabolic or signaling pathway, a behavior, an anatomical characteristic, a physiological trait or state, or a biomarker of one or more of the foregoing. The biological function can also be inferred from effects of gene disruptions in other species, for example phenotypes of mice that have a disruption in a particular gene may be used to identify human variants in the human orthologous gene that may give rise to a related phenotype in humans.

The stringency of a biological context filter can be adjusted so more or fewer variants are allowed to pass the filter. In some embodiments the stringency is adjusted by the user. In some embodiments the stringency is adjusted by a computer and is driven by a predetermined target number of variants surviving the filter or filter cascade.

Selection of a biological function is one way to alter the stringency of a biological context filter. For example a rather low stringency filter would be if a data set of variants is filtered for variants with a known relationship to autoimmune disease. A higher stringency screen would be to filter for the variants with a known relationship to Diabetes mellitus type 1.

Another way to alter the stringency of a biological context filter is to alter the number of hops between a variant and a biological function. Generally, the more hops that are required the less stringent a filter will be. Addition of hops in a biological context filter helps enable discovery of novel causal variants and genes that, when disrupted, can cause human disease.

In a situation where a variant is related to an entity, such as a gene or gene product with a known biological function, through a series of hops it is possible to filter for variants that only work downstream or upstream of a given entity. Accordingly a user can filter, for example, for variants likely to act upstream of one or more known biological processes or entities.

Additionally, a biological context filter can be used to filter for variants that have a specific net effect. For example a screen can be established to screen for variants that, after one or more hops, are likely to result in causal loss of function in one or more particular biological entities or processes. This can be accomplished in some embodiments, by examining the causality between hops. In one non-limiting example, if a user is seeking variants in genes (or gene products) that are within two hops upstream of a biological entity, Gene C, that are known or predicted to cause a net loss-of-function of Gene C or its product, and Gene B is known to activate Gene C, and Gene A is known to activate Gene B, variants that are known or predicted to cause a loss-of-function (but not gain-of-function) in Gene A would be identified as meeting this filter criterion. In another non-limiting example, if a user were looking for variants that are within 2 hops upstream that are known or predicted to cause a net loss-of- function of Gene C or its product, and Gene B is known to repress Gene C, and Gene A is known to activate Gene B, variants that are known or predicted to cause a gain-of-function (but not loss-of- function) in Gene A would can be identified.

### E) Genetic Analysis Filter

Variants can be filtered using genetic logic, for instance by whether they display characteristics consistent with Mendelian inheritance, whether they are frequently observed in one population (e.g. patients affected with a rare hereditary disease, or patients who fail to respond to a particular course of therapy) but not in another (e.g. individuals without disease, or patients who respond to the same course of therapy), whether they frequently perturb the same gene in one population but not in another, and/or whether they frequently perturb the same pathway in one population but not in another. Such a filter is referred to herein as a genetic analysis filter. A genetic analysis filter can involve obtaining genomic information from genetically related subjects. For example, if a researcher or clinician is interested in a genetic disease segregating in one or more families he or she can filter out variants that are not consistent with Mendelian inheritance. In this example the researcher or clinician can obtain genomic information regarding members of a family, wherein some family members have a disease which is following a Mendelian inheritance pattern, but the cause is unknown. The variants can be identified for each family member. Variants which do not satisfy the rules of Mendelian inheritance can be filtered. For example, a variant that is homozygous in one or both parents, but not present in an affected child can be filtered out. A variant present in an affected child, but not present in either of the parents can also be filtered. A variant that is homozygous in child, but absent in one of the parents could also be filtered out. Copy number analysis of the genomic information can be useful for the genetic analysis filter. Single-copy variants that would normally be insufficient to cause loss-of- function could be filtered out, but the same variants occurring in a hemizygous region of the genome could be retained as potentially causal for disease. Likewise, multiple samples from the same individual, such as tumors from different tissue locations or times post-therapeutic treatment, can be compared with the individual's normal genome to filter out variants that are unlikely to be disease-causing due to presence in both the control and matched disease samples for each individual in the data set.

A genetic analysis filter can also utilize known information to include or exclude variants. This can be accomplished by using data contained in the knowledge base regarding human genes and network relationships with other genes. For example, a heterozygous variant that is predicted to perturb a haploinsufficient gene can be included by the genetic analysis filter as potentially giving rise to a disease-causing loss-of- function. A heterozygous variant that is predicted to perturb a gene that is not considered to be haploinsufficient can be excluded by the genetic analysis filter as unlikely to be disease-causing in isolation. The genetic analysis filter can also identify variants that consistently cause loss of function. Often hereditary diseases can have multiple genetic causes that can all give rise to the same or very similar clinical disorder. For example, the disease craniosynostosis can be caused by mutations in Fibroblast Growth Factor Receptor (FGFR) 1, FGFR2, FGFR3, TWIST and EFNB1. New genes that, when mutated, cause craniosynostosis continue to be discovered. For such hereditary diseases that can be caused by mutations in more than one gene, and for those where it is unknown whether or not they can be caused by mutations in more than one gene, it is powerful for the genetic analysis filter to use the Knowledge Base to identify variants that are expected to disrupt function in either the same gene or genes that are within 1-hop or 2-hops away from the gene consistently across one population (e.g. individual(s) with the disease or phenotype of interest) and consistently absent from another population (e.g. individual(s) without the disease or phenotype of interest). Some variants are mutations that cause a single copy of a gene to become overactive, for example by losing a self-inhibitory regulatory sequence. The genetic analysis filter can retain these known or predicted dominant-acting variants regardless of the number of copies found in a genome.

A genetic analysis filter can also determine whether multiple different variants are predicted to disrupt the same gene (or a transcript of a gene) across a population of one or more samples. For example, the genetic analysis filter can determine whether two heterozygous variants might combine in the same sample to disrupt function of a given gene (i.e. compound heterozygous variants) or pathway, and thereby determine whether that same gene (or pathway) is disrupted consistently across one population of individuals (e.g. individual(s) with disease or phenotype of interest), but not in another population (e.g. individuals without the disease or phenotype of interest). This capability can, for example, retain deleterious variants that are heterozygous in both a tumor and a matched normal sample, but are inferred to only cause a loss of gene function in the tumor due to copy number changes or additional (compound) mutations in the gene.

A genetic analysis filter can also take into account the quality of the sequence information. For example a genetic analysis filter may have information regarding the quality or number of representations in a database. Low quality or low representation sequences may be filtered. The stringency of this filter can be adjusted according to metric of data quality. For example, a low stringency version of a genetic analysis filter would allow the inclusion of data with low quality while a high stringency filter could include only high quality data. The genetic analysis filter can include estimates of whether a particular variant is likely to be high quality. For example if a genome is sequenced and a particular variant is only represented one time in the sequencing then the probability of that variant being a sequencing error is higher than if the same variant was sequenced multiple times. The genetic analysis filter can, in some instances, filter sequences that have fewer representations in the database. The genetic analysis filter can also take into account regions of the genome which are more likely to be difficult to acquire quality data for. When a variant is located on or near a genomic feature known to lower sequencing quality and/or to artificially increase the incidence of variants (i.e. a "frequent hitter" region), the genetic analysis filter may filter out such variants. Stringency can be adjusted by inclusion or exclusion of variants that are closer or further from potentially problematic genomic features. For example if a given variant is on or near a highly repetitive region of the genome the genetic analysis filter may exclude that variant.

Accordingly traits such as gain/loss of function, copy number, compound hetereozygosity, haploinsufficiency, frequency in control populations, consistency with Mendelian inheritance patterns, and the consistency of the presence and/or absence of an observation within 2 or more populations at the allele-level, gene-level, and/or pathway-level can all be incorporated into a genetic analysis filter. For example, a genetic analysis filter may identify variants that are consistently enriched or increased in frequency over time at the allele-level, gene-level and/or pathway level over time as a tumor is treated with drug therapy.

### F) Pharmacogenetic Filter

In some instances a user may desire to filter variants based upon known or predicted relationships of the variants to drug targets or proteins involved in drug processing and metabolism. Accordingly, in some embodiments a pharmacogenetic filter filters a list of variants to identify, for example, variants that impact one or more potential drug targets or variants that have been observed or are predicted to impact drug response, metabolism, and/or toxicity. For example, instead of selecting all drugs, a user could select a drug of interest, drug A. The knowledge base can identify that drug A targets gene Z, and the knowledge base can indentify that a loss-of-function of gene Z reduces the effectiveness of drug A in patients. Therefore, the Pharmacogenetic filter can identify that a variant in user's data set that causes or is predicted to cause a loss-of-function in gene Z is expected to have a pharmacogenetic effect relevant to drug A entered by user.

### G. Preconfigurator

Various embodiments of the invention provide systems and methods to analyze sequence variant data from large data sets, including whole genome and whole exome sequencing data. In some cases, the analysis involves searching for sequence variants that may be implicated with a disease or another phenotype of interest. One or more such data sets can be provided by a user and analyzed by the system. Various filtering methods are described above to eliminate sequence variants that are likely unrelated to the studied disease. In various embodiments of the invention, a set of filters can be preconfigured to analyze a desired type of data and identify the most likely interesting variants given the study type. For example, a set of filters can be preconfigured to eliminate sequence variants in the user provided data set based on biological context (e.g. tissue type, disease association, phenotypes, pathways, or processes) while expanding the allowed set of gene variants to one or more hops from those identified by the filters. Sets of filters can be suggested by the system and the user may be allowed to review and modify them. Alternatively, a set of filters can be combined by a user and in some cases saved as a set in the system.

Reducing the number of variants can increase approachability of the application and help users quickly get to, for example, <200 or <50 variants of interest from among thousands, tens of thousands, hundreds of thousands or millions or more variants without manual configuration of the various filters. Whatever the method of combining filters, they can be preconfigured to reduce the number of variants down to a desired number, for example, 1, 2, 3, 4, 5, 6, 7, 8, 9, 10, 15, 20, 25, 50, 100, 200 or more variants. Alternatively, filters can be preconfigured to reduce the number of variants down to less than a desired number, for example, less than 2, 3, 4, 5, 6, 7, 8, 9, 10, 15, 20, 25, 50, 100, 200 or more variants. In some cases, filters can be preconfigured to reduce the number of variants, but not return less than a threshold number, for example, not less than 1, 2, 3, 4, 5, 6, 7, 8, 9, 10, 15, 20, 25, 50, 100, 200 or more variants.

Various embodiments of the invention provide methods to reduce the number of sequence variants using preconfigured filter sets to a target range. In some embodiments, the method is iterative, for example, an initial setting for the set of filters is used to reduce the user provided data set. If the returned number of variants is lower than desired, in some cases, one or more of the filters can be switched to a less stringent setting. In some cases, one or more of the filters can be removed from the set. On the other hand, if the returned number of variants is higher than desired, in some cases, one or more of the filters can be switched to a more stringent setting. In some cases, one or more filters can be added to the set.

In some embodiments filter questions are posed to a user in order to instruct the computer regarding which set of filters to use for a preconfigurator. For example the following questions can be posed to the user:
(1) What best describes what you're trying to accomplish? (radio buttons on an interface can allow user selection - indicated by brackets)
   a.[] Genetic Disease: Identify causal or driver variants for a given disease. (Default)
   b.[ ] Cancer: Identify cancer driver variants
   c.[ ] Stratification: Identify variants that differentiate one group (case) from another (control) group. (Disabled if <1 case or <1 control sample)
   d.[ ] Personal Genome: Find variants that are potentially associated with disease or phenotypes. (Disabled if >1 sample)
   e.[ ] Other: [Describe]
   f. [Next>>]
(2) Is there a particular disease or biological process of interest?
   a. Outlook-like "contains" search on all diseases and processes in the Knowledge Base with autocomplete, user can select 1 or more.
   b. [<<Back] [No, not really.>>] [Yes, selected above>>] (disabled if none selected)
(3) [If "Disease" selected above] What best describes the disease's inheritance pattern? (radio buttons)
   a.[ ] Dominant
   b.] Recessive
   c.[ ] X-linked
   d.] De novo mutation
   e.[ ] Other/Not known
   f. [<<Back] [Next>>] (Disabled if none selected)
(4) [<<Back] [Start Analysis>>]

Depending on the answers to the questions posed to the user a filter logic can select appropriate filters to output a tractable number of variants for follow-up study. An example of a filter logic is:
(1) Automatically add Common Variants filter with default parameters.
(2) Automatically add Predicted Deleterious filter
   a. If Personal Genome is selected AND no specific disease is selected, check only "Pathogenic" & "Possibly Pathogenic"
   b. ELSE, use default parameters except as modified by (4).a.iii.1 below.
(3) If a disease/process is selected, add the Biological Context filter with "Keep only", 2-hops upstream (with "effects" option selected) and 2-hops downstream, with the selected disease/process in the box.
(4) If "Cancer" is selected, add the Genetic Analysis filter with "Keep only" for 100% cases and "Exclude" the same categories of variants present in "1 or more" control samples. Preset options for "Cancer: somatic only (limited to functional impact)"
   a. If all samples are matched, select "Pair/match samples from the same subject" option, and add "nullzygous" and "hemizygous" options. Also, need to add/check "copy number gain", "nullzygous", and "hemizygous" options in the Predicted Deleterious filter.
   b. Add Cancer Driver Variants filter with "Keep only", all options selected.
      i. If disease selected has a cancer disease model, populate Cancer Driver Variants filter with that disease model.
(5) If "Disease" or "Stratification" is selected AND there are 1 or more case and 1 or more control samples: add the Genetic Analysis filter with "Keep only" for 100% cases and "Exclude" the same categories of variants present in "1 or more" control samples.
   a. If "recessive" selected above: set options for "Recessive hereditary disease"
   b. If "dominant" or "other/not known" selected above: set options for "Dominant hereditary disease"
   c. If "X-linked" is selected above, add a physical location filter to keep only those variants that are on the X chromosome.
   d. If "De novo mutation" selected above: set options for "De novo mutation" (i.e., "Restrict to variants consistent with mendelian inheritance" option in Genetic Analysis filter = unchecked.)
(6) If disease selected is a Cancer, add Cancer Driver Variants filter with "Keep only", all options selected.
   a. If disease selected has a cancer disease model, populate Cancer Driver Variants filter with that disease model.
(7) If the result of the bottom-most filter is zero variants
   a. Reduce #/cases required in Genetics filter by 1. If still zero, repeat this step until #/cases in Genetics filter is =1.
   b. Increase Common Variants 1000 Genomes frequency from default to 2%.
   c. If Cancer: Change Genetic Analysis filter from "Cancer: Somatic only (limited to functional impact)" to "Cancer: Somatic only" setting.
   d. Delete the bottom-most filter until result is 1 or more variants.
(8) If the result of the bottom-most filter is >50 variants
   a. reduce Biological context filter downstream hops from 2 to 1. If still >50...
   b. turn off Biological context filter downstream genes. If still >50...
   c. reduce Biological Context filter upstream hops from 2 to 1. If still >50...
   d. Change Biological Context filter upstream setting from "Affects"à"Directly Affects" If still >50...
   e. Turn off Biological context filter upstream genes. If still >50...
   f. Change Predicted Deleterious filter options to remove non-coding variants. If still >50...
   g. Change Predicted Deleterious filter options to keep only variants in the "Pathogenic" category.

In some embodiments the preconfigurator takes into account the context of a user's experiment to adjust relevant content in the computation (eg. what type of cell line did they use, whether they know that certain genes are knocked out or transfected in, etc.). This can allow one to score profiles based on how well they matched up against this background knowledge about the experiment. In other embodiments the preconfigurator preconfigures or provides default selections based on data-driven properties of the variants observed in the user's datasets, for example prespecifying "male" or "female" based on the presense or absence of variants in a given individual's dataset on the Y-chromosome, or prespecifying "cancer" or a cancer type based upon presence (or absence) of certain variants in the dataset. In other embodiments the preconfigurator takes into account medium-throughput data to refine expectations of what is 'normal' for different cells, what proteins potentially can interact, etc. This can provide a normalized baseline across various biological contexts and refine the sensitivity with which one can distinguish statistically significant results.

### H. Pedigree Builder

Various embodiments of the invention provide systems and methods to determine relationships between samples with sequence variations. Taking into account variances or measures of relatedness between samples, some embodiments of the invention may allow pedigrees, or schematics of relationships between samples, to be assembled de novo. This may be achieved by pedigree builder.

In some cases, the pedigree builder may be used to provide phase information about sequence variants identified from sequencing data. Phasing analysis involves searching for the parental source of sequence variants that may be implicated with a disease or another phenotype of interest. In some embodiments for example, a pedigree builder is configured to infer or accept input from the user to identify if a sample is most likely derived from the mother of the individual from whom a given sample was derived. In other embodiments, a pedigree builder is configured to infer or accept input from the user to identify the sample most likely derived from the father of the individual from which a given sample was derived. Phasing information may be important in determining whether one or more variants exist in *cis,* (i.e a single strand of DNA), or in *trans* (i.e. across multiple strands of DNA). This information may be important in assessing the severity of disease of phenotype associated with the variant sequences.

Phasing information about sequence variants may also be utilized by the genetic analysis filter described herein. The genetic filter analysis may utilize phase information to filter variants that are consistent a Mendelian inheritance pattern. This information may also be useful in allowing the pedigree builder to infer trios and family relationships within a given study. For example, this may include but is not limited to clinical trial sample processing.

Further, the pedigree builder is configured to recognize and assign an individual identifier to multiple samples that are taken from a single individual. The pedigree builder is configured to distinguish genetic differences between individuals based on the construction of a genetic pedigree, while retaining the ability to assign the same identifiers to samples that may come from the same individual but reflect some genetic variation. In some embodiments of the invention, this may be useful for the pedigree builder to infer a patient's normal genome from one sample, from tumor genome(s) taken from additional samples taken from the same patient.

In some instances, the pedigree builder may also be configured to indentify inconsistencies between relationships derived from user input and inferred relationships that are derived entirely from computational analysis of the patients' sequence data. In one example, this may include but is not limited to, the identification of cases which may involve non-paternity, sample mislabeling or sample mix-up issues. These issues may otherwise confound analysis and interpretation of a sequence dataset.

### I. Statistical Association Filter

In some instances a user may desire to filter variants based upon statistical association between two or more samples groups and a disease or phenotype of interest. In one embodiment of the invention, a statistical association filter is configured to take the inputs of a previous filter in a filter cascade, and filter variants using a basic allelic, dominant or recessive model. Variants that show a statistically significant difference to one another can be further filtered using a case burden, control burden, or 2-sided burden test. This may indicate how different statistically significant variants perturb a gene differently between two or more sample groups (e.g. phenotype affected vs. unaffected).

In one example, the statistical association filter may be configured to identify variants that are deleterious and contribute to inferred gene-level loss of function and inferred gene-level gain-of- function. This analysis may also utilize the predicted deleterious and genetic analysis filters described herein.

In other embodiments of the invention, the statistical association filter may also be used to filter variants that perturb a whole pathway or gene set. Variants that show statistically significant differences between two or more sample groups may be further filtered using a burden test. In some cases, the burden test may utilize a knowledge base of findings from the literature to identify genes that together form a collective interrelated set based upon shared pathway biology, domain, expression, biological process, disease relevance, group or complex annotation. In some cases the statistical association filter may identify variants that perturb pathways or gene sets significantly more or significantly less between two or more sample groups. In other cases, the burden test may be performed across a library of pathways or gene sets that may be further defined by the user.

### J. Publish Feature

In some embodiments of the invention, a user may want to share or publish results of an analysis. A publish feature may be configured to enable a user to specify an analysis of interest, describe the analysis, and link the details of the analysis to a URL internet link. The URL may be embedded by the user in a publication or other type of disclosure. The publish feature may also be configured such that the user retains the ability to release the published analysis for broad access when the users desires it. In other embodiments of the invention, the publish feature may provide access to the user's published analysis to other users who access the aforementioned URL or who browse a list of available published analyses.

After a given variant has been filtered and identified, various embodiments of the invention provide systems and methods to identify drugs and possible effects on pathways affected by such variants. In some cases, variants are causal variants for diseases or phenotypes. In other cases, variants are drivers of diseases or phenotypes. The druggable pathway feature may be configured to first identify drugs that are known to target, activate and/or repress a gene, gene product, or gene set that co-occurs in the same pathway or genetic network as one or more variants. In some embodiments of the invention, this feature is further configured to predict the net effect of one or more variants in the patient sample on the pathway or genetic network through causal network analysis. In other embodiments, the druggable pathway feature may also further identify drugs that have a net effect on the pathway or genetic network that is directly opposite of the predicted impact of the variant on the pathway or genetic network previously identified.

In some cases, the druggable pathway feature may be used to identify patient samples representing patents likely to respond to one or more specific drugs of interest based on their sequence variant profiles. In some cases the druggable pathway feature may be important in the recruitment, selection or enrollment of patients in pharmaceutical clinical trials. In other cases, the druggable pathway feature may be used in providing novel treatment options for patients.

Various embodiments of the invention also provide systems and methods to identify hypervariable genes or genomic regions. In some embodiments, the the Frequent Hitters filter is configured to access a knowledge base of hypervariable genes and genomic regions that are frequently mutated among a collection of samples derived from individuals unaffected by the disease or phenotype of interest. The Frequent Hitters filters may also filter variants that occur within hypervariable genes or genomic regions. Additionally, the Frequent Hitters filter may also allow annotation of highly repetitive trinucleotide repeats through the Trinucelotide Annotator.

In some cases, the Trinucleotide Annotator is configured to interact with a knowledge base of known trinucleotide repeat regions that contains information on the number of repeats that are benign and the number of repeats that are associated with one or more human phenotypes or severities. In other cases, the Frequent Hitters filter is configured to assess the number of trinucleotide repeats at one or more genomic regions defined in the knowledge base in one or more patient whole genome or exome sequencing samples. In other cases, the Frequency Hitter filter is configured to assess whether the trinucleotide repeat length calculated previously is sufficient to cause a phenotype based on the knowledge base, for each trinucleotide repeat. This information may then be communicated such that the use associated with the trinucleotide repeat length calculated previously may become aware of potential diseases or phenotypes associated with the trinucleotide repeat. Information obtained from the Frequent Hitters filter may also be shared the predicted deleterious filter to enable filtering of variants likely or unlikely to cause a phenotype based on the results of the trinucleotide repeat annotator.

In one example, use of the Frequent Hitter filter may useful for patients with a familial history of Huntington's disease. This neurodegenerative disease is caused by variable length trinucleotide repeats in the Huntingtin gene (HTT). The length of this repeat may vary between individuals as well as between generations. The length of the repeat is thought to affect the severity of Huntington's disease itself. The Frequency Hitter may provide information regarding the length of the tri nucleotide repeat and the severity of the disease known to be associated with that variant length to an individual suspect of having Huntington's disease.

### 5. Applications of the Variants

The invention can be used to aid personalized medicine by elucidating subjects who are more or less likely to respond to a therapy or preventative regimen, or who are more or less likely to experience toxicologic endpoints or adverse events due to a particular treatment regimen, or who are more or less sensitive to a given treatment and therefore may require an alternative dosing, duration of treatment and/or treatment intensity. These discoveries made through use of this invention could manifest, for example, in new companion diagnostics for existing or future treatments to target these treatments to patient populations who will benefit the most and have lowest risk of adverse events.

The invention can also be used to develop individualized cancer treatments by identifying cancer-specific driver variants in specific patients that would be most attractive targets for such therapies as individualized immunotherapy.

The invention can also be used to identify novel variants that are causal, alone or in combination with other variants and/or environmental stimuli, for human diseases or other phenotypes of interest.

In another aspect, this invention comprises a method for identifying diagnostic markers for a given disease. In this aspect, the invention comprises: (a) providing a means for storing and accessing genomics information wherein said means permits computational analysis of complex relationships among the stored concepts and (b) querying the database to identify markers that are associated with the disease. The markers that are associated with the disease can be variants.

The present invention is also useful in the field of pharmacogenomics. For example, in another aspect, the invention provides a method for identifying diagnostic markers specifically for drug response, e.g., unwanted side effects or non-responsiveness. By identifying variants predictive for side-effects or non-responsiveness, a population of patients having a given disease can be stratified into sub-populations based on likelihood of having a serious adverse event or for not responding to a given therapy, for purposes of enrollment in clinical trials or for treatment.

The method of the invention for predicting disease pathways and targets for drug discovery may be enhanced by leveraging the information obtained by querying a database with data obtained from other methods for identifying disease pathways or targets for drug discovery. For example, the method of the invention may include, additionally, the use of absolute and/or differential expression data in conjunction with relationships asserted in the database.

### 6. Providing the data to the system/Accessing the system and Transaction model

The user will provide data to the system in order to analyze or otherwise interpret the data. The data can be uploaded to a local computer running software or the uploading can occur over a network. There can be a combination of both local software and a network or "cloud" based aspect of the system which allows the user to provide the data. In some instances the providing of the data is merely the user allowing the system access to the biological data wherever it is already located, for example the user may allow the system to access a hard drive already containing the data.

The user may repeatedly provide data to the system. In some embodiments, the data is on a computer readable medium, which is provided to the system. For instance the user might buy software which would allow the user to analyze a new dataset at the user's convenience with or without access to a network. Alternatively, the user may access the analysis tools via a network. For instance the user may obtain a password which allows access to the analysis tools over a network. In another embodiment, the user stores data on computer readable media that is operatively linked to the system. The linking can be permitting access to the system.

In one embodiment, the user's ability to provide data to the system is enabled when the user purchases a component necessary for generating the data. For example, the user may be given a code for accessing the system over a network when the user purchases a sequencing instrument or consumable, or purchases sequencing services. In some embodiments, such a transaction comprises the purchase of one or more product(s) or service(s) for the generation of one or more data set(s). Permission to access the data analysis package is optionally provided in a manner that is linked to the transaction. In some embodiments, access to the system and/or payment status is linked to a user's e-mail address. In some embodiments, the access to the data analysis package comprises an access code or partial code. In some embodiments, access is given to the entirety of the data analysis package. In some embodiments, partial access is provided to specific portions of the analysis package. In some embodiments, the access is limited in time, for example, access may be terminated after 3, 6, 9, 12, 25, 24 months or more. In some cases, the access can be extended for periods of time, for example access can be extended for 1, 2, 3, 4, 5, 6 months or more. Additional payment may be required for extensions. In some cases, the data is kept in the system regardless of payment status for the extensions. In various embodiments, the data is loaded to the system regardless of the payment status for access into the system or to any reports generated by the system. The data set is generated using the product or service purchased at the first transaction. In some embodiments, the data collection is at least partially performed by the user. In some embodiments, the data set is shared with the service provider. In some embodiments, the data collection is performed at least partially by a service provider. In some embodiments, the data set is shared with the user. In some embodiments, the first transaction is between the user and the service provider. In some embodiments, the data set is entered into the data analysis package after the data collection. In some embodiments, the data set is entered into the data analysis package during the data collection. In some embodiments, the data is entered to the system by the service provider. In some embodiments, the system provides an output or report to the service provider. In some embodiments, the system provides an output or report to the user. In some embodiments a quote or an option to purchase access to the analysis package is communicated to the user prior to or during the first transaction. A user may be provided with a quote detailing the price for the product or service only, such as the price for sequencing a genome, or a user may be provided a bundled price for gaining access to a data analysis/reporting package and/or any output/reports generated by the package/system, in addition to obtaining the product or service In some embodiments, a second transaction comprises purchasing permission to gain access or partial access to the analysis package. In some embodiments, the first and the second transactions are independent events.

In some embodiments of the invention, the data analysis package accepts one or more user provided data sets in various formats as an input. A user may be the purchaser of the product or service or a secondary entity providing the product or service, such as a sequencing facility. In some embodiments, the data set comprises unprocessed/raw data from an experiment. In various embodiments, the user provided data set is a biological data set. In some embodiments, the user generated data set comprises a whole or partial genome sequence. In some embodiments, the user generated data set comprises RNA sequences or gene expression data.

FIG 11 illustrates an example of a bundled transaction system linking the purchase of a sequencing service to the purchase of an analysis and/or report of the generated sequencing data. In this example, a customer communicates with a service provider and a quote for the sequencing service is generated. The quote includes a bundled option comprising a reporting service resulting from the analysis of the sequencing service, in addition to the sequencing service. An order is placed based on this quote and samples are sent to the sequencing service provider. The generated data is processed. In many cases, the data processing will comprise aligning the sequencing data to other sequencing data in the system, e.g. in a database as described elsewhere in this application, and calling the user data differs, thus identifying sequencing variants. In various cases, a quality control function is performed. Variant Call Files (VCFs) are generated as a result of the data processing. In many cases, the service provider provides the results of the sequencing service to the customer, e.g. by uploading the results into a hard drive and shipping it to the customer. Alternative suitable ways of data transfer, for example by internet, are envisioned and are known to those skilled in the art. In some cases, the VCFs will also be provided to the customer. The VCFs are uploaded to the reporting service, such as a Variant Analysis Report system using suitable methods known in the art, such as via an application programming interface (API) or a user interface (UI). In some implementations, the data is transferred to the reporting system regardless of whether a payment is made for the reporting system. A report can then be generated without further transactions. If the user provided payment or an order for the reporting system, the service provider can send a commission for the report to the report service provider. In various cases, the service provider will communicate to the user the status of the service. A link to access the results of the reporting service can be included during this communication or in a separate communication. The user can use this link to access the report system. If payment is already made for the reporting system, the user can access the report. If payment has not been made an option to make payment to gain access to the system can be provided. In many cases, the user is given permission to manipulate the analysis and generate alternative reports. In some cases, add-on features can be included with the reporting system for fee or for free, such as call support for assisted use of the system.

In FIG. 13 a flow diagram of an embodiment of a system constructed in accordance with the present invention is illustrated. The system is designated generally by the reference numeral **100.** The system **100** provides a method for bundling the transaction for gaining access to a data analysis package with a transaction for a product or service that is used to generate a data set to be entered into the data analysis package for analysis. The flow diagram illustrating system **100,** shows a product or service transaction or discounted transaction **102** and an access or partial access transaction or discounted transaction **103** for the use of the data analysis package. The transaction **102** and **103** are either offered as a selection or a single transaction option including both **102** and **103** is offered. In some embodiments, a price or value is associated with the combined transaction is lower than the sum of two prices or values associated with the subject transactions **102** and **103.** In some embodiments, the price value associated with transaction **102** is zero. In some embodiments, the price value associated with transaction **103** is zero. The system **100,** includes a product or service **110,** which is purchased during the transaction **102.** One or more data sets **111** are generated using the product or service **110.** An access or partial access to the data analysis package **120** is purchased during the transaction **103.** The access or partial access **120** grants permission to use the data analysis package under specified terms. In some embodiments, the transaction **102** grants the purchase of a plurality of products or services **110.** In some embodiments, the transaction **103** grants the purchase of a repeated access or partial access to the data analysis package. In some embodiments, the number of products or services **110** and the number of accesses or partial accesses **120** are linked. In some embodiments, the access or partial access **120** is granted for a specific time period or a specific amount of time.

The system **100** facilitates the generation of data **111** using the product or service **110.** The access or partial access **120** permits the entry of the data **111** into the data analysis package. A first analysis **130** is performed using the data analysis package. The system **100** offers one or more supplementary transactions **140.** An enhanced access or partial access to the data analysis package **150** is purchased during the supplementary transaction **140.** In some embodiments, the supplementary transaction **140** is adjusted for an enhanced partial access **150** to specific parts or functionalities of the data analysis package. An enhanced analysis **160** is performed using the parts and functionalities of the data analysis package purchased during the transaction **150.** In some embodiments an enhanced access or partial access transaction **140** is bundled in an initial transaction **101.**

In some embodiments, an access or partial access to the data analysis package is given through a user registration for the product or service **101.** In some embodiments an access or partial access to the data analysis package is given to a service provider. In some embodiments, the service provider performs all or part of the experiments associated with the prodict or service **110.** In some embodiments, the core lab performs the data analysis.

In some embodiments, a user registration for the product or service **101** comprises an e-mail address and a password. In some embodiments, the password comprises alphanumeric characters. In some embodiments, the password comprises all printable characters. In various embodiments, the password is 1, 2, 3, 4, 5, 6, 7, 8, 9, 10, 11, 12, 13, 14, 15, 16, 17, 18, 19, 20 characters long or longer.

In some embodiments, a right to access parts or all of the data analysis package is provided on a one-time or multiple-time basis. In some embodiments, the right to access is limited within a time period. In some embodiments, the right to access parts or all of the data analysis package is provided with the product or service **110.** In some embodiments a code or serial number accompanies the product or service **110,** which can be used to gain partial or full access to the data analysis package. In some embodiments, the code or serial number accompanying the product or service **101** codifies the type of product or service **101** to the data analysis package. In some embodiments, a user purchases access to the product on a per-sample basis, after which the user is permitted to perform analyses and share that sample and the resulting analyses with other users at no additional charge for prespecified time period. In some embodiments, a user may also run analyses and share analyses of sample collections where such sample collections contain only samples for which access has been previously purchased.

In some embodiments, a computer readable access recognition software recognizes a user. Accordingly, the system grants access to users who have a right to access. In some embodiments, the access recognition software is installed in the user's computer. In some embodiments, the access recognition software is installed remotely. In some embodiments, the access recognition is informed by the user's purchase of a service or product. In various embodiments, the service or product is used to generate a data set that the user analyzes using the data analysis package. In some embodiments, the recognition is based on recognizing a user's computer. In some embodiments, the recognition is based on recognizing a registered e-mail address, IP address, or software (e.g. cookie) stored on the user's computer.

In various embodiments, the product or service **110** is equipped to generate biological data and the generated data 111 comprises a biological data set.

### 7. Examples

### Example 1: identification of the role of IL11RA in craniosyntosis by analyzing comparative whole genome sequencing results using the Ingenuity Knowledge Base

**Variants are identified.** The complete human genome sequence of four subjects is loaded into the system: two genomes from children with a hereditary form of craniosynostosis, and two from their parents who are both unaffected by the disease. The genome of affected Child1 includes 3,714,700 variants, the genome of affected Child2 includes 3,607,874 variants, the genome of the unaffected father includes 3,677,130 variants and the genome of the unaffected mother includes 3,779,223 variants. A total of 5,394,638 variants are found in the combination of the four genomes.

**A common variant filter is applied.** Variants observed in one or more of the subjects in the Complete Genomics 69 Genomes database or the 1000 genome project subjects not observed to have the disease in question are subtracted, reducing the total number of variants to 330,302. The eliminated DNA variants tend to be common in the population and are therefore thought to be unlikely to cause a rare hereditary disease.

**A predicted deleterious filter is applied.** Variants that are not observed to disrupt a biological function or not predicted to do so are identified using the Knowledge Base and are also subtracted, reducing the number of remaining variants to 2,734. For example, coding variants that are synonymous or otherwise predicted to not disrupt protein function by one or more mutation functional prediction algorithms e.g. SIFT and/or Polyphen are removed. Additionally, non-coding variants are removed unless they disrupt a predicted or known splice site, miRNA target, enhancer site, ncRNA, or transcription factor binding site.

**A genetic analysis filter is applied.** The included variants meet the following criteria reducing the number of remaining variants to 12: They must be either (1) homozygous (or possibly homozygous) in both of the affected kids (and neither of the unaffected parents), or (2) expected to otherwise cause loss-of-function in both copies of a given gene (e.g. compound heterozygous) in both of the affected kids (and neither of the unaffected parents), or (3) expected to cause loss-of- function in one or both copies of a given gene that is known by the Ingenuity Knowledge Base to be haploinsufficient in both of the affected kids (and neither of the unaffected parents), or (4) expected to cause loss-of-function in both copies of a gene ("gene1") in the first affected child, and expected to cause loss-of- function in both copies of a different gene ("gene2") in the other affected child where gene2 is in the same pathway or within 1- or 2- network hops of gene1. Optionally, the variants are also filtered such that only variants that are consistent with Mendelian inheritance are retained.

### A biological context filter is applied.

Variants that were not related to the biological context of the disease by network analysis using the knowledge base are filtered out, for example:
Variants that do not alter the function of genes that are either one or two hops upstream (and/or downstream) of other genes previously known to be mutated to cause craniosynostosis based on the knowledge base and ontology, or
Variants that do not alter the function of genes that are within either one or two hops upstream (or downstream) of other genes previously known to be associated with bone formation, a biological process related to craniosyntosis based on the knowledge base and ontology.

The total number of variants is reduced after the final round of filtering to include only one coding variant, in the IL11RA gene, which was confirmed to be the causal variant for the disease in this family.

### Example 2: Identifying prospective driver variants for glioblastoma

A complete or partial human genome sequence of a glioblastoma patient's tumor and another similar genome sequence from the patient's healthy tissue is loaded into the system.

Variants that are observed in one or more of the subjects in the Complete Genomics 69 Genomes database or one or more of the subjects in the 1000 genome project not observed to have the disease in question are subtracted, reducing the total number of variants to 933,866 (Figure 14). These eliminated DNA variants tend to be common in the population and are therefore thought to be unlikely to cause a rare hereditary disease.

Variants that were not previously observed to disrupt a biological function or not predicted to do so are identified using the knowledge base and also subtracted, reducing the number of remaining variants to 10,527. The excluded variants meet one or more of the following criteria:
- Not directly associated with a mutation phenotype finding in the Ingenuity Knowledge Base
- Not synonymous or otherwise innocuous (i.e. not deleterious) based on predictions from one or more mutation functional prediction algorithms e.g. SIFT and/or Polyphen
- Not protein-coding and not known or predicted to occur in splice sites, transcription factor binding sites, ncRNAs, miRNA targets, and/or enhancers

Variants that are homozygous in the healthy tissue are removed, leaving those variants that were picked up by the cancer with the following genetics:
- Homozygous (or possibly homozygous) in the tumor sample(s), or
- would be expected to cause loss-of- function in both copies of a given gene in the tumor sample(s) (e.g., compound heterozygous), or
- would be expected to cause gain-of-function in one or more copies of a given gene in the tumor sample(s), or
- (optionally) would be expected to cause loss-of- function in one or both copies of a given gene that is known by the Ingenuity Knowledge Base to be haploinsufficient

Further, another filter is applied, keeping only variants that are heterozygous in the patient's normal tissue, considering the extremely early onset of the patient's disease in this case suggesting that one of the two copies of a deleterious allele might have been present at birth. Following the application of these genetic analysis filters, the remaining number of variants is reduced to 107.

This patient appears to accumulate mutations at a higher rate than usual, suggesting the biological context of the disease could be related to DNA repair. Thus, all variants that are not related to the biological context of the disease by network analysis using the knowledge base are removed. In this example, only variants meeting one or both of the following criteria are kept, the rest are removed, reducing the remaining number of variants to 2:
- Variants that alter the function of genes that are either 1- or 2-hops upstream (and/or downstream) of other genes previously known to be mutated to cause glioblastoma based on the knowledge base and ontology,
- variants that alter the function of genes that are within either 1- or 2-hops upstream (or downstream) of other genes previously known to be associated with the process of "DNA repair" based on the knowledge base and ontology.

### Example 3: Identifying DNA Variants Toward Development of an Individualized Cancer Therapeutic RNA Cocktail

Figure 15 illustrates the use of a cascade of filters to identify variants for use in a cancer therapeutic RNA cocktail. The complete human genome of a patient's tumor and the patient's normal tissue is loaded into the system providing ~25,000 variants between the two data sets.

The variants that are unique to the tumor and not present in the normal tissue are kept and the rest are removed, reducing the number of variants to ~2,000.

Variants that are not synonymous are candidates to yield a protein-coding difference that the patient's immune system could potentially use to identify tumor cells as different from normal cells and therefore "foreign". These non-synonymous variants are kept and the rest are removed, reducing the number of variants to ~700.

Tumor-specific antigens that can be recognized by a patient's immune system present likely candidates for the immune system to fight the tumor. Thus, variants that are not known to be expressed in the tumor are filtered out, reducing the number of remaining variants to ~150. Variants that are not well-expressed in the tumor are less likely to be presented on the surface of tumor cells at a sufficient abundance to be detected by the immune system.

Variants that would be predicted to be critical to the tumor, i.e. cancer driver variants, are summarized herein. Focusing on these variants reduces the likelihood that the cancer will be able to evolve to "escape" a future immunotherapy treatment. Using the cancer driver variants filter, the number of remaining variants is reduced to ~40.

Variants that are most likely to elicit an immune response can be predicted based on measures from the IEDB database. An additional immunogenicity filter reduces the number of variants to ~30. During the application of successive filters described in this example, the stringencies of the filters above is adjusted such that fewer than 50; ideally fewer than 30 variants survive the filters. This range provides a desired number of variants for inclusion in an RNA vaccine. An RNA vaccine can be developed using the variant information obtained in this example and can be delivered, e.g. to the patient's lymph nodes, where it will be taken up by dendritic cells which will effectively "train" the patient's T-cells to attack the patient's tumor cells.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims.

## Claims

1. A system for implementing a biological context filter, the system comprising:
a memory; and
a processor coupled to the memory, wherein the processor is configured to:
receive a data set comprising variants, wherein the data set comprises a plurality of variants from one or more samples from one or more individuals;
communicate with a database of biological information, wherein the database of biological information is a knowledge base of curated biomedical content and structured with an ontology, wherein the knowledge base comprises a confidence measure associated with the biomedical content;
receive a biological function, a biological concept, a plurality of hops, and a request to filter the data set;
filter the received data set using the database of biological information to predict a subset of variants in the data set most likely related to the biological function based on the biological concept and the plurality of hops by:
identifying a causality between a variant in the subset of variants and the biological function in the knowledge base for each hop of the plurality of hops, wherein each respective hop of the plurality of hops comprises a relationship between biological entities; and
provide the subset of variants in an interactive report; wherein biological entities that are one hop away from each other are nodes connected by an edge in the knowledge base, and
wherein the processor, using the biological context filter, is configured to filter for a subset of variants that have a specific net effect by examining the causality between hops.

2. The system according to claim 1, wherein the specific net effect is causing a net loss-of-function or a net gain-of function with respect to the biological entity.

3. The system according to claim 1 or 2, wherein the filtering comprises at least one of unmasking variants associated with the biological function or masking variants not associated with the biological function.

4. The system according to any one of claims 1 to 3, wherein the filtering comprises at least one of masking variants associated with biological function or unmasking variants not associated with the biological function.

5. The system according to any one of claims 1 to 4, further configured to: infer the biological function for filtering based on the data set or from study design information previously inputted by a user.

6. The system according to any one of claims 1 to 5, wherein the biological context filter is combined with one or more filters in a filter cascade to generate a final variant list.

7. The system according to claim 6, wherein the biological context filter is combined with one or more of the following filters in the filter cascade to reach the final variant list of less than 200 variants: common variant filter, predicted deleterious filter, cancer driver variants filter, physical location filter, genetic analysis filter, expression filter, user-defined variants filter, pharmacogenetics filter, or custom annotation filter.

8. The system according to any one of claims 1 to 7, wherein the biological context filter is combined with one or more of the following filters in a filter cascade to reach a final variant list of less than 50 variants: common variant filter, predicted deleterious filter, cancer driver variants filter, physical location filter, genetic analysis filter, expression filter, user-defined variants filter, pharmacogenetics filter, or custom annotation filter.

9. The system according to any one of claims 1 to 8, wherein the stringency of the biological context filter can be adjusted by a user or adjusted automatically based on a desired number of variants in the subset of variants, and wherein the stringency adjustment from the user alters one or more of the following:
a) the hops of the plurality of hops in an association used for filtering;
b) a strength of the hops of the plurality of hops in an association used for filtering;
c) a net effect of the hops of the plurality of hops in an association used for filtering; and/or
d) an upstream or a downstream nature of the hops of the plurality of hops in an association used for filtering.

10. The system according to any one of claims 1 to 9, wherein the biological function and relationship is received using a user interface.

11. The system according to any one of claims 1 to 10, wherein the biological function is a gene, a transcript, a protein, a molecular complex, a molecular family or enzymatic activity, a therapeutic or therapeutic molecular target, a pathway, a process, a phenotype, a disease, a functional domain, a behavior, an anatomical characteristic, a physiological trait or state, a biomarker or a combination thereof.

12. The system according to any one of claims 1 to 11, wherein the biological context filter is further configured to accept a mask from another filter previously performed on the same data set.

13. A computer program product bearing machine readable instructions to enact the biological context filter according to any of claims 1 to 12.

14. A method for identifying prospective causal variants comprising:
receiving, by one or more computing devices, a data set comprising variants, wherein the data set comprises a plurality of variants from one or more samples from one or more individuals;
communicating, by the one or more computing devices, with a database of biological information, wherein the database of biological information is a knowledge base of curated biomedical content and structured with an ontology, wherein the knowledge base comprises a confidence measure associated with the biomedical content;
receiving, by the one or more computing devices, a biological function, a biological concept, a plurality of hops, and a request to filter the data set; and
filtering, by the one or more computing devices and using a biological context filter, the received data set using the database of biological information to predict a subset of variants in the data set most likely related to the biological function based on the biological concept and the plurality of hops:
identifying a causality between a variant in the subset of variants and the biological function in the knowledge base for each hop of the plurality of hops, wherein each respective hop of the plurality of hops comprises a relationship between biological entities; and providing, by the one or more computing devices, the subset of variants in an interactive report; wherein biological entities that are one hop away from each other are nodes connected by an edge in the knowledge base, and
wherein the biological context filter is configured to filter for a subset of variants that have a specific net effect by examining the causality between hops.

15. The method according to claim 14, wherein the specific net effect is causing a net loss-of-function or a net gain-of function with respect to the biological entity.

16. The method according to claim 14 or 15, further comprising:
filtering, by the one or more computing devices, the received data set with one or more common variants filters;
filtering, by the one or more computing devices, the received data set with one or more predicted deleterious filters;
filtering, by the one or more computing devices, the received data set with one or more genetic analysis filters, wherein a filtered data set is generated by filtering the received dataset using the one or more common variants filters, the one or more predicted deleterious filters, and the one or more genetic analysis filters; and
outputting, by the one or more computing devices, the filtered data set as a list of prospective causal variants.

## Patentansprüche

1. System zum Implementieren eines biologischen Kontextfilters, das System umfassend:
einen Speicher; und
einen Prozessor, der mit dem Speicher gekoppelt ist, wobei der Prozessor konfiguriert ist zum:
Empfangen eines Datensatzes, umfassend Varianten, wobei der Datensatz mehrere Varianten aus einer oder mehreren Proben von einem oder mehreren Individuen umfasst;
Kommunizieren mit einer Datenbank für biologische Information, wobei die Datenbank für biologische Information eine Wissensdatenbank mit sorgfältig ausgewähltem biomedizinischen Inhalt ist und mit einer Ontologie strukturiert ist, wobei die Wissensdatenbank ein Konfidenzmaß umfasst, das mit dem biomedizinischen Inhalt verknüpft ist;
Empfangen einer biologischen Funktion, eines biologischen Konzepts, mehrerer Sprünge und einer Anforderung zum Filtern des Datensatzes;
Filtern des empfangenen Datensatzes unter Verwendung der Datenbank für biologische Information, um einen Teilsatz von Varianten in dem Datensatz vorherzusagen, die sich basierend auf dem biologischen Konzept und den mehreren Sprüngen am wahrscheinlichsten auf die biologische Funktion beziehen, durch:
Identifizieren einer Kausalität zwischen einer Variante in dem Teilsatz von Varianten und der biologischen Funktion in der Wissensdatenbank für jeden Sprung der mehreren Sprünge, wobei jeder jeweilige Sprung der mehreren Sprünge eine Beziehung zwischen biologischen Entitäten umfasst; und
Bereitstellen des Teilsatzes von Varianten in einem interaktiven Bericht; wobei biologische Entitäten, die einen Sprung voneinander entfernt sind, Knoten sind, die durch eine Kante in der Wissensdatenbank verbunden sind, und
wobei der Prozessor unter Verwendung des biologischen Kontextfilters konfiguriert ist, um durch Untersuchen der Kausalität zwischen Sprüngen nach einem Teilsatz von Varianten zu filtern, die einen spezifischen Nettoeffekt umfassen.

2. System nach Anspruch 1, wobei der spezifische Nettoeffekt einen Nettoverlust der Funktion oder einen Nettogewinn der Funktion in Bezug auf die biologische Entität bewirkt.

3. System nach Anspruch 1 oder 2, wobei das Filtern mindestens eines von einem Demaskieren von mit der biologischen Funktion verknüpften Varianten oder einem Maskieren von nicht mit der biologischen Funktion verknüpften Varianten umfasst.

4. System nach einem der Ansprüche 1 bis 3, wobei das Filtern mindestens eines von dem Maskieren von mit der biologischen Funktion verknüpften Varianten oder dem Demaskieren von nicht mit der biologischen Funktion verknüpften Variante umfasst.

5. System nach einem der Ansprüche 1 bis 4, das ferner konfiguriert ist zum: Ableiten der biologischen Funktion zum Filtern basierend auf dem Datensatz oder aus zuvor von einem Benutzer eingegebenen Studiendesigninformationen.

6. System nach einem der Ansprüche 1 bis 5, wobei der biologische Kontextfilter mit einem oder mehreren Filtern in einer Filterkaskade kombiniert wird, um eine endgültige Variantenliste zu erzeugen.

7. System nach Anspruch 6, wobei der biologische Kontextfilter mit einem oder mehreren der folgenden Filter in der Filterkaskade kombiniert wird, um die endgültige Variantenliste von weniger als 200 Varianten zu erhalten: Filter für häufige Varianten, Filter für vorhergesagte Schädlichkeit, Filter für Krebs-Treiber-Varianten, Filter für physische Position, Filter für genetische Analyse, Expressionsfilter, Filter für benutzerdefinierte Varianten, Pharmakogenetikfilter oder Filter für benutzerdefinierte Annotation.

8. System nach einem der Ansprüche 1 bis 7, wobei der biologische Kontextfilter mit einem oder mehreren der folgenden Filter in einer Filterkaskade kombiniert wird, um eine endgültige Variantenliste von weniger als 50 Varianten zu erhalten: Filter für häufige Varianten, Filter für vorhergesagte Schädlichkeit, Filter für Krebs-Treiber-Varianten, Filter für physische Position, Filter für genetische Analyse, Expressionsfilter, Filter für benutzerdefinierte Varianten, Pharmakogenetikfilter oder Filter für benutzerdefinierte Annotation.

9. System nach einem der Ansprüche 1 bis 8, wobei die Stringenz des biologischen Kontextfilters von einem Benutzer angepasst werden kann oder basierend auf einer gewünschten Anzahl von Varianten in dem Teilsatz von Varianten automatisch angepasst werden kann, und wobei die Stringenzanpassung durch den Benutzer eines oder mehrere der Folgenden ändert:
a) die Sprünge der mehreren Sprünge in einer zum Filtern verwendeten Verknüpfung;
b) eine Stärke der Sprünge der mehreren Sprünge in einer zum Filtern verwendeten Verknüpfung;
c) einen Nettoeffekt der Sprünge der mehreren Sprünge in einer zum Filtern verwendeten Verknüpfung; und/oder
d) eine stromaufwärtige oder stromabwärtige Beschaffenheit der Sprünge der mehreren Sprünge in einer zum Filtern verwendeten Verknüpfung.

10. System nach einem der Ansprüche 1 bis 9, wobei die biologische Funktion und Beziehung mittels einer Benutzerschnittstelle empfangen wird.

11. System nach einem der Ansprüche 1 bis 10, wobei die biologische Funktion ein Gen, ein Transkript, ein Protein, ein molekularer Komplex, eine molekulare Familie oder enzymatische Aktivität, ein Therapeutikum oder therapeutisches molekulares Ziel, ein Signalweg, ein Prozess, ein Phänotyp, eine Erkrankung, eine funktionelle Domäne, ein Verhalten, ein anatomisches Merkmal, eine physiologische Eigenschaft oder ein physiologischer Zustand, ein Biomarker oder eine Kombination davon ist.

12. System nach einem der Ansprüche 1 bis 11, wobei der biologische Kontextfilter ferner konfiguriert ist, um eine Maske von einem anderen Filter anzunehmen, der zuvor auf demselben Datensatz ausgeführt wurde.

13. Computerprogrammprodukt, das maschinenlesbare Anweisungen trägt, um den biologischen Kontextfilter nach einem der Ansprüche 1 bis 12 auszuführen.

14. Verfahren zum Identifizieren prospektiver kausaler Varianten, umfassend:
Empfangen, durch eine oder mehrere Rechenvorrichtungen, eines Datensatzes, umfassend Varianten, wobei der Datensatz mehrere Varianten aus einer oder mehreren Proben von einem oder mehreren Individuen umfasst;
Kommunizieren, durch die eine oder die mehreren Rechenvorrichtungen, mit einer Datenbank für biologische Informationen, wobei die Datenbank für biologische Informationen eine Wissensdatenbank mit sorgfältig ausgewähltem biomedizinischem Inhalt ist und mit einer Ontologie strukturiert ist, wobei die Wissensdatenbank ein Konfidenzmaß umfasst, das mit dem biomedizinischen Inhalt verknüpft ist;
Empfangen, durch die eine oder die mehreren Rechenvorrichtungen, einer biologischen Funktion, eines biologischen Konzepts, mehreren Sprüngen und einer Anforderung zum Filtern des Datensatzes; und
Filtern, durch die eine oder die mehreren Rechenvorrichtungen und unter Verwendung eines biologischen Kontextfilters, des empfangenen Datensatzes unter Verwendung der Datenbank für biologische Informationen, um einen Teilsatz von Varianten in dem Datensatz vorherzusagen, die sich basierend auf dem biologischen Konzept und den mehreren Sprüngen am wahrscheinlichsten auf die biologische Funktion beziehen:
Identifizieren einer Kausalität zwischen einer Variante in dem Teilsatz von Varianten und der biologischen Funktion in der Wissensdatenbank für jeden Sprung der mehreren Sprünge, wobei jeder jeweilige Sprung der mehreren Sprünge eine Beziehung zwischen biologischen Entitäten umfasst; und Bereitstellen des Teilsatzes von Varianten in einem interaktiven Bericht durch die eine oder die mehreren Rechenvorrichtungen; wobei biologische Entitäten, die einen Sprung voneinander entfernt sind, Knoten sind, die durch eine Kante in der Wissensdatenbank verbunden sind, und
wobei der biologische Kontextfilter konfiguriert ist, um durch Untersuchen der Kausalität zwischen Sprüngen nach einem Teilsatz von Varianten zu filtern, die einen spezifischen Nettoeffekt umfassen.

15. Verfahren nach Anspruch 14, wobei der spezifische Nettoeffekt einen Nettoverlust der Funktion oder einen Nettogewinn der Funktion in Bezug auf die biologische Entität bewirkt.

16. Verfahren nach Anspruch 14 oder 15, ferner umfassend:
Filtern, durch die eine oder die mehreren Rechenvorrichtungen, des empfangenen Datensatzes mit einem oder mehreren Filtern für häufige Varianten;
Filtern, durch die eine oder die mehreren Rechenvorrichtungen, des empfangenen Datensatzes mit einem oder mehreren Filtern für vorhergesagte Schädlichkeit;
Filtern, durch die eine oder die mehreren Rechenvorrichtungen, des empfangenen Datensatzes mit einem oder mehreren Filtern für genetische Analyse, wobei ein gefilterter Datensatz durch Filtern des empfangenen Datensatzes unter Verwendung des einen oder der mehreren Filter für häufige Varianten, des einen oder der mehreren Filter für vorhergesagte Schädlichkeit und des einen oder der mehreren Filter für genetische Analyse erzeugt wird; und
Ausgeben, durch die eine oder die mehreren Rechenvorrichtungen, des gefilterten Datensatzes als eine Liste prospektiver kausaler Varianten.

## Revendications

1. Système permettant de mettre en œuvre un filtre de contexte biologique, le système comprenant :
une mémoire ; et
un processeur couplé à la mémoire, dans lequel le processeur est configuré pour :
recevoir un ensemble de données comprenant des variants, dans lequel l'ensemble de données comprend une pluralité de variants provenant d'un ou de plusieurs échantillons provenant d'un ou de plusieurs individus ;
communiquer avec une base de données d'informations biologiques, dans lequel la base de données d'informations biologiques est une base de connaissances de contenu biomédical organisé et structurée avec une ontologie, dans lequel la base de connaissances comprend une mesure de confiance associée au contenu biomédical ;
recevoir une fonction biologique, un concept biologique, une pluralité de sauts et une demande de filtrage de l'ensemble de données ;
filtrer l'ensemble de données reçu à l'aide de la base de données d'informations biologiques pour prédire un sous-ensemble de variants dans l'ensemble de données le plus probablement lié à la fonction biologique en fonction du concept biologique et de la pluralité de sauts par :
l'identification d'une causalité entre un variant dans le sous-ensemble de variants et la fonction biologique dans la base de connaissances pour chaque saut de la pluralité de sauts, dans lequel chaque saut respectif de la pluralité de sauts comprend une relation entre des entités biologiques ; et
fournir le sous-ensemble de variants dans un rapport interactif ; dans lequel des entités biologiques qui sont à un saut les unes des autres sont des nœuds connectés par une arête dans la base de connaissances, et
dans lequel le processeur, à l'aide du filtre de contexte biologique, est configuré pour filtrer un sous-ensemble de variants qui ont un effet net spécifique en examinant la causalité entre les sauts.

2. Système selon la revendication 1, dans lequel l'effet net spécifique provoque une perte de fonction nette ou un gain de fonction net par rapport à l'entité biologique.

3. Système selon la revendication 1 ou 2, dans lequel le filtrage comprend au moins l'un parmi le démasquage de variants associés à la fonction biologique ou le masquage de variants non associés à la fonction biologique.

4. Système selon l'une quelconque des revendications 1 à 3, dans lequel le filtrage comprend au moins l'un parmi le masquage de variants associés à une fonction biologique ou le démasquage de variants non associés à la fonction biologique.

5. Système selon l'une quelconque des revendications 1 à 4, configuré en outre pour : inférer la fonction biologique pour le filtrage en fonction de l'ensemble de données ou d'informations de plan d'étude précédemment saisies par un utilisateur.

6. Système selon l'une quelconque des revendications 1 à 5, dans lequel le filtre de contexte biologique est combiné avec un ou plusieurs filtres dans une cascade de filtres pour générer une liste finale de variants.

7. Système selon la revendication 6, dans lequel le filtre de contexte biologique est combiné avec un ou plusieurs des filtres suivants dans la cascade de filtres pour atteindre la liste finale de variants de moins de 200 variants : filtre de variants communs, filtre de variants délétères prédits, filtre de variants provoquant le cancer, filtre d'emplacement physique, filtre d'analyse génétique, filtre d'expression, filtre de variants définis par l'utilisateur, filtre pharmacogénétique ou filtre d'annotation personnalisée.

8. Système selon l'une quelconque des revendications 1 à 7, dans lequel le filtre de contexte biologique est combiné avec un ou plusieurs des filtres suivants dans une cascade de filtres pour atteindre une liste finale de variants de moins de 50 variants : filtre de variants communs, filtre de variants délétères prédits, filtre de variants provoquant le cancer, filtre d'emplacement physique, filtre d'analyse génétique, filtre d'expression, filtre de variants définis par l'utilisateur, filtre pharmacogénétique ou filtre d'annotation personnalisée.

9. Système selon l'une quelconque des revendications 1 à 8, dans lequel la stringence du filtre de contexte biologique peut être ajustée par un utilisateur ou ajustée automatiquement en fonction d'un nombre souhaité de variants dans le sous-ensemble de variants, et dans lequel l'ajustement de la stringence par l'utilisateur modifie un ou plusieurs des éléments suivants :
a) les sauts de la pluralité de sauts dans une association utilisée pour le filtrage ;
b) une intensité des sauts de la pluralité de sauts dans une association utilisée pour le filtrage ;
c) un effet net des sauts de la pluralité de sauts dans une association utilisée pour le filtrage ; et/ou
d) une nature en amont ou en aval des sauts de la pluralité de sauts dans une association utilisée pour le filtrage.

10. Système selon l'une quelconque des revendications 1 à 9, dans lequel la fonction biologique et la relation sont reçues à l'aide d'une interface utilisateur.

11. Système selon l'une quelconque des revendications 1 à 10, dans lequel la fonction biologique est un gène, un transcrit, une protéine, un complexe moléculaire, une famille moléculaire ou une activité enzymatique, une cible thérapeutique ou moléculaire thérapeutique, une voie, un processus, un phénotype, une maladie, un domaine fonctionnel, un comportement, une caractéristique anatomique, un trait ou état physiologique, un biomarqueur ou une combinaison de ceux-ci.

12. Système selon l'une quelconque des revendications 1 à 11, dans lequel le filtre de contexte biologique est en outre configuré pour accepter un masque provenant d'un autre filtre précédemment effectué sur le même ensemble de données.

13. Produit-programme d'ordinateur portant des instructions lisibles par machine pour mettre en œuvre le filtre de contexte biologique selon l'une quelconque des revendications 1 à 12.

14. Procédé pour l'identification de variants causaux prospectifs comprenant :
la réception, par un ou plusieurs dispositifs informatiques, d'un ensemble de données comprenant des variants, dans lequel l'ensemble de données comprend une pluralité de variants provenant d'un ou de plusieurs échantillons provenant d'un ou de plusieurs individus ;
la communication, par le ou les dispositifs informatiques, avec une base de données d'informations biologiques, dans lequel la base de données d'informations biologiques est une base de connaissances de contenu biomédical organisé et structurée avec une ontologie, dans lequel la base de connaissances comprend une mesure de confiance associée au contenu biomédical ;
la réception, par le ou les dispositifs informatiques, d'une fonction biologique, d'un concept biologique, d'une pluralité de sauts et d'une demande de filtrage de l'ensemble de données ; et
le filtrage, par le ou les dispositifs informatiques et à l'aide d'un filtre de contexte biologique, de l'ensemble de données reçu à l'aide de la base de données d'informations biologiques pour prédire un sous-ensemble de variants dans l'ensemble de données le plus probablement lié à la fonction biologique en fonction du concept biologique et de la pluralité de sauts :
l'identification d'une causalité entre un variant dans le sous-ensemble de variants et la fonction biologique dans la base de connaissances pour chaque saut de la pluralité de sauts, dans lequel chaque saut respectif de la pluralité de sauts comprend une relation entre des entités biologiques ; et la fourniture, par le ou les dispositifs informatiques, du sous-ensemble de variants dans un rapport interactif ; dans lequel des entités biologiques qui sont à un saut les unes des autres sont des nœuds connectés par une arête dans la base de connaissances, et
dans lequel le filtre de contexte biologique est configuré pour filtrer un sous-ensemble de variants qui ont un effet net spécifique en examinant la causalité entre les sauts.

15. Procédé selon la revendication 14, dans lequel l'effet net spécifique provoque une perte de fonction nette ou un gain de fonction net par rapport à l'entité biologique.

16. Procédé selon la revendication 14 ou 15, comprenant en outre :
le filtrage, par le ou les dispositifs informatiques, de l'ensemble de données reçu à l'aide d'un ou de plusieurs filtres de variants communs ;
le filtrage, par le ou les dispositifs informatiques, de l'ensemble de données reçu à l'aide d'un ou plusieurs filtres de variants délétères prédits ;
le filtrage, par le ou les dispositifs informatiques, de l'ensemble de données reçu à l'aide d'un ou de plusieurs filtres d'analyse génétique, dans lequel un ensemble de données filtré est généré en filtrant l'ensemble de données reçu à l'aide du ou des filtres de variants communs, du ou des filtres de variants délétères prédits et du ou des filtres d'analyse génétique ; et
la sortie, par le ou les dispositifs informatiques, de l'ensemble de données filtré en tant que liste de variants causaux prospectifs.
